(19) Europäisches Patentamt
European Patent Office
Office européen des brevets

(11) EP 4 177 248 A1

(12) EUROPEAN PATENT APPLICATION
published in accordance with Art. 153(4) EPC

(43) Date of publication:
10.05.2023 Bulletin 2023/19

(21) Application number: 21833899.4

(22) Date of filing: 27.04.2021

(51) International Patent Classification (IPC):
*C07D 405/04* (2006.01) *C07D 211/14* (2006.01)
*C07D 401/06* (2006.01)

(52) Cooperative Patent Classification (CPC):
A61K 31/451; A61K 31/4535; A61K 31/4545;
A61P 11/02; A61P 11/06; A61P 17/00;
A61P 27/14; A61P 29/00; A61P 37/08;
C07D 211/14; C07D 211/70; C07D 401/04;
C07D 401/06; C07D 405/04; C07D 409/04

(86) International application number:
PCT/CN2021/090073

(87) International publication number:
WO 2022/001331 (06.01.2022 Gazette 2022/01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 03.07.2020 CN 202010629198

(71) Applicants:
- Hefei Institute of Pharmaceutical Industry Co., Ltd.
  Hefei, Anhui 230601 (CN)
- Hefei Amvite Pharmaceutical Co., Ltd
  Hefei, Anhui 230601 (CN)

(72) Inventors:
- HE, Guangwei
  Hefei, Anhui 230601 (CN)
- CHU, Zhaoxing
  Hefei, Anhui 230601 (CN)
- XU, Qinlong
  Hefei, Anhui 230601 (CN)
- MO, Jiajia
  Hefei, Anhui 230601 (CN)
- LIN, Gaofeng
  Hefei, Anhui 230601 (CN)
- ZHAO, Yan
  Hefei, Anhui 230601 (CN)
- SHAO, Li
  Hefei, Anhui 230601 (CN)

(74) Representative: Danubia Patent & Law Office LLC
Bajcsy-Zsilinszky út 16
1051 Budapest (HU)

# (54) A CLASS OF TRICYCLIC COMPOUNDS, PREPARATION METHOD THEREFOR, AND MEDICAL USE THEREOF

(57) Provided in the present invention is a class of tricyclic compounds, a preparation method therefor, and the medical use thereof. The tricyclic compounds are compounds having the structure of the following formula (I) or a pharmaceutically acceptable salt, a prodrug, a tautomer, a stereoisomer, or a mixture of stereoisomers thereof. The compounds of the present invention have a significant activity in terms of antagonizing histamine H 1 receptors, and has fewer side effects against anti-M-choline and a lower hERG toxicity. Specifically, the definitions of each group are as set forth in the description.

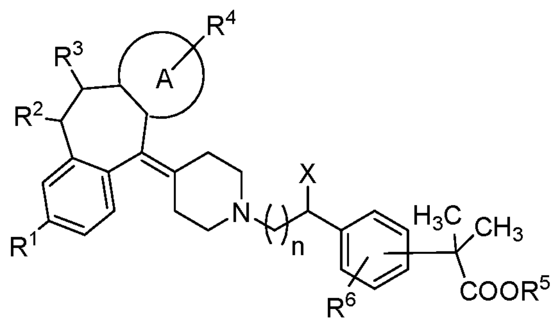

(I)

EP 4 177 248 A1

## Description

### Background of the Invention

The present invention relates to the field of medicinal chemistry, in particular to tricyclic compounds, preparation methods and medical uses thereof. Compared with the prior art, the compound of the present invention has significant antihistamine activity and has extremely low choline inhibition side effects and extremely low *h*ERG toxicity. The present invention also provides its use in the preparation of drugs for the prevention and treatment of allergic diseases.

Allergic diseases such as acute urticaria, chronic idiopathic urticaria, allergic rhinitis, allergic ophthalmia, allergic skin diseases (including eczema, pityriasis rosea, drug eruption, summer dermatitis, contact dermatitis, atopic dermatitis) , Henoch-Schonlein purpura, pollen allergy, bronchial asthma, etc., have greatly reduced people's quality of life. Currently, antihistamines and immunosuppressants are mainly used in the clinical treatment of such diseases.

H1 receptor antagonists are mainly among antihistamine drugs. H1 receptor antagonists can competitively bind to H1 receptors and block the effects of histamine and H1 receptors, thereby inhibiting histamine from exerting biological effects and getting the effect of anti-allergic [Zolaly MA. Histamine H1 antagonists and clinical characteristics of febrile seizures[J]. Int J Gen Med. 2012, 5: 277-281]. Currently commonly used clinical histamine H1 receptor antagonists are mainly divided into two generations. The first-generation antihistamines such as chlorpheniramine, diphenhydramine and hydroxyzine have significant central system side effects and easily cause drowsiness and unconciousness. The second-generation antihistamines such as terfenadine, loratadine, desloratadine and astemizole overcome the shortcomings of the central system side effects of the first-generation antihistamines, but some compounds of them have serious cardiotoxicity. For examples, terfenadine and astemizole can cause arrhythmias, prolong Q-T interval on electrocardiogram and even cause cardiac arrest and sudden death. Although other drugs such as loratadine and desloratadine have not been seen obvious cardiotoxicity, there are obvious side effects of inhibiting choline such as dry mouth and dry eyes, which can cause motor dysfunction in severe cases.

Therefore, it is of great value to develop new H1 receptor blockers with higher safety to overcome the shortcomings of existing therapeutic drugs, reduce the toxicity to the central system and heart, and reduce the activity of inhibiting choline.

The Chinese patent CN107602534B applied by the present inventor discloses a series of desloratadine derivatives with antihistamine and anti-inflammatory activities. Among them, the compound LHC-7 has the best activity.

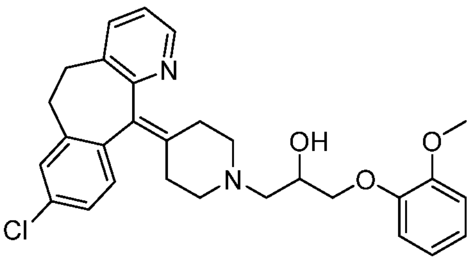

**LHC-7**

However, in subsequent studies, it was found they have strong *h*ERG toxicity (due to strong inhibition of hERG potassium channels, there is a risk of cardiotoxicity).

The Chinese patent CN109096251A applied by the present inventor discloses a series of multi-target desloratadine derivatives, including the compound LP-2.

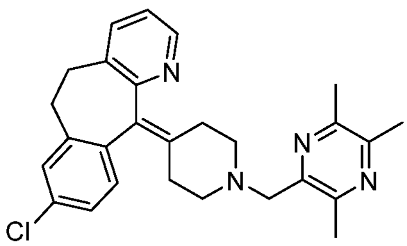

**LP-2**

The world patent application WO9200293A1 also discloses a series of antihistamine compounds, including compound 1 with the following structure:

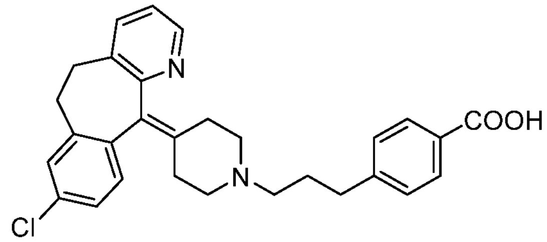

1

However, the implementation method and activity data of compound 1 are not disclosed in the world patent application.

The present invention has researched and developed a series of new compounds on the basis of the prior art, and found that these compounds exhibit excellent effects and functions. In particular, the compound of the present invention not only retains significant antihistamine activity, but also has lower anticholinergic activity and lower hERG toxicity than desloratadine, LHC-7, LP-2 or compound 1. The compound of the present invention has higher security.

## Detailed Description of the Invention

In view of the shortcomings of the prior art, the present invention provides new tricyclic compounds through structural modification. Pharmacodynamic experiments show that the compound of the present invention significantly antagonizes histamine H1 receptor activity, significantly reduces or basically eliminates the anticholinergic activity of the compound disclosed in the prior art and reduces the inhibitory activity of the *h*ERG potassium channel of the compound in the prior art. Specifically, the present invention provides a tricyclic compound having the structure of the following formula (I), pharmaceutically acceptable salts, prodrugs, tautomers, stereoisomers or mixtures of stereoisomers thereof,

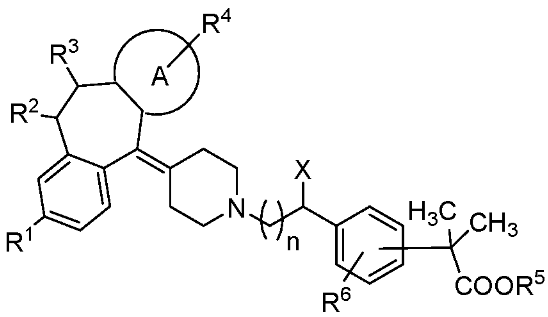

(I)

In formula (I),

X is H or OH;
n is an integer from 0-6;
Ring A is a benzene ring, a pyridine ring or a thiophene ring;
$R^1$, $R^4$ are H or halogen;
$R^2$, $R^3$ are selected from H, hydroxyl or =O;
$R^5$ is H;
$R^6$ is H, any mono- or poly-substituted $C_1$-$C_6$ alkyl, halogen, hydroxy, trifluoromethyl, cyano, oxytrifluoromethyl, methoxy, amino, nitro or carboxy. Preferably, when the alkyl group is substituted, the substituent is halogen.

Preferably, in formula (I), n is an integer of 0-4; $R^1$ is H or Cl; $R^5$ is H; $R^6$ is H, methyl, F, Cl, Br, hydroxyl, trifluoromethyl, cyano, oxytrifluoromethyl, methoxy, amino or nitro.

Preferably, the above-mentioned compound of the present invention is a compound of the following formula:

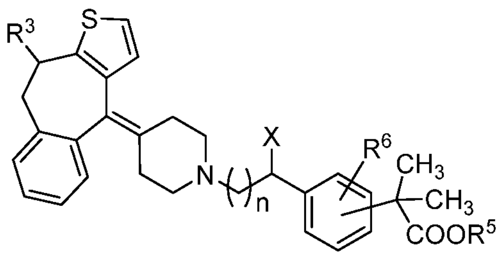

Wherein, n is an integer of 0-6; X is H or OH; $R^3$ is H, hydroxyl or =O; $R^5$ is H; $R^6$ is H, any mono- or poly-substituted $C_1$-$C_6$ alkyl, halogen, hydroxyl, trifluoromethyl, cyano, oxytrifluoromethyl, methoxy, amino, nitro or carboxy. Preferably, when the alkyl group is substituted, the substituent is halogen.

Preferably, the above-mentioned compound of the present invention is a compound of the following formula

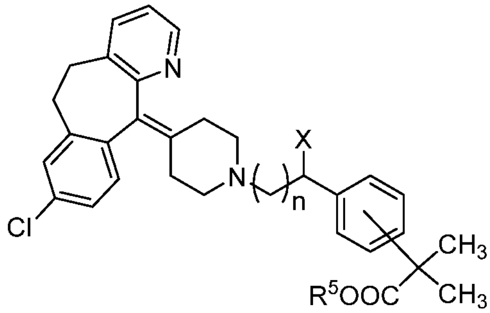

Wherein, n is an integer of 0-6; X is H or OH; $R^5$ is H.

The pharmaceutically acceptable salt of the present invention is a salt formed by a compound of formula (I) with an alkali metal, alkaline earth metal, an amino acid or a basic compound containing an amino group, or a salt formed by a compound of formula (I) with an inorganic acid or organic acid, or a complex salt formed by a compound of formula (I) with a polybasic acid and an alkali metal or alkaline earth metal.

Preferably, wherein the said pharmaceutically acceptable salt is as follows: potassium, sodium or ammonium salt of the compound of formula (I); a salt formed by compound of formula (I) and hydrochloric acid, sulfuric acid, phosphoric acid, hydrobromic acid, maleic acid, fumaric acid, citric acid, methanesulfonic acid, p-toluenesulfonic acid, tartaric acid or acetic acid; or a complex salt formed by compound of formula (I) with a polybasic acid and an alkali metal or alkaline earth metal, wherein the said polybasic acid is selected from the group consisting of citric acid, succinic acid, tartaric acid, fumaric acid, maleic acid, oxalic acid, sulfuric acid, phosphoric acid, sulfurous acid and malic acid, and the said alkali metal or alkaline earth metal is selected from sodium, potassium, calcium, magnesium and zinc.

Preferably, wherein the compound is selected from the group consisting of compound 1 to compound 42:

| Example | Structural formula | Example | Structural formula |
|---|---|---|---|
| 1 | 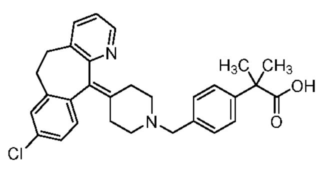 | 22 | 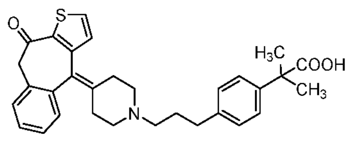 |
| 2 | 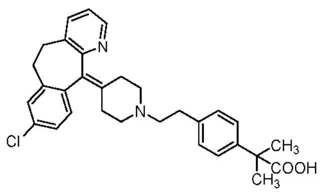 | 23 | 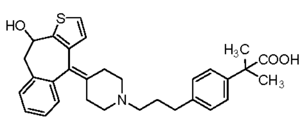 |

(continued)

| Example | Structural formula | Example | Structural formula |
|---|---|---|---|
| 3 | 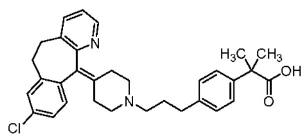 | 24 | 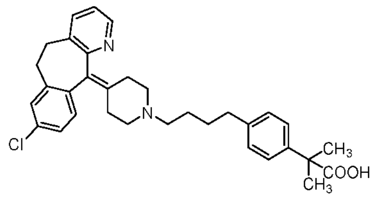 |
| 4 | 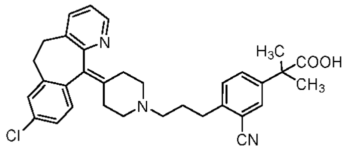 | 25 | 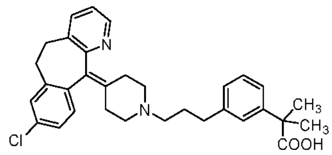 |
| 5 | 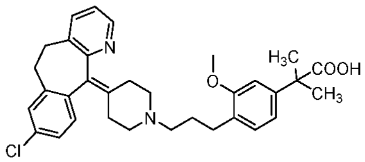 | 26 | 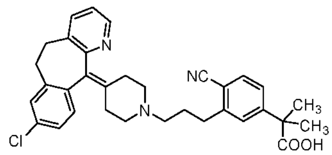 |
| 6 | 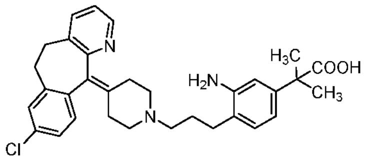 | 27 | 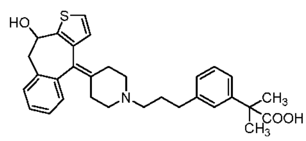 |
| 7 | 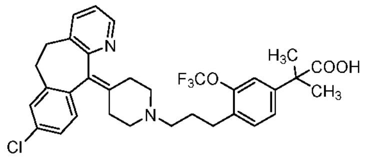 | 28 | 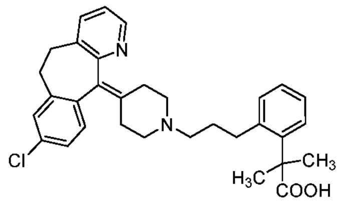 |
| 8 | 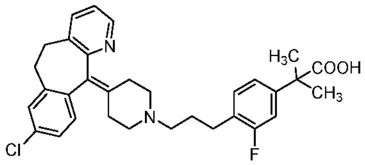 | 29 | 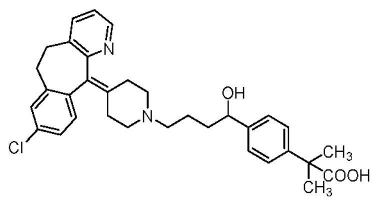 |
| 9 | 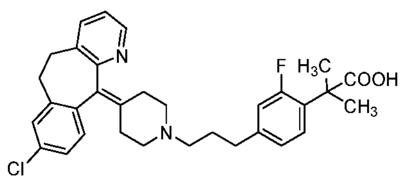 | 30 | 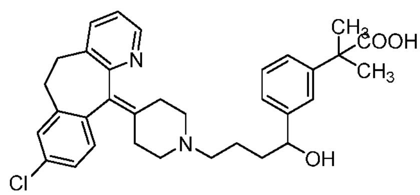 |
| 10 | 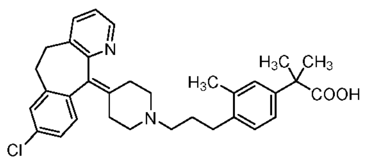 | 31 | 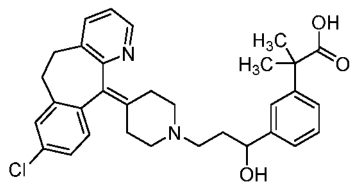 |

(continued)

| Example | Structural formula | Example | Structural formula |
|---|---|---|---|
| 11 | 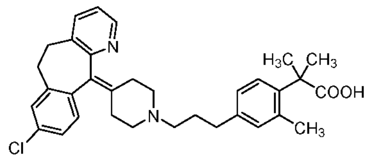 | 32 | 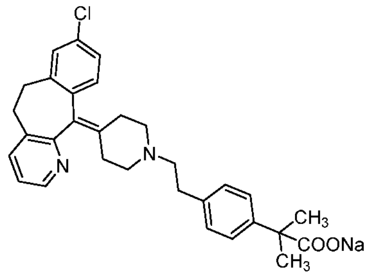 |
| 12 | 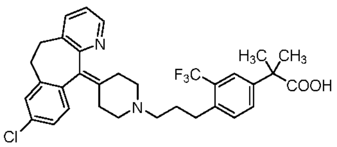 | 33 | 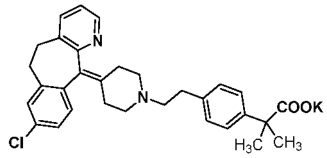 |
| 13 | 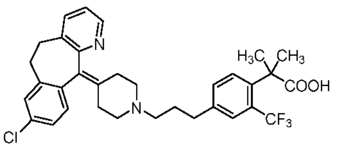 | 34 | 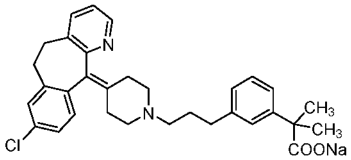 |
| 14 | 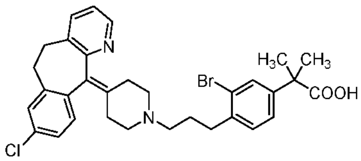 | 35 | 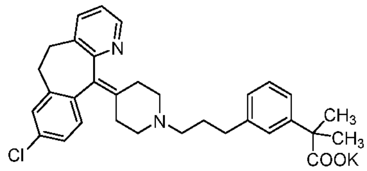 |
| 15 | 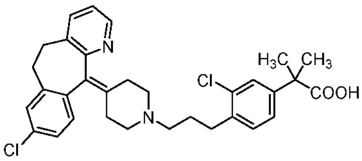 | 36 | 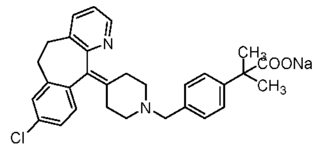 |
| 16 | 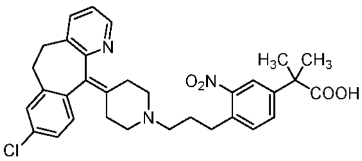 | 37 | 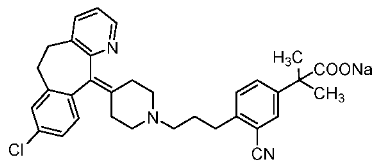 |
| 17 | 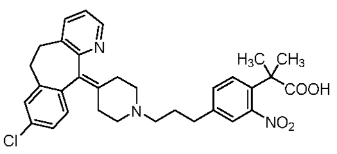 | 38 | 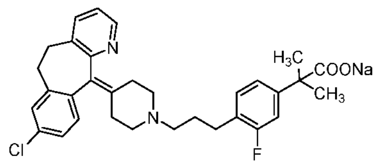 |
| 18 | 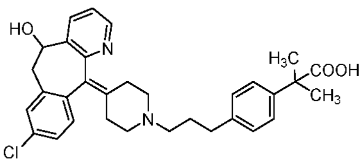 | 39 | 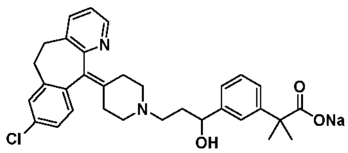 |

(continued)

| Example | Structural formula | Example | Structural formula |
|---|---|---|---|
| 19 | 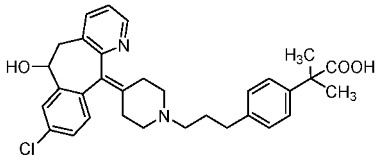 | 40 | 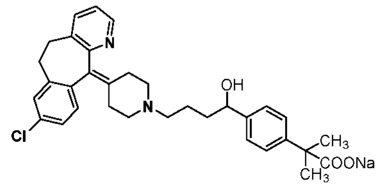 |
| 20 | 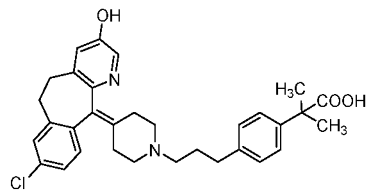 | 41 | 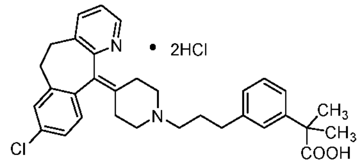 |
| 21 | 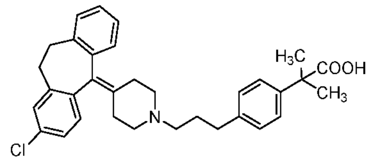 | 42 | 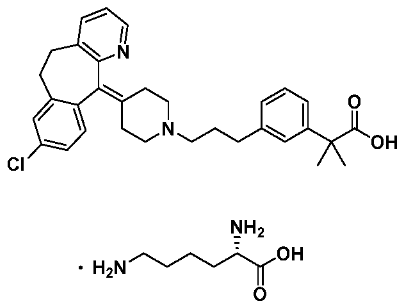 |

The compound of the present invention also includes the crystal form or solvate of the above-mentioned compound.

The present invention also provides a pharmaceutical composition, which contains the above-mentioned compound and pharmaceutically acceptable excipients. Preferably, the pharmaceutical composition is a tablet, capsule, granule, oral liquid, oral spray, suppositories, transdermal preparations, injections, nasal drops, eye drops or nasal sprays.

The present invention also provides the use of the aforementioned compounds in the preparation of antihistamine drugs. Preferably, the drugs are drugs for the prevention or treatment of allergic diseases. Preferably, the allergic diseases are selected from allergic rhinitis, urticaria, chronic urticaria, allergic purpura, asthma, allergic dermatitis, eczema, allergic conjunctivitis, atopic dermatitis, sinusitis or chronic sinusitis.

As another object of the present invention, there is also provided a method for preparing the compound of formula (I) mentioned above, including the step of reacting the compound of formula (II) to produce the compound of formula (I):

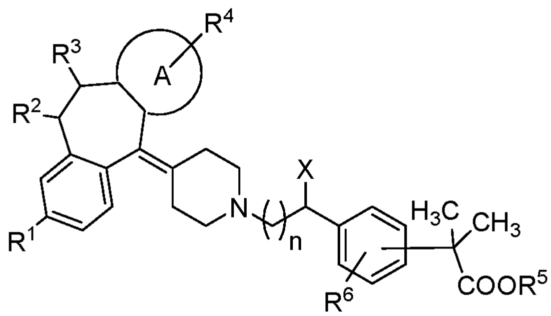

(II)

Wherein, X, n, A, $R^1$, $R^2$, $R^3$, $R^4$ and $R^6$ are defined as above, $R^5$ is $C_1$~$C_6$ alkyl.

Preferably, a compound of formula (II) with the structure shown below is provided,

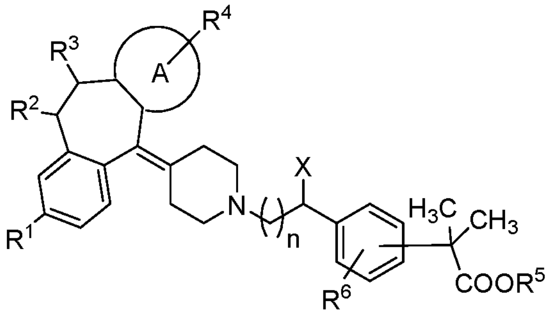

(II)

Wherein, X, n, A, $R^1$, $R^2$, $R^3$, $R^4$ and $R^6$ are defined as above, $R^5$ is $C_1$~$C_6$ alkyl.

Preferably, the compound of formula (II) is a compound of the following structure:

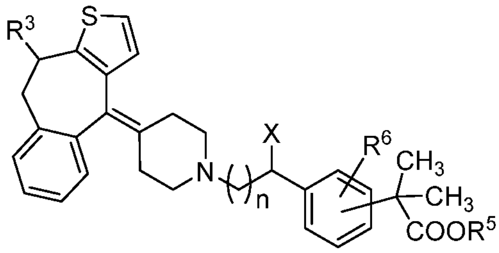

Wherein, n is an integer of 0-6; X is H or OH; $R^3$ is H, hydroxyl or =O; $R^5$ is $C_1$-$C_6$ alkyl; $R^6$ is H, any mono- or poly-substituted $C_1$-$C_6$ alkane group, halogen, hydroxyl, trifluoromethyl, cyano, oxytrifluoromethyl, methoxy, amino, nitro or carboxy. Preferably, when the alkyl group is substituted, it is substituted by halogen.

Or, the compound of formula (II) is a compound of the following structure:

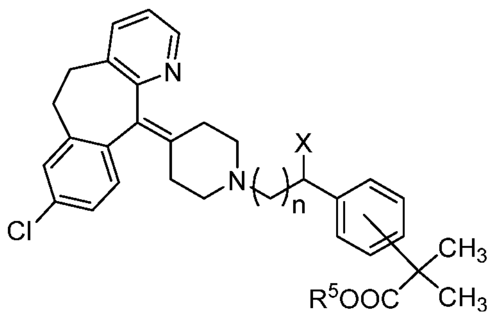

Wherein, n is an integer of 0-6; X is H or OH; $R^5$ is $C_1$-$C_6$ alkyl.

## Biological activity

### 1. The inhibitory effect of the compound of the present invention on histamine-induced vascular permeability in mice

ICR mice, weighing 18-22 g, were bred adaptively for 7 days, and were randomly divided into groups with 10 mice each group, namely the model group, the compound group of the present invention, the positive control drug desloratadine group, the compound LHC-7 group, the compound LP -2 group and the compound 1 group (the said compound 1 refers to the compound disclosed in WO9200293A1; the same below). Intragastric administration was performed 1 hour before modeling. The model group was given the corresponding solvent. The compound group of the present invention was given 5 mg/kg of the corresponding compound, and the positive control drug desloratadine and other compounds groups were given 5 mg/kg of the corresponding drug. 10% sodium sulfide was used to depilate the back of mice (3cm×3cm). The mice were injected 1 ug/0.1mL of histamine phosphate physiological saline solution into the depilation site one hour after the administration, and injected 0.25% Evans Blue solution 4ml/kg from the tail vein

immediately after the injection. After 30 minutes, the mice were dislocated and sacrificed. The blue-stained part of the skin was removed and perforated (diameter 15mm), cut into pieces and soaked in 1ml of acetone-saline (7:3) solution. After 24h, it was centrifuged (3000r/min, 10min) to take the supernatant. The absorbance of the sample was measured with an ultraviolet spectrophotometer at a wavelength of 610nm and a blank zero calibration with acetone-physiological saline solution.

The results are shown in Table 1. Compared with the model group, each compound group and desloratadine group significantly inhibited histamine-induced skin vascular permeability in mice ($P<0.01$). Compared with the control group, the inhibitory rate of the compound of the present invention is equivalent to that of desloratadine, compound LHC-7 or compound LP-2, and is better than that of compound 1 ($P<0.05$). It shows that the compound of the present invention has strong antihistamine activity.

Table 1 The inhibitory effect of the compound of the present invention on the vascular permeability of mice induced by histamin

| Example | Dose(mg/kg) | Inhibition rate (%) | Example | Dose(mg/kg) | Inhibition rate (%) |
|---|---|---|---|---|---|
| 1 | 5 | 52.22 | 19 | 5 | 53.26 |
| 2 | 5 | 53.98 | 20 | 5 | 57.95 |
| 3 | 5 | 41.66 | 21 | 5 | 42.65 |
| 4 | 5 | 65.37 | 22 | 5 | 63.28 |
| 5 | 5 | 47.93 | 23 | 5 | 66.73 |
| 6 | 5 | 53.21 | 24 | 5 | 47.65 |
| 7 | 5 | 42.56 | 25 | 5 | 66.82 |
| 8 | 5 | 34.87 | 26 | 5 | 68.15 |
| 9 | 5 | 50.60 | 27 | 5 | 48.76 |
| 10 | 5 | 41.21 | 28 | 5 | 43.76 |
| 11 | 5 | 36.86 | 29 | 5 | 64.26 |
| 12 | 5 | 44.65 | 30 | 5 | 52.22 |
| 13 | 5 | 51.02 | 31 | 5 | 47.95 |
| 14 | 5 | 42.76 | **Compound LHC-7** | 5 | 53.20 |
| 15 | 5 | 52.76 | **Compound 1** | 5 | 36.10 |
| 16 | 5 | 50.76 | **Compound LP-2** | 5 | 54.10 |
| 17 | 5 | 49.24 | **Desloratadine** | 5 | 58.46 |
| 18 | 5 | 46.58 | | | |

**2. Test results of the compound of the present invention on pilocarpine-induced salivation in rats**

Wistar male rats, 180-220g, were divided into blank group, model group, desloratadine group, compound group of the present invention, compound LHC-7 control group, compound LP-2 control group and compound 1 control group with each group 10 pieces. Rats in each group were given corresponding drugs by intragastric administration. 15 minutes later, pilocarpine (1 mg/kg, dissolved in physiological saline) was injected intraperitoneally to induce saliva secretion. The rat was immediately anesthetized by intraperitoneal injection of 10% chloral hydrate. After the rat wass anesthetized, the residual saliva in the oral cavity was taken out with a cotton ball in that the weighed cotton ball (M1) was placed in the oral cavity (two under the tongue and one for each on both sides). The cotton ball was taken out 10 minutes later and the weight of the cotton ball (M2) was immediately measured.

$$\text{Saliva secretion} = M2 - M1$$

Inhibition rate = Saliva secretion in drug administration group / Saliva secretion in model group×100%

The experimental results are shown in Table 2 below. In the model group that pilocarpine 1mg/kg was injected intraperitoneally, the saliva secretion increased significantly. The model was successful. The saliva secretion by intragastric administration of desloratadine (10mg/kg), compound LP-2 (10mg/kg), compound LHC-7 (10mg/kg) or compound 1 (10mg/kg) decreased (P< 0.01), indicating that desloratadine, compound LP-2, compound LHC-7 and compound 1 have a significant inhibitory effect on choline. The amount of saliva secreted by intragastric administration of the compound of the present invention (10 mg/kg) was not statistically different from that of the model group (P>0.05), but was a significant difference (P<0.05) compared with the positive control group. This shows that compared with compound LHC-7, compound LP-2, compound 1 and desloratadine, the compound of the present invention has very low inhibitory activity of choline.

Table 2 The inhibitory effect of the compound of the present invention on the salivation of rats induced bv pilocarpine

| Example | Dose(mg/kg) | Inhibition rate (%) | Example | Dose(mg/kg) | Inhibition rate (%) |
|---|---|---|---|---|---|
| 1 | 10 | 18.53 | 19 | 10 | 11.83 |
| 2 | 10 | 7.64 | 20 | 10 | 7.65 |
| 3 | 10 | 7.02 | 21 | 10 | 16.54 |
| 4 | 10 | 8.72 | 22 | 10 | 18.43 |
| 5 | 10 | 11.60 | 23 | 10 | 8.54 |
| 6 | 10 | 6.34 | 24 | 10 | 10.32 |
| 7 | 10 | 4.65 | 25 | 10 | 9.68 |
| 8 | 10 | 8.23 | 26 | 10 | 13.28 |
| 9 | 10 | 12.01 | 27 | 10 | 5.48 |
| 10 | 10 | 5.13 | 28 | 10 | 19.65 |
| 11 | 10 | 5.20 | 29 | 10 | 11.28 |
| 12 | 10 | 7.48 | 30 | 10 | 6.74 |
| 13 | 10 | 5.77 | 31 | 10 | 13.25 |
| 14 | 10 | 3.78 | 32 | 10 | 18.93 |
| 15 | 10 | 8.42 | **Compound LHC-7** | 10 | 95.60 |
| 16 | 10 | 10.76 | **Compound 1** | 10 | 84.30 |
| 17 | 10 | 6.54 | **Compound LP-2** | 10 | 98.40 |
| 18 | 10 | 12.50 | **Desloratadine** | 10 | 92.02 |

**3. The *h*ERG potassium channel activity test of the compound of the present invention**

Compared with the comparative compound LHC-7, compound LP-2, compound 1 and desloratadine, the compound of the present invention has a significantly reduced activity in inhibiting *h*ERG potassium channels. Especially for compounds 1-4, 7, 8, 11, 21, 26, 29 and 32, $IC_{50}$ of the inhibiting *h*ERG is more than 30μm. The risk of cardiotoxicity is small. The compound of the present invention has higher safety.

| Example | *h*ERG $IC_{50}$ (μm) |
|---|---|
| 1 | >30 |
| 2 | >30 |
| 3 | >30 |
| 4 | >30 |

(continued)

| Example | *h*ERG $IC_{50}$ ($\mu$m) |
|---|---|
| 7 | >30 |
| 8 | >30 |
| 11 | >30 |
| 21 | >30 |
| 26 | >30 |
| 29 | >30 |
| 32 | >30 |
| **Compound LHC-7** | 0.248 |
| **Compound 1** | 2.033 |
| **Compound LP-2** | 4.032 |
| **Desloratadine** | 1.214 |

## Detailed Description of the Preferred Embodiments

The general production methods of the compounds of the present invention are exemplified below. In addition, extraction or purification can be processed as usual in organic chemistry.

The synthesis of the compound of the present invention can be carried out while referring to procedures known in the art.

Commercially available compounds, compounds described in this specification, compounds described in documents cited in this specification and other known compounds can be used as the raw material compound.

In the compounds of the present invention, tautomers may exist. The present invention contains these compounds, including all possible isomers and mixtures thereof.

When the salt of the compound of the present invention is to be obtained, it can be purified directly if the compound of the present invention is obtained in the form of a salt. In addition, if the compound of the present invention is obtained in a free form, it can be dissolved or suspended in a suitable organic solvent and add an acid or a base to form a salt using a conventional method.

In addition, the compound of the present invention and pharmaceutically acceptable salt thereof may also exist in the form of adducts (hydrates or solvates) with water or various solvents, and these adducts are also included in the present invention.

### Example 1

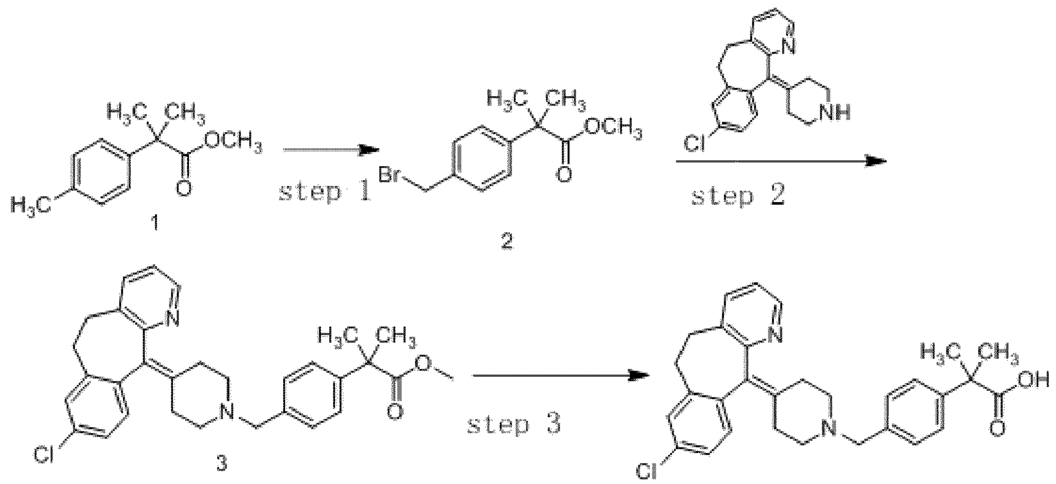


compound of example 1

Step 1 **Methyl 2-(4-(bromomethyl)phenyl)-2-methylpropionate (2)**

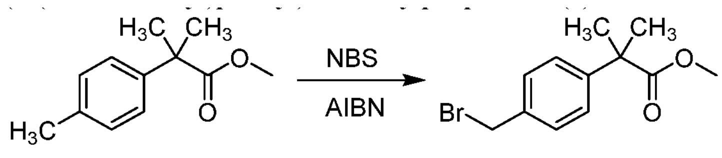


1 2

Methyl 2-(4-methylphenyl)-2-methylpropionate (200 mg, 1.04 mmol) was dissolved in $CCl_4$ (15 mL), and NBS (194 mg, 1.09 mmol) and AIBN (17 mg, 0.10 mmol) were added sequentially. Under the protection of nitrogen, react at 80 °C for 2.5 h. TLC (*V* petroleum ether: *V* ethyl acetate=20:1) monitored the reaction to be complete. The reaction solution was concentrated to dryness and purified by flash preparative chromatography (20 g, *V* petroleum ether: *V* ethyl acetate = 50:1) to obtain 242 mg of intermediate 2 with a yield of 85.8%.

Step 2 **2-(4-((4-(8-chloro-5,6-dihydro-11H-benzo[5,6]cyclohept[1,2-b]pyridine-11-enyl)piperidine-1 -yl)methyl)phenyl)-2-methylpropionic acid methyl ester (3)**

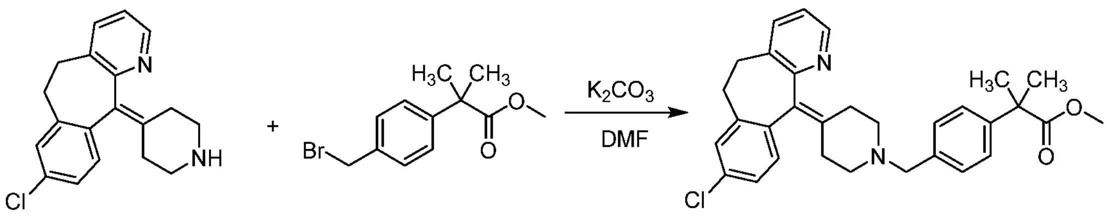


2 3

Dissolve desloratadine (240 mg, 0.77 mmol) in DMF (6 mL), then add intermediate 2 (230 mg, 0.85 mmol) and $K_2CO_3$ (267 mg, 1.93 mmol) in sequence. Under the protection of nitrogen, react at 60 °C overnight. TLC (*V* dichloromethane: *V* methanol = 20:1) detected that the reaction was complete. Cool to room temperature, add $H_2O$ (30 mL), and extract with ethyl acetate (20 mL×3). The organic phases were combined, washed with saturated brine (20 mL×3), dried with anhydrous sodium sulfate, filtered, concentrated and purified by flash preparative chromatography (20 g, *V* dichloromethane: *V* methanol = 30:1) to obtain 300 mg of light red Oily intermediate 3, the yield was 77.5%.

$^1$H NMR(400 MHz, $CDCl_3$) $\delta$(ppm) 8.39-8.37(m, 1H, ArH), 7.42(d, *J*=6.55 Hz, 1H, ArH), 7.24-7.26(m, 4H, ArH), 7.01-7.17(m, 4H, ArH), 3.64(s, 3H, O-$CH_3$), 3.47(s, 2H, ArH-$CH_2$), 3.42-3.32(m, 2H, ArH-$CH_2$), 2.87-2.69(m, 4H,

$N(CH_2)_2$), 2.52(br, 1H, ArH-CH), 2.46-2.37(m, 1H, ArH-$CH_2$), 2.36-2.29(m, 2H, $N(CH_2)_2$), 2.17-2.12(m, 1H, ArH-CH), 1.92-1.82(m, 1H, ArH-CH), 1.76-1.69(m, 1H, ArH-$CH_2$), 1.56(s, 6H, $(CH_3)_2$).

Step 3 **2-(4-((4-(8-chloro-5,6-dihydro-11H-benzo[5,6]cyclohept[1,2-b]pyridine-11-enyl)piperidine-1 -yl)methyl)phenyl)-2-methylpropionic acid (Example 1)**

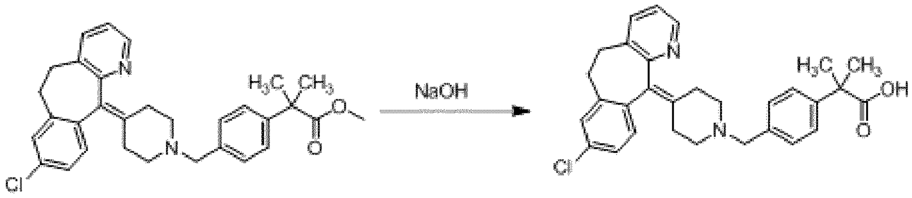


3 compound of example 1

Intermediate **3** (295 mg, 0.59 mmol) was dissolved in MeOH (8 mL), and then NaOH (94 mg, 2.35 mmol) in $H_2O$ (2 mL) was added. React overnight at 60 °C. TLC (*V* dichloromethane: *V* methanol = 20:1) detected the reaction was complete. Add water (15 mL), remove methanol under reduced pressure, adjust the pH to 4-5 with 2 N HCl solution, extract with ethyl acetate (20 mL×3 ), combine the organic phases, dry with anhydrous sodium sulfate, filter, concentrate under reduced pressure and purify by flash preparative chromatography (20 g, *V* dichloromethane: *V* methanol = 12.5:1) to obtain 120 mg of the compound of Example 1 as a white solid. The yield was 41.9%.

$^1H$ NMR (400 MHz, $CD_3OD$) $\delta$(ppm): 8.22-8.21 (m, 1H, ArH), 7.55 (d, *J*=7.81 Hz, 1H, ArH), 7.35-7.29(m, 2H, ArH), 7.27-7.22 (m, 2H, ArH), 7.18-7.12 (m, 2H, ArH), 7.10-7.05 (m, 1H, ArH), 7.03-6.98 (m, 1H, ArH), 3.80 (br, 2H, ArH-$CH_2$), 3.37-3.25 (m, 2H, ArH-$CH_2$), 3.03-2.96 (m, 2H, $N(CH_2)_2$), 2.81-2.72 (m, 2H, $N(CH_2)_2$), 2.65-2.58 (m, 2H, $N(CH_2)_2$), 2.50-2.44 (m, 2H, $N(CH_2)_2$), 2.40-2.30 (m, 1H, ArH-$CH_2$), 2.28-2.18 (m, 1H, ArH-$CH_2$), 1.42 (s, 6H, $(CH_3)_2$).

$^{13}C$ NMR (100 MHz, $CD_3OD$) $\delta$(ppm): 181.08, 156.39, 147.76, 145.75, 139.88, 138.38, 136.77, 134.48, 134.16, 133.06, 130.19, 130.07, 128.98, 126.11, 125.80, 122.92, 60.29, 53.40, 52.72, 31.12, 30.63, 28.17, 28.05, 26.28.

**Example 2**

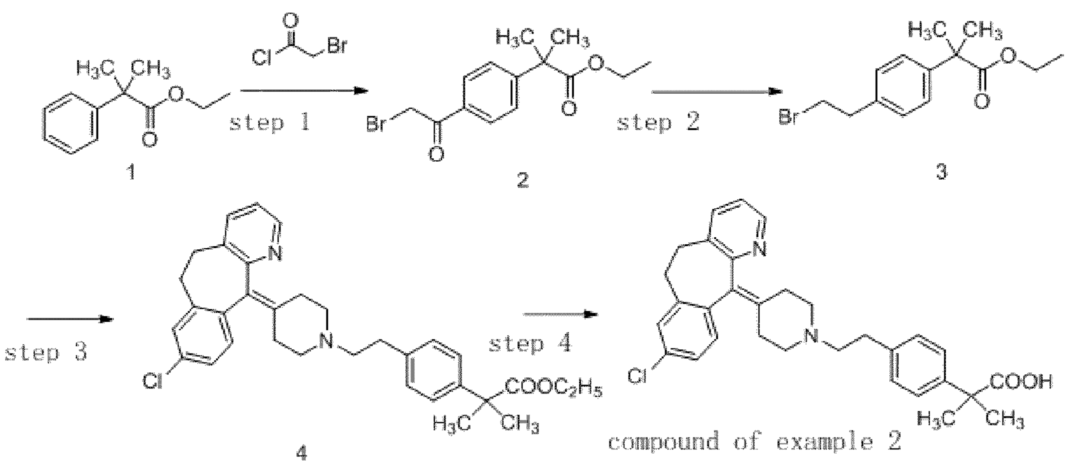


1 2 3 4 compound of example 2

**Step 1 Synthesis of ethyl 2-(4-(2-bromoacetyl)phenyl)-2-methylpropionate (2)**

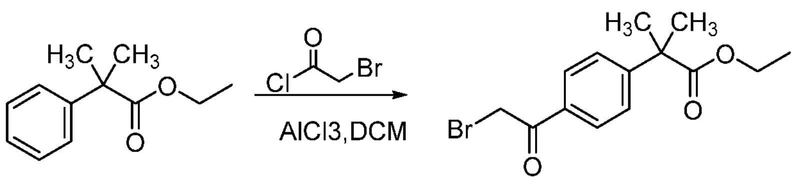


1 2

Add aluminum trichloride (6.93 g, 52.0 mmol) to dichloromethane (35 mL), protect with nitrogen, cool to -20 °C, and add **1** (5.00 g, 26.0 mmol) and bromoacetyl chloride (60, 8.18 g, 52.0 mmol) in sequence. After the addition, the temperature was raised to 0 °C and reacted for 6 h. TLC (*V* petroleum ether: Methyl acetate = 10:1) showed that the reaction was complete. Add saturated sodium bicarbonate solution to adjust the pH to 7, filter, separate the filtrate, extract the aqueous layer with dichloromethane (50 mL), combine the organic phases, wash with saturated brine (30 mL×4), dry with anhydrous sodium sulfate, filter, concentrate and purify by chromatography on silica gel column (*V* petroleum ether: *V* ethyl acetate=40:1) to obtain 4.01 g of light yellow oily intermediate **2** with a yield of 49.2%.

Step 2 **Synthesis of ethyl 2-(4-(2-bromoethyl)phenyl)-2-methylpropionate (3)**

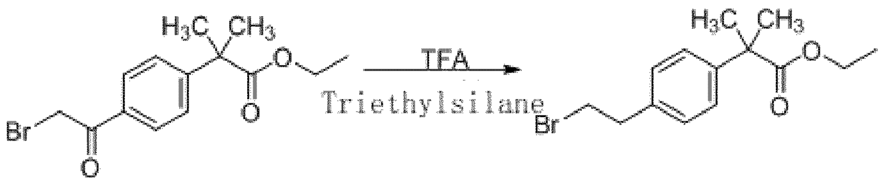


2 3

Dissolve **2** (1.00 g, 3.2 mmol) in trifluoroacetic acid (2.0 mL, 26.8 mmol), protect with nitrogen, cool to 0 °C, add triethylsilane (1.0 ml, 3.2 mmol) dropwise and stir for 30 min. Warm up to 60 °C and react overnight. TLC (*V* petroleum ether: *V* ethyl acetate = 20:1) showed that the reaction was complete. Cool in an ice water bath and add saturated sodium bicarbonate solution dropwise to adjust the pH to 7. Extract with ethyl acetate (30 mL×3), combine the organic phases, wash with saturated brine (40 mL×3) and dry with anhydrous sodium sulfate. It was filtered, concentrated and purified by silica gel column chromatography (*V* petroleum ether: *V* ethyl acetate=100:1) to obtain 520 mg of colorless oil **3** with a yield of 54.4%.

Step 3 **2-(4-(2-(4-(8-chloro-5,6-dihydro-11H-benzo[5,6]]cyclohept[1,2-b]pyridine-11-enyl) piperidin-1-yl)ethyl)phenyl)-2-methylpropionic acid ethyl ester (4)**

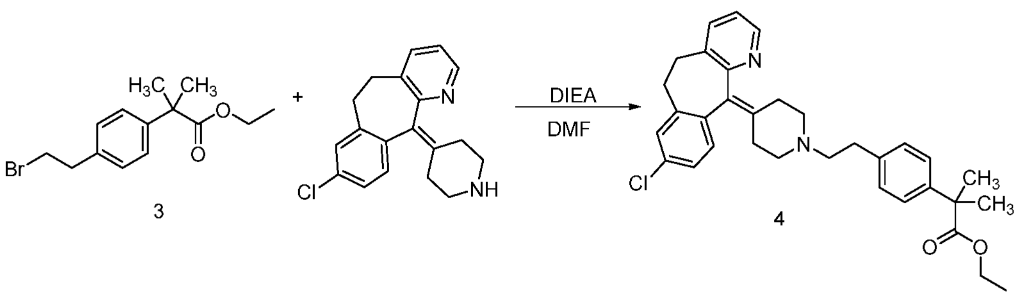


3 4

Dissolve **3** (375 mg, 1.25 mmol) in DMF (6 mL), then add desloratadine (324 mg, 1.04 mmol) and diisopropylethylamine (269 mg, 2.08 mmol) in sequence. Replace with nitrogen and react at 70 °C overnight. TLC (*V* dichloromethane: *V* methanol: MeOH=20: 1) detected that the reaction was complete. Cool in an ice-water bath, add water (15 mL) to quench the reaction, extract with ethyl acetate (20 mL×3), combine the organic phases, wash with saturated brine (30 mL×3), dry with anhydrous sodium sulfate, filter and concentrate. After separation by preparation plate (*V* dichloromethane: *V* methanol = 20:1) and purification, 307 mg of yellow solid **4** was obtained with a yield of 55.7%.

Step 4 **2-(4-(2-(4-(4-(8-chloro-5,6-dihydro-11H-benzo[5,6]]cyclohept[1,2-b]pyridine-11-enyl)piperidi n-1-yl)ethyl)phenyl)-2-methylpropionic acid (Example 2)**

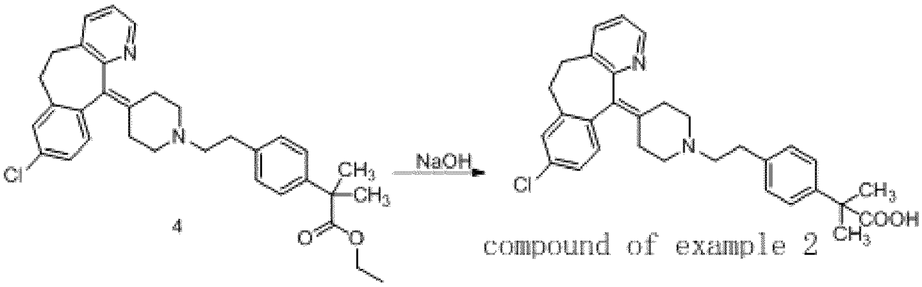


Dissolve **4** (307 mg, 0.58 mmol) in EtOH (4 mL), add $H_2O$ (2 mL) and sodium hydroxide (70 mg, 1.74 mmol), and react at 70 °C overnight. TLC (*V* dichloromethane: *V* methanol = 20:1) detected that the reaction was complete. Add 3 N dilute hydrochloric acid dropwise to adjust the pH to 3, concentrate under reduced pressure, add dichloromethane (10 mL) to dissolve, filter and filter out inorganic salts. The filtrate was concentrated and separated by a preparation plate (*V* dichloromethane: *V* methanol = 10:1) and purified to obtain 105 mg of the compound of Example 2 as a pale yellow solid, with a yield of 36.1%.

$^1$H NMR (400 MHz, $CDCl_3$) $\delta$(ppm): 8.44 (dd, $J_1$=1.6 Hz, $J_2$=4.8 Hz, 1H, ArH), 7.49 (dd, $J_1$=1.2 Hz, $J_2$=4.4 Hz, 1H, ArH), 7.38 (d, *J*=8.4 Hz, 2H, ArH), 7.17-7.12 (m, 3 H, ArH), 7.08-7.06 (m, 1H, ArH), 6.92 (d, *J*=8.4 Hz, 2H, ArH), 3.39-3.29 (m, 2H, ArH-$CH_2$), 2.69-2.57 (m, 14H, ArH-$CH_2$,$N(CH_2)_4$), 1.57 (s, 6H, $(CH_3)_2$);

**Example 3**

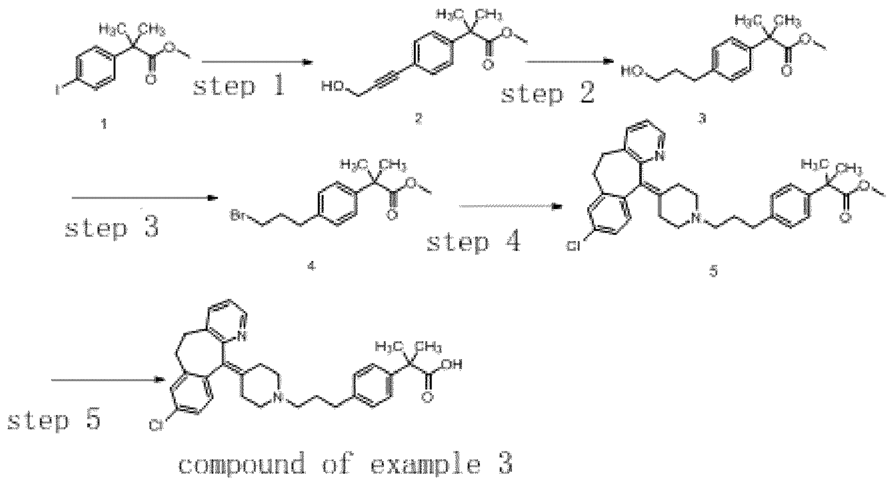


Step 1 **Methyl 2-(4-(3-hydroxypropyl-1-yn-1-yl)phenyl)-2-methylpropionate (2)**

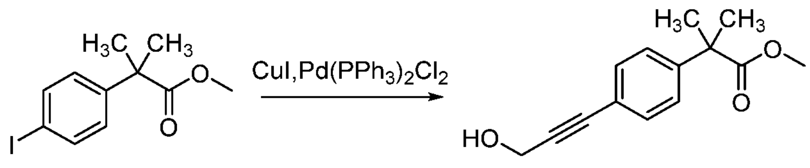


1 2

Dissolve methyl 2-(4-iodophenyl)-2-methylpropionate (1, 5.00 g, 16.44 mmol) in 50 mL of acetonitrile and then add CuI (313 mg, 1.64 mmol) and $Pd(PPh_3)\ _2Cl_2$ (577 mg, 0.82 mmol) in sequence. Protect by nitrogen, cool to -10 °C, add triethylamine (7.50 g, 73.98 mmol) dropwise, continue stirring for 10 minutes and then add propargyl alcohol (1.30 mg, 19.73 mmol). After stirring for 10 min, move to room temperature and react for 4 h. TLC (*V* petroleum ether: *V* ethyl acetate=3:1) detects the complete reaction of the raw materials. Quench with water (50 mL), extract with ethyl acetate (50 mL×2), combine the organic phases, wash with saturated brine (50 mL×4), dry with anhydrous sodium sulfate, filter, concentrate and chromatography on silica gel column (*V* petroleum ether: *V* ethyl acetate = 5:1) to purify to obtain 3.50 g of yellow solid intermediate **2**, with a yield of 91.6%.

Step 2 **Methyl 2-(4-(3-hydroxypropyl)phenyl)-2-methylpropionate** (3)

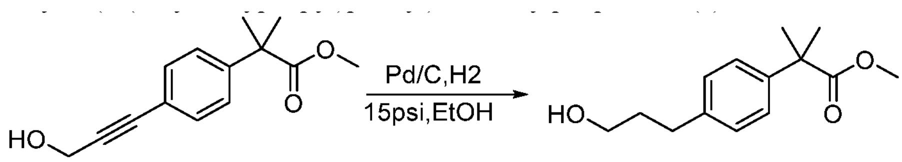


2 3

Intermediate **2** (3.50 g, 15.1 mmol) was dissolved in ethanol (35 mL), 10% Pd/C (1.05 g, 10% wt) was added, and the mixture was reacted overnight at RT under a hydrogen atmosphere (15 psi). TLC (*V* petroleum ether: *V* ethyl acetate=2:1) detected that the raw material reactionwas complete. Add celite to filter, concentrate the filtrate and purify it by column chromatography (*V* petroleum ether: *V* ethyl acetate=10:1) to obtain 1.80 g of intermediate **3** as a colorless oil with a yield of 50.5%.

$^1H$ NMR (400 MHz, $CDCl_3$) $\delta$(ppm): 7.26-7.23 (m, 2H, ArH), 7.17-7.14 (m, 2H, ArH), 3.69-3.67 (m, 2H, $ArHCH_2$), 3.65 (s, 3H, $OCH_3$), 2.70-2.66 (m, 2H, $(CH_2)_2$), 1.92-1.85 (m, 2H, $(CH_2)_2$), 1.56 (s, 6H, $(CH_3)_2$)

Step 3 **Methyl 2-(4-(3-bromopropyl)phenyl)-2-methylpropionate (4)**

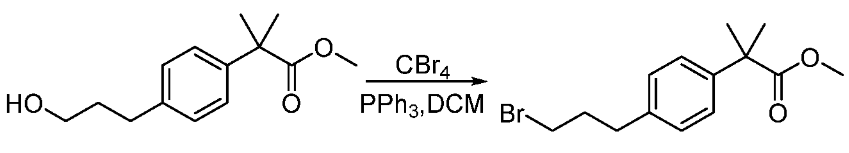


3 4

Intermediate **3** (1.80 g, 7.6 mmol) was dissolved in dichloromethane (30 mL), added $CBr_4$ (3.78 g, 11.4 mmol), protected by nitrogen, cooled to 0 °C and $PPh_3$ (3.09 g, 11.8 mmol) in dichloromethane (5 mL) solution was added dropwise. React at RT overnight. TLC (*V* petroleum ether: *V* ethyl acetate=2:1) detected that the raw material reaction was complete. It was directly concentrated and purified by silica gel column chromatography (*V* petroleum ether: *V* ethyl acetate=40:1) to obtain 1.98 g of intermediate **4** as a colorless oil, with a yield of 86.9%.

ESI-Mass m/z: 299.09 ($M^+$+H).

Step 4 **2-(4-(3-(4-(8-chloro-5,6-dihydro-11H-benzo[5,6]cyclohepta[1,2-b]pyridine-11-enyl)piperidine -1-yl)propyl)phenyl)-2- methylpropionic acid methyl ester (5)**

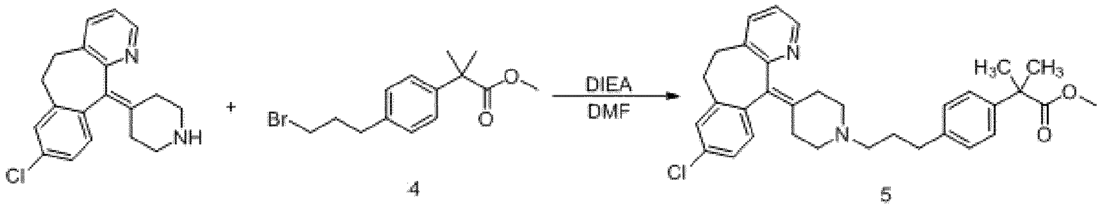


4

5

Intermediate **4** (1.98 g, 6.62 mmol) was dissolved in DMF (30 mL), desloratadine (2.47 g, 7.94 mmol) and diisopropylethylamine (1.71 g, 13.23 mmol) were added in sequence. Protect by nitrogen and react at 70 °C overnight. TLC (*V* dichloromethane: *V* methanol = 20:1) detected that the reaction was complete. Cool to room temperature, quench with water (30 mL), extract with ethyl acetate (40 mL×2), combine the organic phases, wash with saturated brine (40 mL×3), dry with anhydrous sodium sulfate, filter, concentrate and purify by flash preparative chromatography (80 g, *V* dichloromethane: *V* methanol = 50:1) to yield 0.97 g of pink solid intermediate 5 with a yield of 79.4%.

$^1H$ NMR(400 MHz, $CD_3OD$) $\delta$ (ppm): 8.30-8.32 (m, 1H, ArH), 7.65-7.63 (m, 1H, ArH), 7.26-7.21 (m, 4H, ArH), 7.18-7.11 (m, 4H, ArH), 3.63 (s, 3H, O-$CH_3$), 3.44-3.35 (m, 2H, ArH-$CH_2$), 2.87-2.80 (m, 4H, ArH-$CH_2$), 2.63-2.59 (m, 2H ArH-$CH_2$), 2.48-2.23 (m, 8H, $N(CH_2)_4$), 1.88-1.82 (m, 2H, $N(CH_2)$), 1.53 (s, 6H, $(CH_3)_2$).

Step 5 **2-(4-(3-(4-(8-chloro-5,6-dihydro-11H-benzo[5,6]cyclohept[1,2-b]pyridine-11-enyl)piperidine -1-yl)propyl)phenyl)-2-methylpropionic acid (Example 3)**

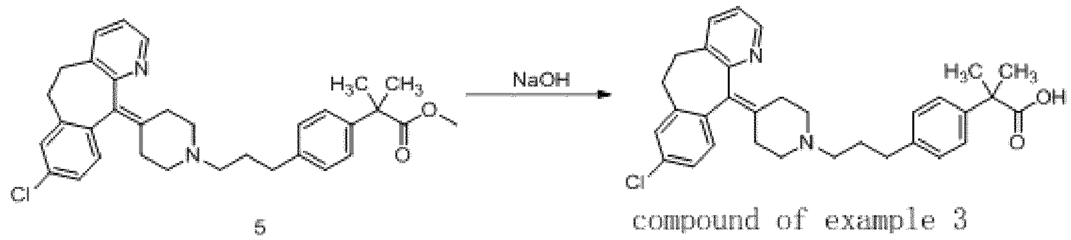


5

compound of example 3

Intermediate **5** (500 mg, 0.94 mmol) was dissolved in methanol (12 mL), and water (4 mL) and NaOH (151 mg, 3.78 mmol) were added. React at 60 °C overnight. TLC (*V* dichloromethane: *V* methanol = 10:1) detected that the reaction was complete. Concentrate to remove methanol, add water (5 mL), wash with ethyl acetate (5 mL×3), adjust the pH of the aqueous phase to 5 with 1 N dilute hydrochloric acid solution. A viscous solid was precipitated. After filtering, the filter cake was dissolved in methanol (5 mL) and dried with anhydrous sodium sulfate. Filter, concentrate, purify through the preparative plate chromatography (*V* dichloromethane: *V* methanol = 6: 1) to obtain 115 mg of the compound of Example 3, a white solid, with a yield of 23.6%.

$^1H$ NMR (400 MHz, $CD_3OD$) $\delta$ (ppm): 8.24 (dd, $J_1$=1.2 Hz, $J_2$=4.8 Hz, 1H, ArH), 7.56-7.54 (m, 1H, ArH), 7.22-7.13 (m, 4H, ArH), 7.09-6.96 (m, 4H, ArH), 3.34-3.24 (m, 2H, ArH-$CH_2$), 3.09-3.02 (m, 2H, ArH-$CH_2$), 2.82-2.35 (m, 6H, $N(CH_2)_3$), 2.56-2.47 (m, 4H, ArH-$CH_2$), 2.41-2.25 (m, 2H, $N(CH_2)$), 1.86-1.76 (m, 2H, ArH-$CH_2$), 1.38 (s, 6H, $(CH_3)_2$).

$^{13}C$ NMR (100 MHz, $CD_3OD$) $\delta$(ppm): 182.24, 155.80, 145.30, 144.88, 139.44, 137.91, 137.63, 136.23, 134.00, 133.81, 133.74, 132.60, 129.58, 128.50, 127.21, 125.33, 125.19, 122.46, 55.88, 52.26, 31.64, 30.61, 30.14, 27.51, 27.37, 26.22, 26.18, 25.65.

**Example 4 2-(4-(3-(4-(8-chloro-5,6-dihydro-11H-benzo[5,6]cyclohepta[1,2-b]pyridine-11-ylidene)piperid ine -1-yl)propyl)-3-cyano)-2-methylpropionic acid**

Refer to the synthesis method of Compound of Example 3 and replace methyl 2-(4-iodophenyl)-2-methylpropionate with methyl 2-(3-cyano-4-iodophenyl)-2-methylpropionate. 175 mg of the compound of Example 4 was prepared as a white solid with a yield of 80.0%.

$^1H$ NMR (400 MHz, $CDCl_3$) $\delta$(ppm): 8.42 (d, $J$=4.4Hz, 1H, ArH), 7.68 (d, $J$=1.6Hz, 1H, ArH), 7.57-7.60 (m, 1H, ArH), 7.47-7.46 (m, 1H, ArH), 7.16-7.12 (m, 4H, ArH), 7.04 (d, $J$=8.0Hz, 1H, ArH), 3.40-3.27 (m, 2H, ArH-$CH_2$), 2.99 (br, 2H, ArH-$CH_2$), 2.99-2.49 (m, 10H, ArH-$CH_2$, $N(CH_2)_4$), 2.33 (s, 2H, ArH-$CH_2$), 1.96-1.93 (m, 2H, ArH-$CH_2$), 1.58-1.56 (m, 6H, $(CH_3)_2$).

$^{13}C$ NMR (100 MHz, DMSO-$d_6$) $\delta$ (ppm): 181.50, 156.26, 147.01, 145.79, 142.01, 139.94, 138.41, 134.50, 134.11, 133.11, 131.23, 130.06, 129.99, 129.01, 129.00, 125.84, 122.96, 117.83, 111.42, 56.02, 52.81, 21.10, 30.69, 30.64, 28.03, 27.89, 26.26, 25.59.

### Example 5 2-(4-(3-(4-(8-chloro-5,6-dihydro-11H-benzo[5,6]cyclohepta[1,2-b]pyridine-11-enyl)piperidine -1-yl)propyl)-3-methoxyphenyl)-2-methylpropionic acid

Refer to the synthesis method of Compound of Example 3 and replace methyl 2-(4-iodophenyl)-2-methylpropionate with methyl

2-(3-methoxy-4-iodophenyl)-2-methylpropionate. 104 mg of the compound of Example 5 was prepared as a pale yellow solid with a yield of 30.8%.

$^1H$ NMR (400 MHz, $CD_3OD$)$\delta$ (ppm): 8.35 (dd, $J_1$=4.9 Hz, $J_2$=1.5 Hz, 1H, ArH), 7.67 (dd, $J_1$=7.8 Hz, $J_2$=1.4 Hz, 1H, ArH), 7.31-7.25 (m, 2H, ArH), 7.20 (dd, $J_1$=8.1 Hz, $J_2$=2.1 Hz, 1H, ArH), 7.1 (d, $J$=8.2 Hz, 1H, ArH), 7.07 (d, $J$=7.8 Hz, 1H, ArH), 6.95 (d, $J$=1.6 Hz, 1H, ArH), 6.91 (dd, $J_1$=7.8 Hz, $J_2$=1.8 Hz, 1H, ArH), 3.81 (s, 3H, O-$CH_3$), 3.48-3.35 (m, 4H, ArH-$CH_2$), 3.15-3.00 (m, 4H, $N(CH_2)_2$), 2.94-2.81 (m, 2H, ArH-$CH_2$), 2.72-2.61 (m, 4H, $N(CH_2)_2$), 2.59-2.42 (m, 2H, $(CH_2)$), 2.05-1.94 (m, 2H, $(CH_2)$), 1.52 (s, 6H, $(CH_3)_2$).

$^{13}C$ NMR (400 MHz, $CD_3OD$) $\delta$(ppm): 180.16, 157.20, 155.81, 145.83, 140.05, 138.62, 136.49, 135.37, 134.59, 133.32, 132.23, 129.85, 129.33, 129.03, 126.26, 125.89, 123.12, 117.60, 108.17, 56.30, 54.37, 52.60, 46.49, 30.98, 30.62, 27.48, 27.31, 26.55, 26.00, 24.30.

### Example 6 2-(3-Amino-4-(3-(4-(8-chloro-5,6-dihydro-11H-benzo[5,6]cyclohept[1,2-b]pyridine-11-enyl)pi peridin-1-yl)propyl)phenyl)-2-methylpropionic acid

Refer to the synthesis method of Compound of Example 3 and replace methyl 2-(4-iodophenyl)-2-methylpropionate with methyl 2-(3-amino4-iodophenyl)-2-methylpropionate. 240 mg of the compound of Example 6 was prepared with a yield of 85%.

$^1H$ NMR 400MHz ($CD_3OD$) $\delta$(ppm): 8.31 (dd, $J_1$=1.6 Hz, $J_2$=5.2 Hz, 1H, ArH), 7.63 (dd, $J_1$=2.0 Hz, $J_2$=8.0 Hz, 1H, ArH), 7.27-7.22 (m, 2H, ArH), 7.18-7.16 (m, 1H, ArH), 7.10 (d, $J$=3.6 Hz, 1H, ArH), 6.71-6.88 (m, 3H, ArH), 3.44-3.31 (m, 2H, ArH-$CH_2$), 3.00-2.96 (m, 2H, ArH-$CH_2$), 2.87-2.83 (m, 2H, ArH-$CH_2$), 2.61 (m, 2H, ArH-$CH_2$), 2.54-2.40 (m, 7H, ArH-$CH_2$, $N(CH_2)_2$), 2.37-2.28 (m, 1H, ArH-$CH_2$), 1.86-1.80 (m, 2H, $N(CH_2)_2$), 1.45 (s, 6H, $(CH_3)_2$).

$^{13}C$ NMR 100 MHz ($CD_3OD$) $\delta$(ppm): 156.70, 146.37, 145.73, 144.21, 139.83, 138.29, 136.84, 135.83, 134.51, 133.46, 132.94, 130.24, 128.96, 128.88, 125.72, 123.47, 122.85, 116.25, 114.00, 99.97, 56.40, 53.22,31.19, 30.61, 28.84, 28.74, 27.83, 26.73, 26.71, 24.92.

### Example 7 2-(4-(3-(4-(8-chloro-5,6-dihydro-11H-benzo[5,6]cyclohepta[1,2-b]pyridine-11-enyl)piperidine -1-yl)propyl)-3-(trifluoromethoxy)phenyl)-2-methylpropionic acid

Refer to the synthesis method of Compound of Example 3 and replace methyl 2-(4-iodophenyl)-2-methylpropionate with methyl 2-(3-oxotrifluoromethyl 4-iodophenyl)-2-methylpropionate. 54 mg of the compound of Example 7 was prepared as a white solid with a yield of 18.7%.

$^1H$ NMR (400 MHz, $CD_3OD$) $\delta$ (ppm): 8.32 (dd, $J_1$=5.2 Hz, $J_2$=1.6 Hz, 1H, ArH), 7.55 (dd, $J_1$=7.6 Hz, $J_2$=1.6 Hz, 1H, ArH), 7.33-7.16 (m, 6H, ArH), 7.11 (d, $J$=8.0Hz, 1H, ArH), 3.44-3.34 (m, 2H, ArH-$CH_2$), 3.20-3.11 (m, 2H, ArH-$CH_2$), 2.91-2.74 (m, 6H, $N(CH_2)_3$), 2.69-2.57 (m, 4H, ArH-$CH_2$, $N(CH_2)_4$), 2.50-2.44 (m, 1H, ArH-$CH_2$), 2.40-2.34 (m, 1H, ArH-$CH_2$), 1.94-1.87 (m, 2H,$(CH_2)$), 1.49 (s, 6H, $(CH_3)_2$).

$^{13}C$ NMR (100 MHz, $CD_3OD$) $\delta$ (ppm): 181.64, 156.26, 148.09, 147.04, 145.79, 139.94, 138.43, 136.69, F (134.53, 134.43) , 134.04, 133.14, 130.55, 130.05, 129.82, 129.00, 125.83, 124.95, 122.98, 118.20, 56.22, 52.77, 31.09, 30.63, 27.98, 27.84, 26.42, 26.39, 26.11, 24.79.

### Example 8 2-(4-(3-(4-(8-chloro-5,6-dihydro-11H-benzo[5,6]cyclohepta[1,2-b]pyridine-11-enyl)piperidine -1-yl)propyl)-3-fluorophenyl)-2-methylpropionic acid

Refer to the synthesis method of Compound of Example 3and replace methyl 2-(4-iodophenyl)-2-methylpropionate with methyl 2-(3-fluoro-4-iodophenyl)-2-methylpropionate. 60 mg of the compound of Example 8 was prepared with a yield of 19.4%.

$^1$H NMR 400MHz ($CD_3OD$) $\delta$ (ppm): 8.32 (dd, $J_1$=1.6 Hz, $J_2$=4.8 Hz, 1H, ArH), 7.65 (d, $J$=8.0 Hz, 1H, ArH), 7.28-7.06 (m, 7H, ArH), 3.45-3.35 (m, 2H, ArH-$CH_2$), 3.17 (s, 2H, ArH-$CH_2$), 2.92-2.75 (m, 6H, N($CH_2$)$_3$), 2.67-2.56 (m, 4H, ArH-$CH_2$), 2.52-2.35 (m, 2H, ArH-$CH_2$), 1.97-1.89 (m, 2H, ($CH_2$)), 1.48 (s, 6H, ($CH_3$)$_2$).

$^{13}$C NMR 100 MHz ($CD_3OD$) $\delta$ (ppm): 161.96, 159.55, 156.25, C-F(148.35, 148.28), 145.79, 139.94, 138.41, 136.70, (134.51, 134.43), 134.01, 133.13, 130.04, (129.78, 129.73), 128.99, 125.82, 124,65, 124.49, 122.96, (121.67, 121.63), 112.63, 112.39, 70.17, 56.27, 52.79, 31.09, 30.63, 27.99, 27.85, 26.44, 26.41, 25.39, 24.96.

**Eample 9 2-(4-(3-(4-(8-chloro-5,6-dihydro-11H-benzo[5,6]cyclohepta[1,2-b]pyridine-11-enyl)piperidine -1-yl)propyl)-2-fluorophenyl)-2-methylpropionic acid**

Refer to the synthesis method of Compound of Example 3and replace methyl 2-(4-iodophenyl)-2-methylpropionate with ethyl 2-(2-fluoro-4-iodophenyl)-2-methylpropionate. 231 mg of the compound of Example 9 was prepared as a white solid with a yield of 75.3%.

$^1$H NMR (400 MHz, $CD_3OD$) $\delta$ (ppm): 8.33 (d, $J$=4.28 Hz, 1H, ArH), 7.77 (br, 1H, ArH), 7.35 (br, 1H, ArH), 7.27-7.18 (m, 2H, ArH), 7.15-7.11 (m, 1H, ArH), 7.07 (br, 1H, ArH), 6.95 (d, $J$=7.05 Hz, 1H, ArH), 6.88 (d, $J$=12.09 Hz, 1H, ArH), 3.59-3.27 (m, 4H, ArH-$CH_2$), 3.03 (br, 2H, ArH-$CH_2$), 2.95-2.16 (m, 10H, ArH-$CH_2$,N($CH_2$)$_4$), 2.00 (br, 2H, ($CH_2$)), 1.42 (s, 6H, ($CH_3$)$_2$).

$^{13}$C NMR (100 MHz, $CD_3OD$) $\delta$ (ppm): 178.56, 161.58, 159.14, (140.79, 140.71), 139.57, 135.49, 133.21, 130.33, 129.38, 128.63, 126.12, 125.64, (123.35, 123.32), 114.68, 114.45, 55.46, 51.93, 42.95, 30.89, 30.14, 31.10, 26.90, 24.75, 24.40.

**Example 10 2-(4-(3-(4-(8-chloro-5,6-dihydro-11H-benzo[5,6]cyclohepta[1,2-b]pyridine-11-enyl)piperidine -1-yl)propyl)-3-methylphenyl)-2-methylpropionic acid**

Refer to the synthesis method of Compound of Example 3 and replace methyl 2-(4-iodophenyl)-2- methylpropionate with ethyl 2-(3-methyl-4-iodophenyl)-2-methylpropionate. 150 mg of the compound of Example 10 was prepared with a yield of 32.8%.

$^1$H NMR (400 MHz, $CD_3OD$) $\delta$ (ppm): 8.23 (dd, $J_1$=1.2 Hz, $J_2$=4.4 Hz, 1H, ArH), 7.56 (d, $J$=7.2 Hz, 1H, ArH), 7.18-7.14 (m, 2H, ArH), 7.10-7.01 (m, 4H, ArH), 6.88 (s, 1H, ArH), 3.34-3.25 (m, 2H, ArH-$CH_2$), 2.99 (s, 1H, ArH-$CH_2$), 2.82-2.49 (m, 10H, ArH-$CH_2$,N($CH_2$)$_4$), 2.39-2.35 (m, 1H, ArH-$CH_2$), 2.28-2.25 (m, 1H, ArH-$CH_2$), 2.17-2.14 (m, 3H, ($CH_3$)), 1.79-1.71 (m, 2H, ArH-$CH_2$), 1.37 (s, 6H, ($CH_3$)$_2$).

$^{13}$C NMR (100 MHz, $CD_3OD$) $\delta$(ppm): 156.49, 145.74, 145.17, 139.89, 138.33, 136.79, 136.41, 134.98, 134.50, 133.93, 133.03, 130.12, 128.96, 128.06, 127.39, 125.76, 123.27, 122.88, 56.75, 53.06, 46.86, 31.31, 30.62, 29.57, 28.44, 28.31, 26.58, 26.57, 25.45, 18.23.

**Example 11 2-(4-(3-(4-(8-chloro-5,6-dihydro-11H-benzo[5,6]cyclohepta[1,2-b]pyridine-11-enyl)piperidine -1-yl)propyl)-2-methylphenyl)-2-methylpropionic acid**

Refer to the synthesis method of Compound of Example 3 and replace methyl 2-(4-iodophenyl)-2- methylpropionate with ethyl 2-(2-methyl-4-iodophenyl)-2-methylpropionate. 177 mg of the compound of Example 11 was prepared as a brown solid with a yield of 46.9%.

$^1$H NMR (400 MHz, $CD_3OD$) $\delta$ (ppm): 8.34 (d, $J$=4.28 Hz, 1H, ArH), 7.67 (d, $J$=7.55 Hz, 1H, ArH), 7.33-7.24 (m, 3H, ArH), 7.22-7.17 (m, 1H, ArH), 7.15-7.10 (m, 1H, ArH), 7.01-6.94 (m, 2H, ArH), 3.47-3.36 (m, 2H, ArH-$CH_2$), 3.15-3.13 (m, 2H, ArH-$CH_2$), 2.96-2.68 (m, 6H, N($CH_2$)$_3$), 2.65-2.55 (m, 4H, ArH-$CH_2$,N($CH_2$)), 2.50-2.46 (m, 1H, ArH-$CH_2$), 2.40-2.36 (m, 1H, ArH-$CH_2$), 2.31 (s, 3H, ($CH_3$)), 2.02-1.87 (m, 2H, ArH-$CH_2$), 1.51 (s, 6H, ($CH_3$)$_2$).

$^{13}$C NMR (100 MHz, $CD_3OD$) $\delta$(ppm): 184.87, 157.69, 147.21, 144.56, 141.36, 139.86, 139.45, 138.13, 137.77, 135.93, 135.75, 135.60, 134.53, 132.65, 131.49, 130.44, 127.26, 126.74, 126.62, 124.40, 57.78, 54.22, 33.41, 32.53, 32.06, 29.55, 29.41, 28.17, 27.65, 21.12.

**Example 12 2-(4-(3-(4-(8-chloro-5,6-dihydro-11H-benzo[5,6]cyclohept[1,2-b]pyridyl-11-enyl)piperidine-1 -yl))propyl)-3-(trifluoromethyl)phenyl)-2-methylpropionic acid**

Refer to the synthesis method of Compound of Example 3 and replace methyl 2-(4-iodophenyl)-2-methylpropionate with ethyl

2-(3-trifluoromethyl-4-iodophenyl)-2-methylpropionate. 95 mg of the compound of Example 12 was prepared as a white solid with a yield of 24.9%.

$^1$H NMR (400 MHz, $CD_3OD$) $\delta$ (ppm): 8.32 (dd, $J_1$=1.2 Hz, $J_2$=5.2 Hz, 1H, ArH), 7.67-7.63 (m, 2H, ArH), 7.56-7.54 (m, 1H, ArH), 7.27-7.22 (m, 3H, ArH), 7.18-7.16 (m, 1H, ArH), 7.11 (d, $J$=4.0 Hz, 1H, ArH), 3.44-3.34 (m, 2H,

ArH-$CH_2$), 3.13-3.05 (m, 2H, ArH-$CH_2$), 2.91-2.67 (m, 8H,$N(CH_2)_4$), 2.61-2.55 (m, 2H, ArH-$CH_2$), 2.49-2.33 (m, 2H, ArH-$CH_2$), 1.93-1.85 (m, 2H, ArH-$CH_2$), 1.50 (s, 6H, $(CH_3)_2$).

$^{13}C$ NMR (100 MHz, $CD_3OD$) $\delta$ (ppm): 182.30, 156.42, 146.60, 145.76, 139.91, 138.37, (136.83, 136.77), 134.72, 134.51, (134.08, 133.05), 130.50, 130.12, 129.82, 129.00, 127.51, 127.22, 125.80, 123.05, 122.93, 56.46, 52.94, 52.92, 31.13, 30.63, 28.97, 28.29, 28.16, 26.55.

**Example 13 2-(4-(3-(4-(8-chloro-5,6-dihydro-11H-benzo[5,6]cyclohept[1,2-b]pyridyl-11-enyl)piperidine-1 -yl)) propyl)-2-(trifluoromethyl)phenyl)-2-methylpropionic acid**

Refer to the synthesis method of Compound of Example 3 and replace methyl 2-(4-iodophenyl)-2-methylpropionate with ethyl

2-(2-trifluoromethyl-4-iodophenyl)-2-methylpropionate. 72 mg of the compound of Example 13 was prepared as a white solid with a yield of 18.9%.

$^1H$ NMR (400 MHz, $CD_3OD$) $\delta$ (ppm): 8.36 (dd, $J_1$=1.2 Hz, $J_2$=4.8 Hz, 1H, ArH), 7.68 (dd, $J_1$=1.6 Hz, $J_2$=11.6 Hz, 1H, ArH), 7.61-7.44 (m, 3H, ArH), 7.31-7.27 (m, 2H, ArH), 7.22-7.13 (m, 2H, ArH), 3.45-3.38 (m, 2H, ArH-$CH_2$), 3.25-3.21 (m, 2H, ArH-$CH_2$), 2.95-2.83 (m, 6H, ArH-$CH_2$, $N(CH_2)_3$), 2.77-2.60 (m, 4H, ArH-$CH_2$), 2.55-2.41 (m, 2H, ($CH_2$)), 2.07-1.98 (m, 2H, ($CH_2$)), 1.61 (s, 6H, $(CH_3)_2$).

$^{13}C$ NMR (100 MHz, $CD_3OD$) $\delta$(ppm): 181.33, 156.14, 145.81, 141.94, 139.96, 139.36, 138.47, 136.64, 134.69, 134.52, 133.52, 133.17, 131.48, 129.98, 129.02, 128.18, (128.09, 128.04, 127.88, 127.85), 126.64, 125.85, 123.92, 123.01, 56.04, 52.75, 52.72, 47.11, 31.53, 31.06, 30.61, 27.83, 27.67, 25.69.

**Example 14 2-(3-Bromo-4-(3-(4-(8-chloro-5,6-dihydro-11H-benzo[5,6]cyclohept[1,2-b]pyridine-11-enyl)p iperidin-1-yl)propyl)phenyl)-2-methylpropionic acid**

Refer to the synthesis method of Compound of Example 3 and replace methyl 2-(4-iodophenyl)-2-methylpropionate with ethyl 2-(3-bromo-4-iodophenyl)-2-methylpropionate. 85 mg of the compound of Example 14 was prepared as a pale yellow solid with a yield of 40%.

$^1H$ NMR 400MHz ($CD_3OD$) $\delta$ (ppm): 8.23 (dd, $J_1$=1.2Hz, $J_2$=4.8Hz, 1H, ArH), 7.54 (dd, $J_1$=1.6Hz, $J_2$=7.6Hz, 1H, ArH), 7.47 (d, $J$=2.0Hz, 1H, ArH), 7.22-7.14 (m, 3H, ArH), 7.07 (dd, $J_1$=2.0Hz, $J_2$=8.0$H_Z$, 1H, ArH), 7.03-7.01 (m, 2H, ArH), 3.35-3.24 (m, 2H, ArH-$CH_2$), 3.13-3.06 (m, 2H, ArH-$CH_2$), 2.82-2.70 (m, 6H, ArH-$CH_2$,$N(CH_2)_2$), 2.60 (t, $J$=7.6Hz, 2H, N($CH_2$)), 2.55-2.49 (m, 2H, N($CH_2$)), 2.43-2.39 (m, 1H, ArH-$CH_2$), 2.33-2.28 (m, 1H, ArH-$CH_2$), 1.84-1.77 (m, 2H, ArH-$CH_2$), 1.38 (s, 6H, $(CH_3)_2$).

$^{13}C$ NMR 100 MHz ($CD_3OD$) $\delta$(ppm): 181.75, 156.19, 147.93, 145.80, 139.96, 138.46, 137.24, 136.66, 134.52, 133.84, 133.09, 130.04, 129.97, 129.67, 129.01, 125.85, 125.40, 125.35, 123.56, 123.00, 56.08, 52.72, 32.33, 31.09, 30.63, 27.89, 27.74, 26.39, 26.37, 24.67.

**Example 15 2-(3-chloro-4-(3-(4-(8-chloro-5,6-dihydro-11H-benzo[5,6]cyclohept[1,2-b]pyridine-11-enyl)pi peridin-1-yl)propyl)phenyl)-2-methylpropionic acid**

Refer to the synthesis method of Compound of Example 3 and replace methyl 2-(4-iodophenyl)-2-methylpropionate with ethyl 2-(3-chloro-4-iodophenyl)-2-methylpropionate. 100 mg of the compound of Example 15 was prepared as a pale yellow solid with a yield of 33%.

$^1H$ NMR 400MHz ($CD_3OD$) $\delta$ (ppm): 8.33 (dd, $J_1$=1.6Hz, $J_2$=4.8Hz, 1H, ArH), 7.65 (dd, $J_1$=1.6Hz, $J_2$=7.6Hz, 1H, ArH), 7.40 (d, $J$=2.0Hz, 1H, ArH), 7.29-7.24 (m, 3H, ArH), 7.07 (dd, $J_1$=2.4Hz, $J_2$=8.4Hz, 1H, ArH), 7.12 (d, $J$=8.0Hz, 1H, ArH), 3.45-3.35 (m, 2H, ArH-$CH_2$), 3.20-3.17 (m, 2H, ArH-$CH_2$), 2.93-2.80 (m, 6H, ArH-$CH_2$, $N(CH_2)_2$), 2.70 (t, $J$=8.0Hz, 2H, ArH-$CH_2$), 2.67-2.59 (m, 2H, N($CH_2$)), 2.53-2.48 (m, 1H, ArH-$CH_2$), 2.43-2.37 (m, 1H, ArH-$CH_2$), 1.96-1.88 (m, 2H, ArH-$CH_2$), 1.49 (s, 6H, $(CH_3)_2$).

$^{13}C$ NMR 100 MHz ($CD_3OD$) $\delta$(ppm): 181.75, 156.19, 147.93, 145.80, 139.96, 138.46,137.24, 136.66, 134.52, 133.84, 133.09, 130.04, 129.97, 129.67, 129.01, 125.85, 125.40, 125.35, 123.56, 123.00, 56.08, 52.72, 32.33, 31.09, 30.63, 27.89, 27.74, 26.39, 26.37, 24.67;

**Example 16 2-(4-(3-(4-(8-chloro-5,6-dihydro-11H-benzo[5,6]cyclohepta[1,2-b]pyridine-11-enyl)piperidine -1-yl)propyl)-2-nitrophenyl)-2-methylpropionic acid**

Refer to the synthesis method of Compound of Example 3 and replace methyl 2-(4-iodophenyl)-2-methylpropionate with ethyl 2-(2-nitro-4-iodophenyl)-2-methylpropionate. 260 mg of the compound of Example 16 was prepared as a white solid with a yield of 72.0%.

$^1H$ NMR (400 MHz, $CD_3OD$) $\delta$ (ppm): 8.34 (d, $J$=4.8 Hz, 1H, ArH), 7.75 (s, 1H, ArH), 7.67 (d, $J$=3.6 Hz, 1H,

ArH), 7.62 (d, $J$=8.0 Hz, 1H, ArH), 7.51 (dd, $J_1$=1.6 Hz, $J_2$=8.4 Hz, 1H, ArH), 7.25-7.29 (m, 2H, ArH), 7.20 (d, $J$=8.4 Hz, 1H, ArH), 7.14 (d, $J$=8.0 Hz, 1H, ArH), 3.27-3.45 (m, 4H, ArH-$CH_2$), 2.48-3.01 (m, 12H, ArH-$CH_2$,$N(CH_2)_4$), 2.03-2.06 (m, 2H, ArH-$CH_2$), 1.61 (s, 6H, $(CH_3)_2$).

$^{13}$C NMR (100 MHz, $CD_3OD$) $\delta$(ppm): 179.00, 155.05, 148.47, 144.79, 139.23, 138.98, 137.77, 137.48, 135.58, 133.85, 133.53, 132.16, 132.08, 131.54, 128.95, 128.00, 127.36, 124.84, 123.53, 122.01, 54.74, 51.47, 45.75, 30.23, 30.02, 29.59, 26.47, 26.28, 24.23.

**Example 17 2-(4-(3-(4-(8-chloro-5,6-dihydro-11H-benzo[5,6]cyclohepta[1,2-b]pyridine-11-enyl)piperidine -1-yl)propyl)-3-nitrophenyl)-2-methylpropionic acid**

Refer to the synthesis method of Compound of Example 3 and replace methyl 2-(4-iodophenyl)-2-methylpropionate with ethyl 2-(3-nitro-4-iodophenyl)-2-methylpropionate. 115 mg of the compound of Example 17 was prepared as a pale yellow solid with a yield of 13%.

$^1$H NMR 400MHz ($CD_3OD$) $\delta$ (ppm): 8.33 (dd, $J_1$=1.6Hz, $J_2$=4.8Hz, 1 H), 7.96 (d, $J$=2.0Hz, 1 H), 7.85 (dd, $J_1$=1.2Hz, $J_2$=7.6Hz, 1 H), 7.60 (dd, $J_1$=2.0Hz, $J_2$=8.4Hz, 1 H), 7.24-7.29 (m,3 H), 7.18 (dd, $J_1$=2.0Hz, $J_2$=8.0Hz, 1 H), 7.12 (d, $J$=8.0Hz, 1 H), 3.36-3.45 (m, 2 H), 3.23-3.27 (m, 2 H), 2.79-2.99 (m, 8 H), 2.62-2.69 (m ,2 H), 2.49-2.55 (m, 1 H), 2.39-2.46 (m, 1 H), 1.92-1.98 (m, 2 H), 1.52 (s,6 H);

$^{13}$C NMR 100 MHz ($CD_3OD$) $\delta$(ppm): 181.31, 156.21, 148.89, 147.91, 145.80, 139.95, 138.43, 136.67, 134.52, 133.84, 133.14, 132.82, 131.20, 131.03, 130.02, 128.99, 125.84, 122.97, 121.82, 56.17, 52.73, 31.08, 30.63, 29.25, 27.87, 27.72, 26.23, 26.21, 25.48.

**Example 18 2-(4-(3-(4-(8-chloro-5-hydroxy-5,6-dihydro-11H--benzo[5,6]cyclohepta[1,2-b]pyridine-11-en yl)piperidin-1-yl)propyl)-3-nitrophenyl)-2-methylpropionic acid**

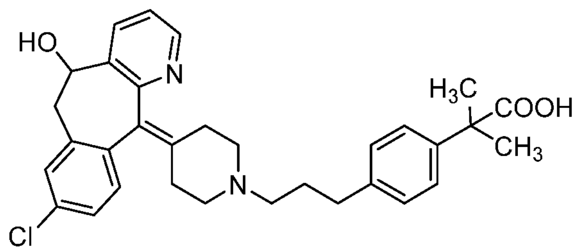

Refer to the synthesis method of Compound of Example 4 and replace desloratadine with 5-hydroxydesloratadine. 43 mg of the compound of Example 18 was prepared as a pale yellow solid with a yield of 10.2%.

ESI-HRMS: m/z calcd for $C_{32}H_{34}ClN_3O_4$ $[M+H]^+$ 531.2414, found 531.2408.

**Example 19 2-(4-(3-(4-(8-chloro-6-hydroxy-5,6-dihydro-11H--benzo[5,6]cyclohepta[1,2-b]pyridine-11-en yl)piperidin-1-yl)propyl)-3-nitrophenyl)-2-methylpropionic acid**

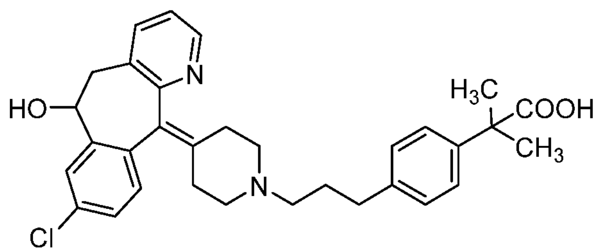

Refer to the synthesis method of Compound of Example 4 and replace desloratadine with 5-hydroxydesloratadine. 71 mg of the compound of Example 19 was prepared as a pale yellow solid with a yield of 26.1%.

ESI-HRMS: m/z calcd for $C_{32}H_{34}ClN_3O_4$ $[M+H]^+$ 531.2414, found 531.2407.

**Example 20 2-(4-(3-(4-(8-chloro-3-hydroxy-5,6-dihydro-11H--benzo[5,6]cyclohepta[1,2-b]pyridine-11-en yl)piperidin-1-yl)propyl)-3-nitrophenyl)-2-methylpropionic acid**

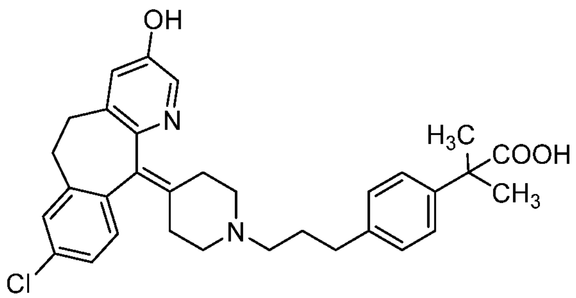

Refer to the synthesis method of Compound of Example 3 and replace desloratadine with 5-hydroxydesloratadine. 53 mg of the compound of Example 20 was prepared as a pale yellow solid with a yield of 14.8%.

ESI-HRMS: m/z calcd for $C_{32}H_{34}ClN_3O_4$ $[M+H]^+$ 531.2414, found 531.2412.

**Example 21 2-(4-(3-(4-(2-chloro-10,11-dihydro-5H-dibenzo[a,d] [7]cycloheptenyl-naphthylene)piperidine-1- yl) propyl) phenyl)-2-methylpropionic acid**

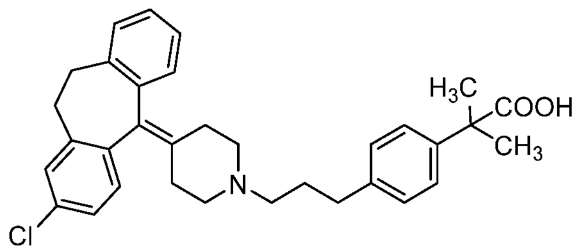

Refer to the synthesis method of Compound of Example 3 and replace desloratadine with 4-(2-chloro-10,11-dihydro-5H-dibenzo[a,d] [7]cycloheptenyl-5-alkylene)piperidine. 36 mg of the compound of Example 21 was prepared as a pale yellow solid with a yield of 10.4%.

HRMS(ESI): m/z Calcd for $C_{33}H_{36}ClNO_2$ $[M+H]^+$ 513.2435, found 513.2346.

**Example 22 2-Methyl-2-(4-(3-(4-(10-oxo-9,10-dihydro-4H-benzo[4,5]cyclohepta[1,2-b]thiophene-4 -alkylene) piperidin 1-yl) propyl) phenyl) propionic acid**

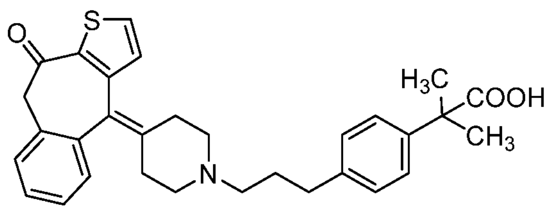

Refer to the synthesis method of Compound of Example 3 and replace desloratadine with 4-(piperidine-4-alkylene)-4,9-dihydro-10H-benzo[4,5]cyclohepta[1,2-b]thiophene-10-ketone. 63 mg of the compound of Example 22 was obtained as a pale yellow solid with a yield of 43.2%.

HRMS(ESI): m/z Calcd for $C_{31}H_{33}NO_3S[M+H]^+$ 499.2181, found 499.2178.

**Example 23 2-(4-(3-(4-(10-hydroxy-9,10-dihydro-4H-benzo[4,5]cyclohept[1,2-b]thiophen-4-alkylene)pipe ridin-1-yl) propyl) phenyl)-2-methylpropionic acid**

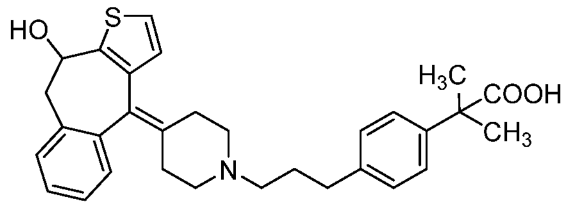

Refer to the synthesis method of Compound of Example 3 and replace desloratadine with 4-(piperidine-4-alkylene)-9,10-dihydro-4H-benzo[4,5]cyclohepta[1,2-b]thiophene-10-alcohol. 92 mg of the compound of Example 23 was obtained as a pale yellow solid with a yield of 51.6%.

HRMS(ESI): m/z Calcd for $C_{31}H_{35}NO_3S$ $[M+H]^+$501.2338, found 501.2346.

**Example 24**

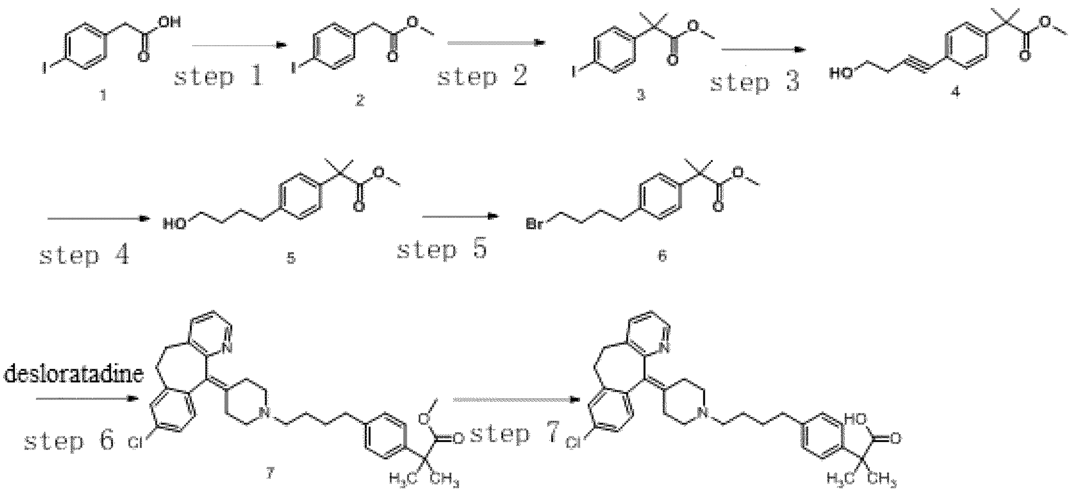


compound of example 24

Step 1 **Methyl 2-(4-iodophenyl)acetate (2)**

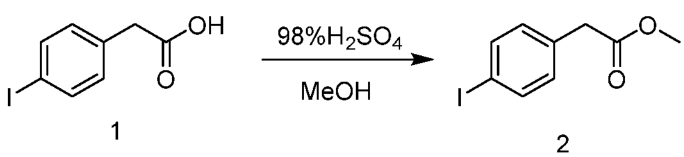


1

2

Dissolve **1** (10.0 g, 38.2 mmol) in methanol (100 mL), add concentrated sulfuric acid (3.74 g, 38.2 mmol) dropwise to the above reaction solution under stirring at room temperature and react at 65 °C for 3 h under nitrogen protection. TLC (*V* petroleum ether: *V* ethyl acetate=4:1) detects the end of the reaction. Concentrate, add ice water (100 mL), extract with ethyl acetate (50 mL×3), collect the organic phase, wash with saturated sodium chloride solution (50 mL×3) and combine the organic phases. Dry with anhydrous sodium sulfate, filter with suction, concentrate and purify by flash preparative chromatography (120 g, *V* petroleum ether: *V* ethyl acetate = 98: 2-95: 5) to obtain 10.0 g of colorless oil 2 with a yield of 95.0 %.

Step 2 **Methyl 2-(4-iodophenyl)-2-methylpropionate (3)**

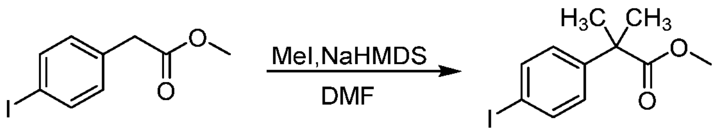


2 3

Intermediate **2** (9.00 g, 32.6 mmol) was dissolved in DMF (150 mL) and the temperature was reduced to -20 °C under nitrogen protection. NaHMDS (2 M in THF, 17.9 g, 97.8 mmol) in tetrahydrofuran solution (49 mL) was slowly added dropwise to the above reaction solution and the reaction was incubated for 30 min. Continue to add methyl iodide (18.5 g, 130.4 mmol) dropwise. After the addition was complete, keep it for 15 minutes and then transfer to room temperature to react for 7 hours. TLC (*V* petroleum ether: *V* ethyl acetate = 10:1) monitored the end of the reaction. The reaction solution was poured into a rapidly stirring 1 M citric acid (200 mL) ice water solution. Extract with ethyl acetate (100 mL×4), wash with saturated sodium chloride solution (300 mL×3) and collect the organic phase. It was dried with anhydrous sodium sulfate, filtered with suction, concentrated under reduced pressure and purified by silica gel column chromatography (*V* petroleum ether: *V* ethyl acetate=20:1) to obtain 9.63 g of yellow oil **3** with a yield of 97.1%.

$^1$H NMR (400 MHz, $CDCl_3$) $\delta$(ppm): 7.58-7.55 (m, 2H, ArH), 7.02-6.99 (m, 2H, ArH), 3.57 (s, 3H, $OCH_3$), 1.47 (s, 6H, $(CH_3)_2$).

Step 3 **Methyl 2-(4-(4-hydroxy-1-yn-1-yl)phenyl)-2-methylpropionate (4)**

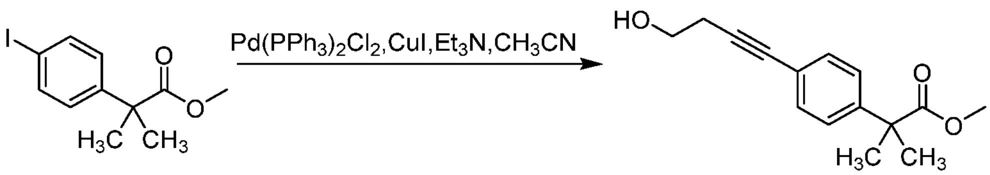


3 4

Dissolve **3** (5.38 g, 17.7 mmol) in acetonitrile (80 mL), then add cuprous iodide (270 mg, 1.42 mmol) and $Pd(PPh_3)_2Cl_2$ (497 mg, 0.71 mmo) in sequence. Stir at -10 °C for 20 min under nitrogen protection. Triethylamine (8.06 g, 79.7 mmol) and propargyl butanol (1.49 g, 21.2 mmol) were successively added dropwise to the above reaction solution. Stir at -10 °C for 10 min and transfer to room temperature to react overnight. TLC (*V* petroleum ether: *V* ethyl acetate=10:1) monitored the reaction was over. Concentrate, add ethyl acetate (50 mL) and stir for 0.5 h, suction filter under a celite pad, concentrate the filtrate and purify by silica gel column chromatography (*V* Dichloromethane: *V* methanol=100: 1) to obtain 5.12 g of yellow oil 4 with a yield of 98%.

Step 4 **Methyl 2-(4-(4-hydroxybutyl)phenyl)-2-methylpropionate (5)**

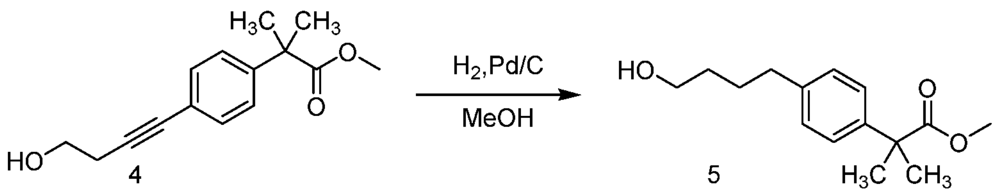


4 5

Dissolve **4** (5.12 g, 85% purity, 17.7 mmol) in methanol (150 mL), add 10% Pd/C (1.74 g, 40% Wt), replace with hydrogen three times and react at room temperature overnight (15 psi). TLC (*V* petroleum ether: *V* ethyl acetate=4:1) monitored the completion of the reaction. Suction filter under a celite pad, concentrate and purify through rapid preparative

chromatography (80 g, *V* petroleum ether: *V* ethyl acetate=100:1- 78: 22) to obtain 3.00 g of a colorless oil **5** with a yield of 67.7%.

$^1$H NMR (400 MHz, $CDCl_3$) $\delta$ (ppm): 7.26-7.22 (m, 2H, ArH), 7.15-7.13(m, 2H, ArH), 3.67-3.64 (m, 5H, $OCH_3$, ArH-$CH_2$), 2.62 (t, *J*=7.2 Hz, 2H, $CH_2OH$), 1.61-1.73 (m, 4H, $(CH_2)_2$), 1.56 (s, 6H, $(CH_3)_2$).

Step 5 **Methyl 2-(4-(4-bromobutyl)phenyl)-2-methylpropionate (6)**

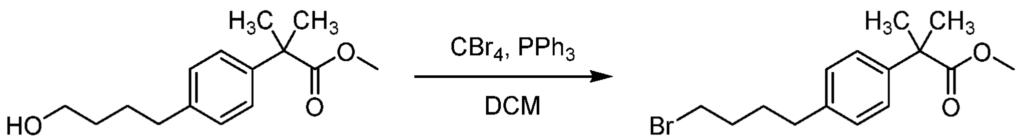


5 6

Dissolve **5** (1.00 g, 4.00 mmol) in dichloromethane (20 mL), add carbon tetrabromide (1.99 g, 6.00 mmol), slowly add triphenylphosphine (1.63 g, 6.20 mmol) under nitrogen protection and react overnight at room temperature. TLC (*V* petroleum ether: *V* ethyl acetate=10:1) monitored the completion of the reaction. Concentrate under reduced pressure and purify by silica gel column chromatography (*V* petroleum ether: *V* ethyl acetate=20:1-10:1) to obtain 1.15 g of light yellow oil **6** with a yield of 91.8%.

Step 6 **2-(4-(4-(4-(8-chloro-5,6-dihydro-11H-benzo[5,6]cyclohept[1,2-b]pyridine-11-enyl)piperidine -1-yl)butyl)phenyl)-2-methylpropionic acid methyl ester (7)**

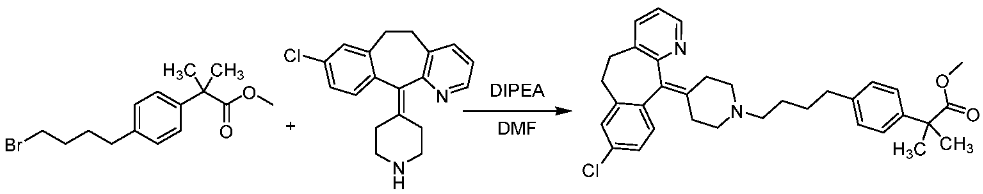


6 7

Dissolve **6** (1.15 g, 3.67 mmol) in DMF (17 mL), then add desloratadine (0.95 g, 3.06 mmol) and diisopropylethylamine (0.79 g, 6.12 mmol) in sequence. Under nitrogen, react overnight at 68 °C. TLC (*V* dichloromethane: *V* methanol=20:1) detected that the reaction was complete. The reaction solution was cooled to room temperature. Add water (60 mL), extract with ethyl acetate (20 mL×4),collect the organic phase and wash with saturated sodium chloride solution (50 mL×3). The organic phases were combined and dried over anhydrous sodium sulfate. Filter with suction, concentrate under reduced pressure and purify by flash preparative chromatography (40 g, *V* dichloromethane: *V* methanol = 97.5: 2.5) to obtain 787 mg of off-white solid 7 with a yield of 47.4%.

Step 7 **2-(4-(4-(8-Chloro-5,6-dihydro-11H-benzo[5,6]cyclohept[1,2-b]pyridine-11-enyl)piperidine-1-yl)butyl)phenyl)-2-methylpropionic acid (Example 24)**

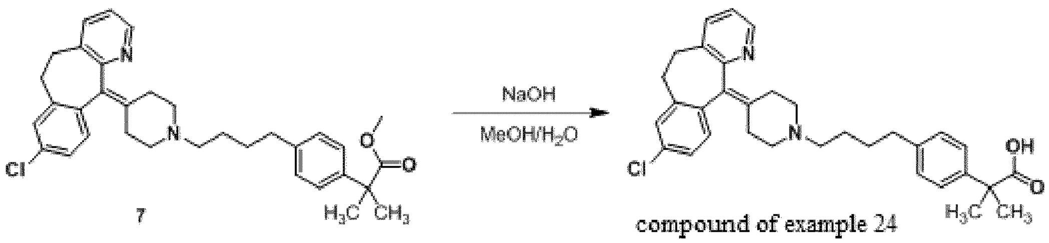


7 compound of example 24

Dissolve 7 (787 mg, 1.45 mmol) in methanol (16 mL), add an aqueous solution (4 mL) of NaOH (232 mg, 5.80

mmol), and react overnight at 60 °C under nitrogen protection. TLC (*V* dichloromethane: *V* methanol = 15:1) detected the completion of the reaction. Concentrate to remove the solvent, add water (2 mL) to dilute the remaining aqueous phase, wash with ethyl acetate (3 mL), adjust the aqueous phase pH to 8 with 3 N dilute hydrochloric acid and then adjust the pH to 5-6 with 1 N dilute hydrochloric acid to precipitate a large amount of solids, fully stir for 30 minutes, suction filter, wash with water (5 mL×3), collect filter cake, beat the filter cake with mixed solvent (*V* methanol: *V* water = 1:2, 15 mL), filter with suction and dry the filter cake to obtain 101 mg of the compound of Example 24 as a white solid with a yield of 13.2%.

$^1$H NMR (400 MHz, $CD_3OD$) $\delta$ (ppm): 8.23 (dd, $J_1$=4.9 Hz, $J_2$=1.6 Hz, 1H, ArH), 7.55 (dd, $J_1$=7.8, $J_2$=1.6 Hz, 1H, ArH), 7.23-7.20 (m, 2H, ArH), 7.18-7.14 (m, 2H, ArH), 7.09-7.07 (m, 1H, ArH), 7.03-6.98 (m, 3H, ArH), 3.34-3.24 (m, 2H, ArH-$CH_2$), 3.04-2.96 (m, 2H, ArH-$CH_2$), 2.82-2.61 (m, 6H, $N(CH_2)_3$), 2.54-2.45 (m, 4H, $N(CH_2)$, $(CH_2)_2$), 2.39-2.23 (m, 2H, $(CH_2)$), 1.50-1.48 (m, 4H ArH-$CH_2$), 1.38 (s, 6H, $(CH_3)_2$).

$^{13}$C NMR (100 MHz, $CD_3OD$) $\delta$(ppm): 181.61, 155.33, 144.77, 143.99, 138.90, 138.13, 137.37, 135.75, 133.46, 133.38, 133.18, 132.06, 129.08, 127.99, 126.69, 124.81, 124.62, 121.93, 55.58, 51.77, 33.47, 30.11, 29.63, 27.40, 27.10, 26.95, 25.62, 23.09.

**Example 25**

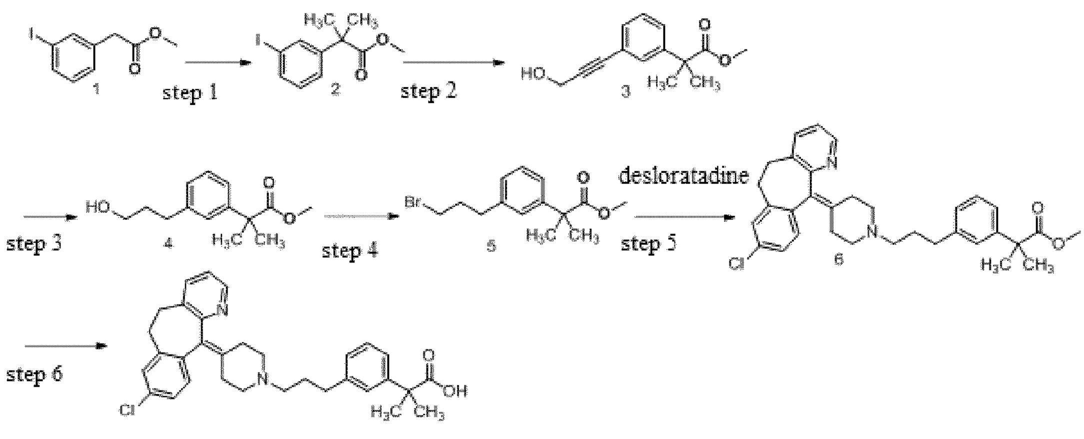


compound of example 25

Step 1 **Methyl 2-(3-iodophenyl)-2-methylpropionate (2)**

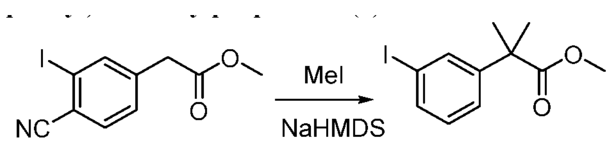


1 2

Dissolve **1** (6.3 g, 22.82 mmol) in DMF (100 mL), replace with $N_2$ three times, add NaHMDS (2 mol/L, 34 mL, 68.46 mmol) dropwise at -20 °C and continue the reaction for 40 min after the addition. MeI (13.0 g, 91.28 mmol) was added dropwise and the reaction was continued for 4 h after the addition. TLC (*V* petroleum ether: *V* ethyl acetate = 5:1) detected that the raw material reaction was complete. Add saturated ammonium chloride (250 mL) to quench, extract with ethyl acetate (100 mL×3), combine the organic phases, wash with saturated sodium chloride solution (100 mL×5), dry with anhydrous sodium sulfate, filter, concentrate and purify by silica gel column chromatography (V petroleum ether: V ethyl acetate=100:1) to obtain 4.2 g of colorless oil **2** with a yield of 60.5%.

$^1$H NMR (400 MHz, $CDCl_3$) $\delta$ (ppm): 7.67-7.66 (m, 1H, ArH), 7.59-7.57 (m, 1H, ArH), 7.30-7.28 (m, 1H, ArH), 7.07-7.03 (m, 1H, ArH), 3.66 (s, 3H, $OCH_3$), 1.55 (s, 6H, $(CH_3)_2$).

Step 2 **Methyl 2-(3-(3-hydroxypropyl-1-yn-1-yl)phenyl)-2-methylpropionate (3)**

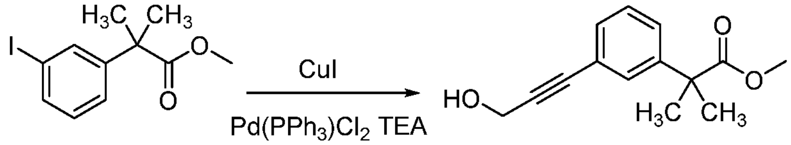


2 3

Dissolve **2** (4.2 g, 13.81 mmol) in acetonitrile (40 mL), add CuI (260 mg, 1.38 mmol) and $Pd(PPh_3)$ $Cl_2$ (480 mg, 0.69 mmol) successively, replace with $N_2$ three times, cool and add triethylamine (6.3 g, 62.14 mmol) at -10 °C, continue stirring for 10 min after the addition, add propargyl alcohol (1.1 g, 16.57 mmol) and stir for 10 min after the addition, move to room temperature and react for 4 h. TLC (*V* petroleum ether: *V* ethyl acetate = 5:1) detected that the raw material reaction was complete. Quench with water (40 mL), extract with ethyl acetate (50 mL×3), combine the organic phases, wash with saturated sodium chloride solution (50 mL), dry over anhydrous sodium sulfate, filter, concentrate and purify by silica gel column chromatography (*V* petroleum ether: *V* ethyl acetate=100:1) to obtain 3.1 g of yellow oil **3** with a yield of 96.6%.

**Step 3 Methyl 2-(3-(3-hydroxypropyl)phenyl)-2-methylpropionate (4)**

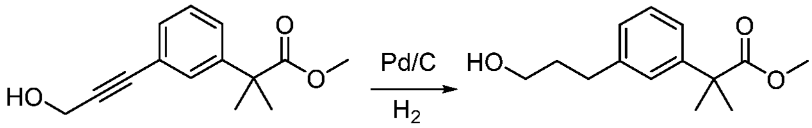


3 4

Dissolve **3** (3.1 g, 13.35 mmol) in ethanol (30 mL), add Pd/C (10% Pd, 1.2 g, 40% wt) and react for 3 h at room temperature under hydrogen (15 psi) atmosphere. Replace the hydrogen balloon and react at room temperature overnight. TLC (*V* petroleum ether: *V* ethyl acetate=2:1) detected that the raw material reaction was complete. Add diatomaceous earth to filter, concentrate the filtrate and purify by silica gel column chromatography (*V* petroleum ether: *V* ethyl acetate = 10:1) to obtain 1.56 g of colorless oil **4** with a yield of 49.5%.

$^1H$ NMR (400 MHz, $CDCl_3$) $\delta$ (ppm): 7.25-7.22 (m, 1H, ArH), 7.17-7.15 (m, 2H, ArH), 7.09-7.07 (m, 1H, ArH), 3.70-3.66 (m, 2H, ArH-$CH_2$), 3.65 (s, 3H, $OCH_3$), 2.72-2.68 (m, 2H, $CH_2OH$), 1.91-1.87 (m, 2H, $CH_2$), 1.57 (s, 6H, $(CH_3)_2$).

Step 4 **Methyl 2-(3-(3-bromopropyl)phenyl)-2-methylpropionate (5)**

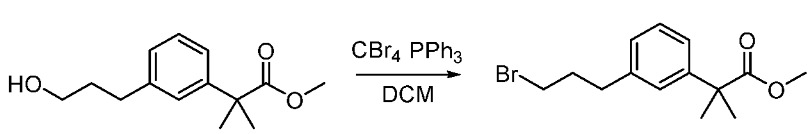


4 5

Dissolve **4** (1.66 g, 7.02 mmol) in dichloromethane (30 mL), add $CBr_4$ (3.49 g, 10.54 mmol), replace with $N_2$ three times after the addition, cool to 0 °C and add $PPh_3$ (2.86 g, 10.89 mmol) in dichloromethane (5 mL), react overnight at RT after the addition. TLC (*V* petroleum ether: *V* ethyl acetate=2:1) detected that the raw material reaction was complete. Concentrate directly and purify by column chromatography (*V* petroleum ether: *V* ethyl acetate = 50:1) to obtain 1.68 g of colorless oil **5** with a yield of 80.0%.

$^1H$ NMR (400 MHz, $CDCl_3$) $\delta$ (ppm): 7.24-7.21 (m, 1H, ArH), 7.16-7.11(m, 2H, ArH), 7.04-7.01 (m, 1H, ArH), 3.70-3.66 (m, 2H, Ph-$CH_2$), 3.65 (s, 3H, $OCH_3$), 2.71-2.64 (m, 2H, $CH_2Br$), 1.88-1.84(m, 2H, $CH_2$), 1.56(s, 6H, $(CH_3)_2$).

Step 5 **2-(3-(3-(4-(8-chloro-5,6-dihydro-11H-benzo[5,6]cyclohepta[1,2-b]pyridine-11-enyl)piperidine -1-yl)propyl)phenyl)-2-methylpropionic acid methyl ester(6)**

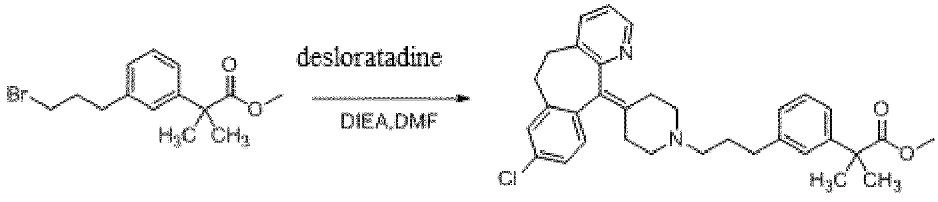


5

6

Dissolve **5** (1.8 g, 6.02 mmol) in DMF (20 mL), then add desloratadine (1.56 g, 5.01 mmol) and diisopropylethylamine (1.30 g, 10.03 mmol) successively, replace with $N_2$ 3 times, increase the temperature and react at 70 °C overnight. TLC (*V* dichloromethane: *V* methanol = 20:1) detected that the reaction was complete. Cool to room temperature, quench with water (20 mL), extract with ethyl acetate (20 mL×3), combine the organic phases, wash with saturated brine (20 mL×5), dry over anhydrous sodium sulfate, filter, concentrate and purify by silica gel column chromatography (*V* dichloromethane: *V* methanol=100:1-50:1) to yield 1.35 g of light yellow oily substance **6** with a yield of 66.0%.

Step 6 **2-(3-(3-(4-(8-chloro-5,6-dihydro-11H-benzo[5,6]cyclohepta[1,2-b]pyridine-11-enyl)piperidine -1-yl)propyl)phenyl)-2-methylpropionic acid (Example 25)**

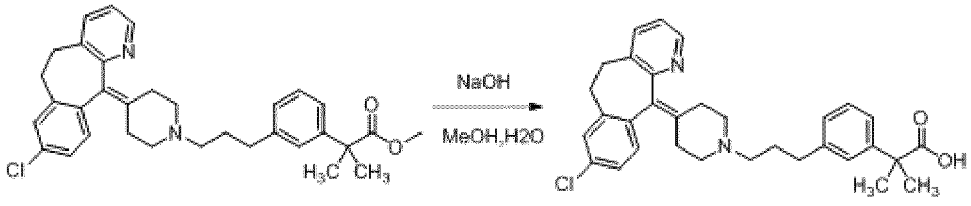


6

Compound of example 25

Dissolve 6 (600 mg, 1.13 mmol) in methanol (10 mL), add water (3 mL) and NaOH (227 mg, 5.67 mmol) and heat to 60 °C to react overnight. TLC (*V* dichloromethane: *V* methanol = 10:1) detected that the reaction was complete. Concentrate to remove methanol, quench with water (10 mL), wash with ethyl acetate (10 mL×2), adjust the pH to 5 with 2 N dilute hydrochloric acid and extract the aqueous phase with a mixed solvent (10 mL×3, *V* dichloromethane: *V* methanol = 20:1). The organic phases were combined, dried over anhydrous sodium sulfate, filtered, concentrated under reduced pressure and purified by a preparation plate (*V* dichloromethane: *V* methanol = 10:1) to obtain 170 mg of the compound of Example 25 as a white solid with a yield of 29.1%.

$^1$H NMR (400 MHz, $CD_3OD$) $\delta$(ppm): 8.33-8.31 (m, 1H, ArH), 7.65-7.63 (m, 1H, ArH), 7.27-7.16 (m, 6H, ArH), 7.11 (d, *J*=8.4Hz, 1H, ArH), 7.01-6.99 (m, 1H, ArH), 3.42-3.35 (m, 2H, ArH-$CH_2CH_2$), 3.15-3.11 (m, 1H, ArH-$CH_2CH_2$), 2.89-2.53 (m, 10H, $(CH_2)_2N(CH_2)_2$, $(CH_2)_2$), 2.49-2.44 (m, 1H, $(CH_2)_2$), 2.39-2.33 (m, 1H, $(CH_2)_2$), 1.97-1.89 (m, 2H), 1.49 (s, 6H, $CH_3CCH_3$).

$^{13}$C NMR (100 MHz, $CD_3OD$) $\delta$(ppm): 182.36, 156.30, 147.60, 145.79, 140.23, 139.94, 138.41, 136.74, 134.50, 134.28, 134.25, 133.09, 130.09, 129.01, 127.94, 125.89, 125.83, 125.63, 123.46, 122.97, 56.30, 52.87, 32.75, 31.12, 30.65, 28.15, 28.01, 26.68, 26.67, 26.16.

**Example 26 2-(3-(3-(4-(8-chloro-5,6-dihydro-11H-benzo[5,6]cyclohepta[1,2-b]pyridine-11-enyl)piperidine -1-yl)) propyl)-4-cyanophenyl)-2-methylpropionic acid**

Refer to the preparation method of Example 25 and replace methyl 2-(3-iodophenyl)acetate with methyl 2-(4-cyano-3-iodophenyl)acetate as the raw material. 260mg of the compound of Example 26 was prepared as a white solid with a yield of 63.2%.

$^1$H NMR (400 MHz, $CD_3OD$) $\delta$(ppm): 8.34 (dd, $J_1$=1.6 Hz, $J_2$=4.8 Hz, 1H, ArH), 7.67-7.65 (m, 1H, ArH), 7.60 (d, *J*=8.0 Hz, 1H, ArH), 7.46-7.42 (m, 2H, ArH), 7.29-7.25 (m, 2H, ArH), 7.20-7.18 (m, 1H, ArH), 7.12 (d, *J*=8.4 Hz, 1H,

ArH), 3.45-3.35 (m, 2H, ArH-$CH_2CH_2$), 3.27-3.23 (m, 2H, ArH-$CH_2CH_2$), 2.96-2.81 (m, 8H, $(CH_2)_2N(CH_2)_2$), 2.67-2.57 (m, 2H, $(CH_2)_2$), 2.54-2.38 (m, 2H, $(CH_2)_2$), 2.08-2.00 (m, 2H, $(CH_2)_2$), 1.52 (s, 6H, $CH_3CCH_3$).

$^{13}$C NMR (400 MHz, $CD_3OD$) $\delta$(ppm): 181.13, 156.23, 153.61, 145.89, 144.07, 140.05, 138.55, 136.73, 134.68, 134.60, 133.76, 133.22, 132.47, 130.10, 129.10, 127.61, 125.93, 124.94, 123.09, 117.82, 109.22, 55.53, 52.91, 31.31, 31.14, 30.70, 28.07, 27.93, 26.31, 25.62.

**Example 27 2-(3-(3-(4-(10-hydroxy-9,10-dihydro-4H-benzo[4,5]cyclohepta[1,2-b]thiophene-4-alkylene)pi peridin-1-yl)propyl)phenyl)-2-methylpropionic acid**

Refer to the preparation method of Example 25 and replace desloratadine with 4-(piperidine-4-alkylene)-9,10-dihydro-4H-benzo[4,5]cyclohepta[1,2-b]thiophene-10-alcohol as a raw material. 690 mg of the compound of Example 27 was prepared as a yellow solid with a yield of 86%.

$^1$H NMR (400 MHz, DMSO-$d_6$): $\delta$ ppm 7.35-7.38 (m, 1 H), 7.30-7.32 (m, 0.5 H), 7.19-7.24 (m, 1.5 H), 7.13-7.18 (m, 2 H), 7.07-7.11 (m, 3 H), 6.99-7.04 (m, 1 H), 6.76 (d, *J*=5.2 Hz, 1 H), 5.89 (d, *J*=7.2 Hz, 0.7 H), 5.37 (d, *J*=5.6 Hz, 0.3 H), 5.04-5.07 (m, 0.3 H), 4.56-4.62 (m, 0.7 H), 3.57 (s, 3 H), 3.25-3.32 (m, 1 H), 2.89-2.95 (m, 1 H), 2.67 (b, 1 H), 2.56-2.60 (m, 3 H), 2.40-2.45 (m, 1 H), 2.15-2.33 (m, 5 H), 1.98-2.02 (m, 1 H), 1.67-1.74 (m, 2 H), 1.48 (s, 6 H).

**Example 28**

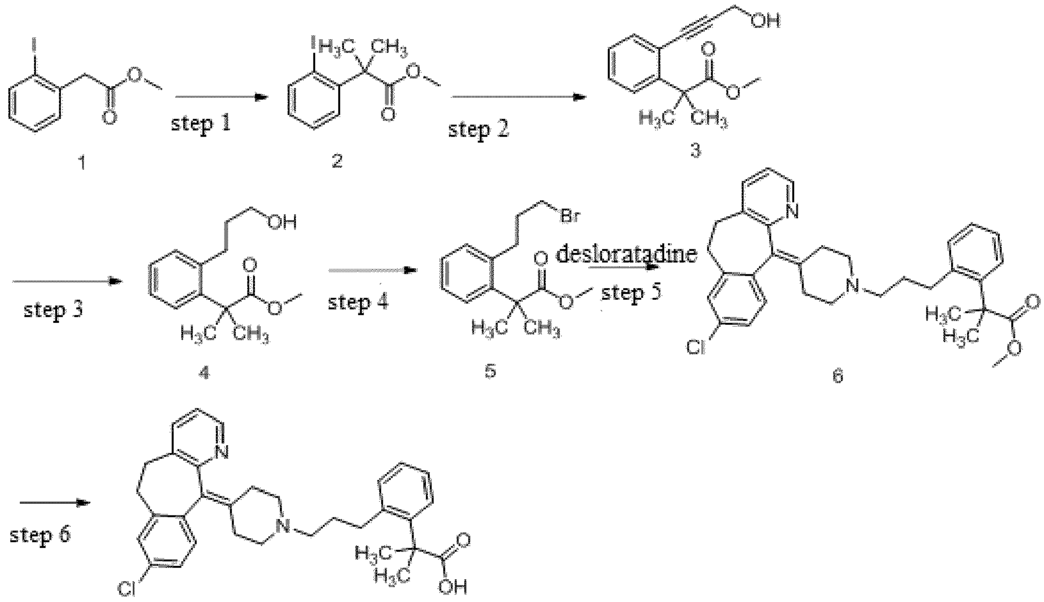

compound of example 28

Step 1 **Methyl 2-(2-iodophenyl)-2-methylpropionate (2)**

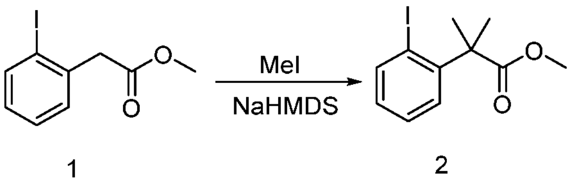


Dissolve **1** (4.83 g, 17.50 mmol) in DMF (70 mL), replace with $N_2$ three times, add NaHMDS (2 mol/L, 26.2 mL, 52.49 mmol) solution dropwise at -20 °C and continue reacting for 40 min after the addition. MeI (9.93 g, 69.98 mmol) was added dropwise and the reaction was continued for 4 h after the addition. TLC (*V* petroleum ether: *V* ethyl acetate = 5:1) detected that the reaction was complete. Add saturated ammonium chloride (200 mL) to quench, extract with

ethyl acetate (100 mL×3), combine the organic phases, wash with saturated sodium chloride (100 mL×5), dry with anhydrous sodium sulfate, filter, concentrate and purify by silica gel column chromatography (*V* petroleum ether: *V* ethyl acetate=200:1) to obtain 4.05 g of light yellow oil **2** with a yield of 76.1%.

$^1H$ NMR (400 MHz, $CDCl_3$) $\delta$ (ppm): 7.92-7.90 (m, 1H, ArH), 7.42-7.33 (m, 2H, ArH), 6.94-6.91 (m, 1H, ArH), 3.70 (s, 3H, $OCH_3$), 1.66 (s, 6H, $(CH_3)_2$).

Step 2 **Methyl 2-(2-(3-hydroxypropyl-1-yn-1-yl)phenyl)-2-methylpropionate (3)**

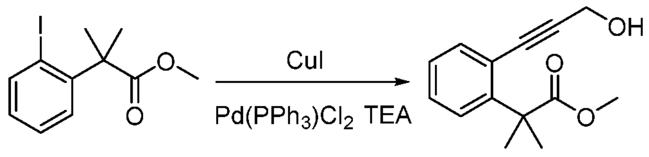


2 3

Dissolve **2** (4.05 g, 13.32 mmol) in acetonitrile (40 mL) and add CuI (254 mg, 1.33 mmol) and $Pd(PPh_3)Cl_2$ (467 mg, 0.67 mmol) in sequence. Replace with $N_2$ three times and add triethylamine (6.06 g, 59.93 mmol) at -10 °C. Continue to stir for 10 min after the addition and then add propargyl alcohol (1.06 g, 15.98 mmol). Stir for 10 min after the addition, move to room temperature and react for 4 h and then heat to 30 °C to react overnight. TLC (*V* petroleum ether: *V* ethyl acetate=5:1) detected about 60% of the raw material remaining. Quench with water (40 mL), extract with ethyl acetate (50 mL×3), combine the organic phases, wash with saturated sodium chloride (50 mL), dry with anhydrous sodium sulfate, filter, concentrate and purify by silica gel column chromatography (*V* Petroleum ether: *V* ethyl acetate=100:1) to obtain 600 mg of brown oil **3** with a yield of 19.4%.

Step 3 **Methyl 2-(2-(3-hydroxypropyl)phenyl)-2-methylpropionate (4)**

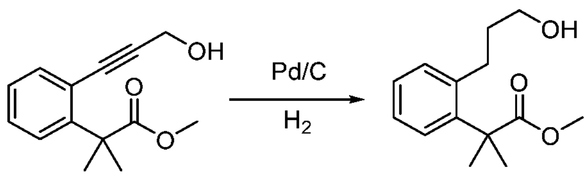


3 4

Dissolve **3** (1.25 g, 5.38 mmol) in ethanol (30 mL), add Pd/C (10%Pd, 500 mg, 40%wt), react at room temperature under hydrogen (15 psi) atmosphere for 3 hours, replace the hydrogen balloon and then react at room temperature overnight. TLC (*V* petroleum ether: *V* ethyl acetate=3:1) detected that the reaction was complete. Add diatomaceous earth to filter, concentrate the filtrate and purify by silica gel column chromatography (*V* petroleum ether: *V* ethyl acetate=10:1) to obtain 500 mg of pale yellow oil 4 with a yield of 39.4%.

Step 4 **Methyl 2-(2-(3-bromopropyl)phenyl)-2-methylpropionate (5)**

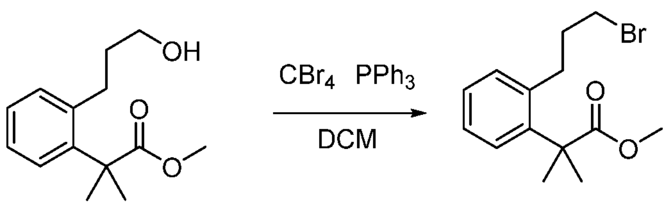


4 5

Dissolve **4** (500 mg, 2.12 mmol) in dichloromethane (12 mL) and add $CBr_4$ (1.05 g, 3.17 mmol). Replace with $N_2$ three times after the addition. Cool down to 0 °C, add $PPh_3$ (860 mg, 3.28 mmol) in dichloromethane solution (3 mL) dropwise and react overnight at RT after the addition. TLC (*V* petroleum ether: *V* ethyl acetate=2:1) detected that the raw material reaction was complete. It was directly concentrated and purified by silica gel column chromatography (V petroleum ether: V ethyl acetate=100:1) to obtain 450 mg of colorless oil **5** with a yield of 71.1%.

$^1H$ NMR (400 MHz, $CDCl_3$) $\delta$ (ppm): 7.38-7.36 (m, 1H, ArH), 7.24-7.18 (m, 3H, ArH), 3.68 (s, 3H, $OCH_3$), 3.47-3.44 (m, 2H, ArH-$CH_2$), 2.64-2.60 (m, 2H, $CH_2Br$), 2.16-2.09 (m, 2H, $CH_2$), 1.59 (s, 6H, $(CH_3)_2$).

Step 5 **2-(3-(4-(8-chloro-5,6-dihydro-11H-benzo[5 ,6]cyclohepta[1,2-b]pyridine-11-enyl)piperidin-1-yl)propyl)phenyl)-2-methylpropionic acid methyl ester (6)**

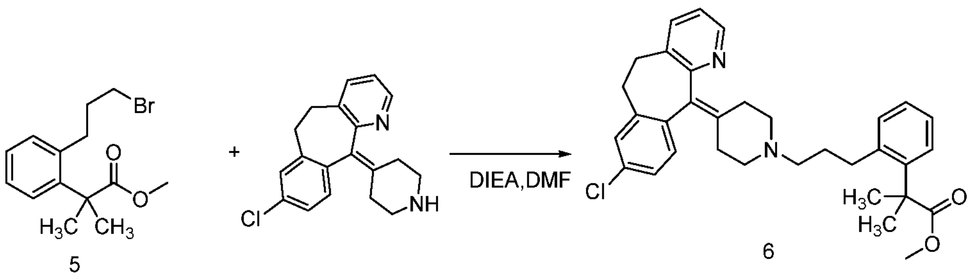


5

6

Dissolve **5** (450 mg, 1.50 mmol) in DMF (10 mL), then add desloratadine (514 mg, 1.65 mmol) and diisopropylethylamine (389 mg, 3.01 mmol) successively, replace with $N_2$ three times and heat to react at 70 °C overnight. TLC (*V* dichloromethane: *V* methanol = 20:1) detected that the reaction was complete. Cool to room temperature, quench with water (10 mL), extract with ethyl acetate (20 mL×3), combine the organic phases, wash with saturated brine (20 mL×5), dry with anhydrous sodium sulfate, filter, concentrate and purify by silica gel column chromatography (*V* dichloromethane: *V* methanol = 50:1) to obtain 650 mg of pale yellow oily substance 6 with a yield of 81.7%.

Step 6 **2-(2-(3-(4-(8-chloro-5,6-dihydro-11H-benzo[5,6]cyclohepta[1,2-b]pyridine-11-enyl)piperidine -1-yl)propyl)phenyl)-2-methylpropionic acid (Example 28)**

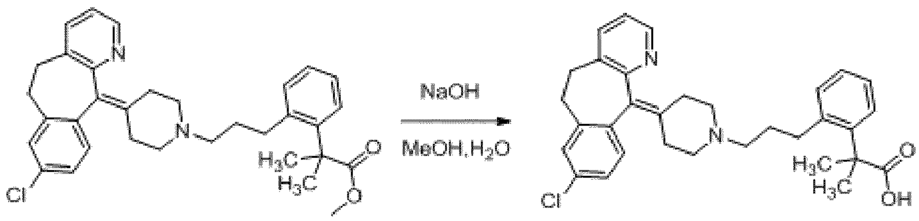


6

compound of example 28

Dissolve 6 (650 mg, 1.23 mmol) in methanol (10 mL), add water (3 mL) and NaOH (492 mg, 12.27 mmol) and heat to 70 °C to react for four days. TLC (*V* dichloromethane: *V* methanol = 10:1) detected about 50% of the raw material remaining. Concentrate to remove methanol, add water (10 mL), wash with ethyl acetate (15 mL×2), add water (10 mL) to ethyl acetate, and adjust the pH to 5 with 2 N dilute hydrochloric acid (10 drops). Extract with ethyl acetate (15 mL×2), combine the organic phases, dry with anhydrous sodium sulfate, filter, concentrate and purify by flash preparative chromatography (*V* dichloromethane: *V* methanol = 16: 1) to obtain 150 mg of the compound of Example 28 as a white solid with a yield of 23.7%.

$^1H$ NMR (400 MHz, $CD_3OD$) $\delta$ (ppm): 8.35-8.33 (m, 1 H, ArH), 7.67-7.64 (m, 1 H, ArH), 7.41-7.38 (m, 1 H, ArH), 7.28-7.18 (m, 4 H, ArH), 7.15-7.13 (m, 3 H, ArH), 3.45-3.35 (m, 2 H, ArH-$CH_2CH_2$), 3.29-3.25 (m, 2 H, ArH-$CH_2CH_2$), 3.14-3.02 (m, 4 H, $N(CH_2)_2$), 2.91-2.68 (m, 6 H, $N(CH_2)_2$, $(CH_2)_3$), 2.63-2.45 (m, 2 H, $(CH_2)_3$), 2.14-2.07 (m, 2 H, $(CH_2)_3$), 1.49 (s, 6 H, $CH_3CCH_3$).

$^{13}C$ NMR (100 MHz, $CD_3OD$) $\delta$(ppm): 186.31, 156.99, 146.94, 146.76, 140.90, 139.35, 139.21, 137.52, 135.83, 135.41, 134.10, 133.99, 130.88, 130.04, 129.92, 126.78, 126.76, 126.69, 126.14, 123.92, 57.17, 53.91, 48.46, 31.95,

31.53, 29.51, 28.47, 28.33, 28.28, 25.76.

**Example 29**

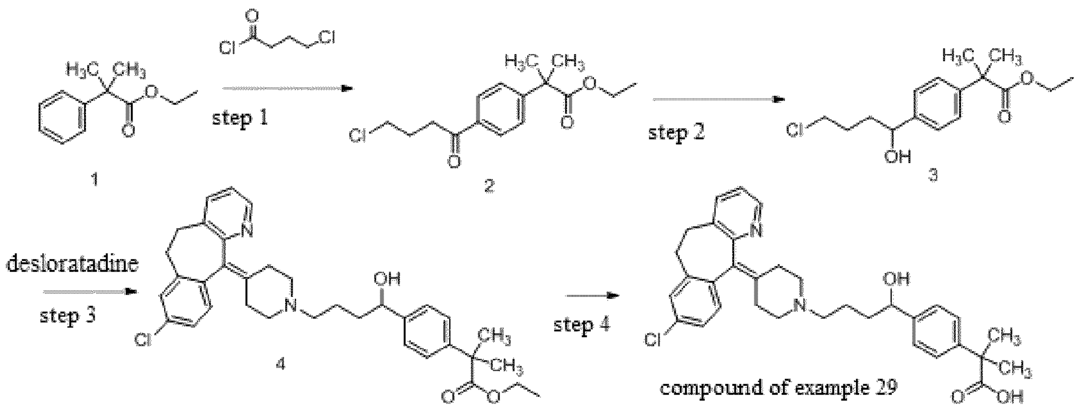


Step 1 **Ethyl 2-(4-(4-chlorobutyryl)phenyl)-2-methylpropionate (2)**

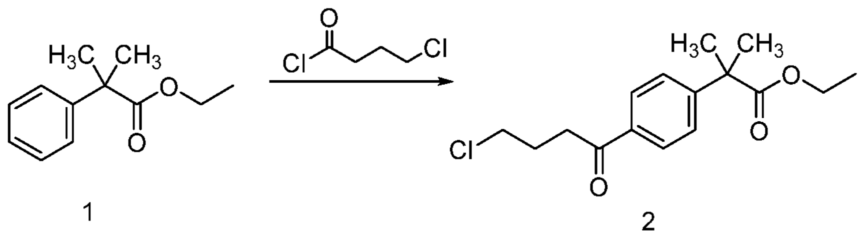

Add $AlCl_3$ (13.80 g, 104 mmol) to dichloromethane (100 mL), protect with nitrogen and reduce the temperature to -20 °C. 1 (10.0 g, 52 mmol) and 4-chlorobutyryl chloride (14.6 g, 104 mmol) were added dropwise successively. After the addition, the mixture was moved to room temperature and reacted overnight. TLC (*V* petroleum ether: *V* ethyl acetate=10:1) detected that the reaction was complete. Dichloromethane (100 mL) was added to dilute the reaction solution and water (100 mL) was added to quench the reaction. Separate the liquids, wash the organic phase with saturated sodium bicarbonate solution (200 mL×3) and then with saturated brine (50 mL×3), dry with anhydrous sodium sulfate, filter, concentrate and purify through silica gel column chromatography (*V* petroleum ether: *V* ethyl acetate=30:1) to obtain 2.86 g of yellow oily substance **2** with a yield of 18.5%.

$^1H$ NMR (400 MHz, $CDCl_3$) $\delta$ (ppm): 7.98-7.84 (m, 2H, ArH), 7.57-7.41 (m, 2H, ArH), 4.16-4.10 (m, 2H, $OCH_2CH_3$), 3.70-3.66 (m, 2H, $COCH_2$), 3.19-3.14 (m, 2H, $ClCH_2$), 2.27-2.19 (m, 2H, $CH_2$), 1.61-1.59 (m, 6H, $(CH_3)_2$), 1.21-1.16 (m, 3H, $CH_2CH_3$).

Step 2 **Ethyl 2-(4-(4-chloro-1-hydroxybutyl)phenyl)-2-methylpropionate (3)**

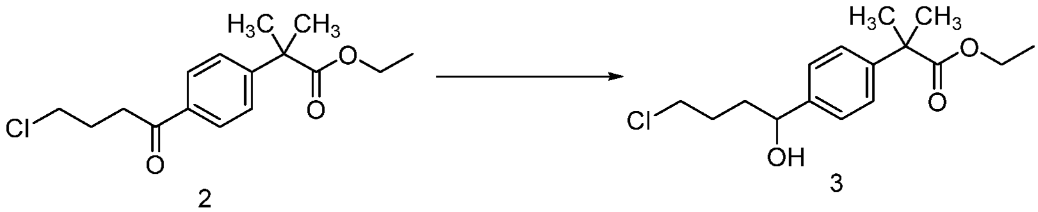

Dissolve **2** (2.96 g, 9.64 mmol) in methanol (20 mL), cool in an ice-water bath, add sodium borohydride (0.73

g, 19.27 mmol) in batches and move to room temperature to react for 1 h after the addition. TLC (*V* petroleum ether: *V* ethyl acetate = 5:1) detected that the reaction was complete. Concentrate under reduced pressure, add ethyl acetate (60 mL) to dissolve the product, wash with 2 N dilute hydrochloric acid solution (20 mL) and then with saturated brine (20 mL×3), dry with anhydrous sodium sulfate, filter, concentrate under reduced pressure and purify by silica gel column chromatography (*V* petroleum ether: *V* ethyl acetate = 10:1) to obtain 2.03 g of yellow oil **3** with a yield of 70.5%.

Step 3 **Ethyl 2-(4-(4-(4-(8-chloro-5,6-dihydro-11H-benzo[5,6]]cyclohept[1,2-b]pyridine-11-alkenyl)piperi din-1-yl)-1-hydroxybutyl)phenyl)-2-methylpropionate (4)**

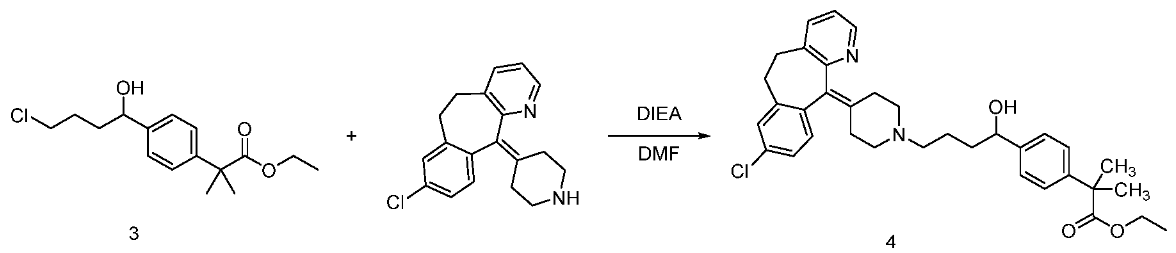

3
4

Dissolve **3** (2.03 g, 6.78 mmol) in DMF (20 mL), then add desloratadine (1.91 g, 6.18 mmol) and diisopropylethylamine (1.59 g, 12.3 mmol) in sequence, protecte with nitrogen and react at 70 °C overnight. TLC (*V* dichloromethane: *V* methanol = 10:1) detected that the reaction was complete. Cool to room temperature, quench with water (30 mL), extract with ethyl acetate (30 mL×2), combine the organic phases, wash with saturated brine (30 mL×3), dry with anhydrous sodium sulfate, filter, concentrate and purify by flash preparative chromatography (40 g, *V* dichloromethane: *V* methanol = 50:1) to yield 1.01 g of yellow solid 4 with a yield of 25.0%.

Step 4 **2-(4-(4-(4-(8-chloro-5,6-dihydro-11H-benzo[5,6]]cyclohept[1,2-b]pyridine-11-enyl)piperidin-1-yl)-1-hydroxybutyl)phenyl)-2-methylpropionic acid (Example 29)**

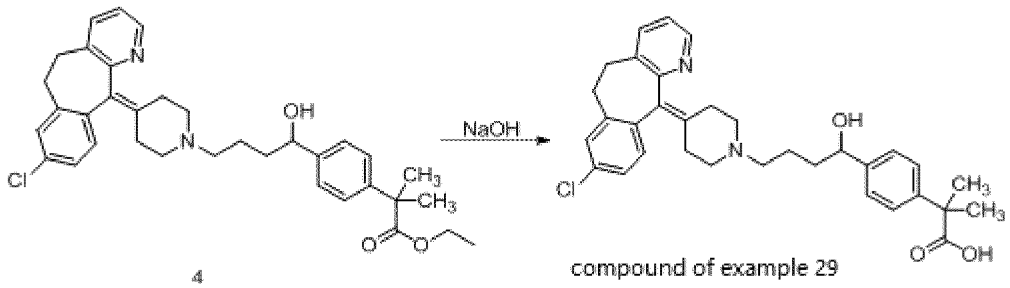

4
compound of example 29

Dissolve 4 (400 mg, 0.70 mmol) in EtOH (6 mL), add $H_2O$ (3 mL) and sodium hydroxide (112 mg, 2.80 mmol) and react at 80 °C overnight. TLC (*V* dichloromethane: *V* methanol = 10:1) detected that the reaction was complete. Concentrate under reduced pressure, add water (6 mL), wash with ethyl acetate (6 mL×6), slowly add 1 N HCl solution dropwise to the water phase to precipitate out solid, adjust the pH to 5, stir for 20 min, filter, wash the filter cake with water (5 mL×2) and dry to obtain 110 mg of the compound of Example 29 as a white solid with a yield of 28.9%.

$^1H$ NMR (400 MHz, $CD_3OD$) $\delta$ (ppm): 8.23 (dd, $J_1$=1.6 Hz, $J_2$=4.8 Hz, 1H, ArH), 7.57-7.54 (m, 1H, ArH), 7.29-7.19 (m, 2H, ArH), 7.18-7.09 (m, 4H, ArH), 7.08-7.01 (m, 2H, ArH), 4.57-4.50 (m, 1H, $(CH_2)_3CH$), 3.33-3.25 (m, 2H, ArH-$CH_2CH_2$), 2.98-2.95 (m, 2H, ArH-$CH_2CH_2$), 2.82-2.70 (m, 2H, $N(CH_2)_2$), 2.66-2.24 (m, 8H, $N(CH_2)_2$, $(CH_2)_3CH$), 1.71-1.51 (m, 4H, $(CH_2)_3CH$), 1.41-1.39 (m, 6H, $CH_3CCH_3$).

$^{13}C$ NMR (100 MHz, $CD_3OD$) $\delta$(ppm): 155.41, 146.56, 145.48, 144.76, 143.24, 141.22, 138.90, 135.76, 133.48, 133.06, 132.07, 129.11, 128.00, 126.74, 124.81, 124.33, 123.44, 122.65, 122.09, 121.93, 72.19, 71.84, 55.87, 51.89, 35.17, 30.14, 29.62, 27.14, 25.66, 20.27, 20.12.

**Example 30**

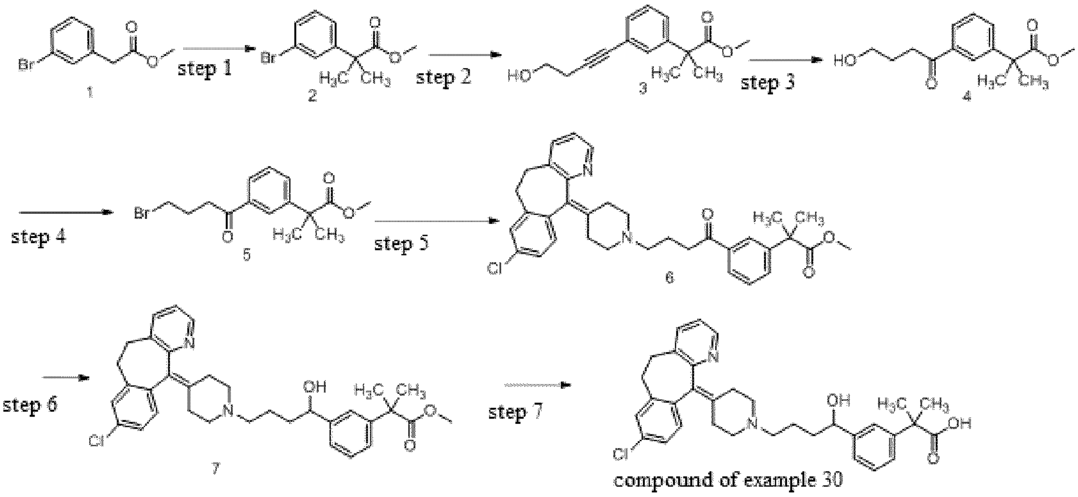


Step 1 **Methyl 2-(3-bromophenyl)-2-methylpropionate (2)**

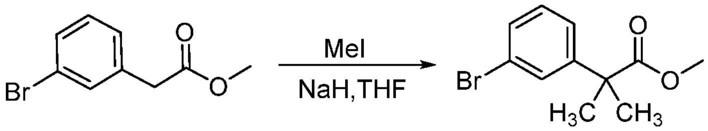


1 2

Dissolve **1** (6.87 g, 30 mmol) in THF (100 mL), add NaH (60%, 3.6 g, 90 mmol) in batches under a nitrogen atmosphere at 0 °C, incubate and react for 0.5 h after the addition, then add iodine Methane (10.65 g, 75 mmol), then react at room temperature for 3 h. TLC (*V* petroleum ether: *V* ethyl acetate = 10:1) detected that the reaction was complete. Add water (100 mL) at 0 °C. After warming to room temperature, extract with ethyl acetate (100 mL×3). Combine the organic phases. Wash with saturated brine (100 mL), dry with anhydrous sodium sulfate, filter, and concentrate under reduced pressure to obtain 7.7 g of pale yellow oil **2** with a yield of 99%.

Step 2 **Methyl 2-(3-(4-hydroxy-1-yn-1-yl)phenyl)-2-methylpropionate (3)**

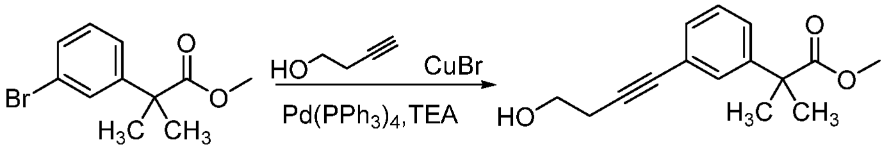


2 3

Dissolve **2** (2.57 g, 10 mmol) in triethylamine (35 mL), then add cuprous bromide (171 mg, 1.2 mmol), $Pd(PPh_3)_4$ (462 mg, 0.4 mmol) and 3-butyn-1-ol in sequence (1.4 g, 20 mmol), react at 90 °C for 4 h under nitrogen atmosphere after addition. TLC (*V* petroleum ether: *V* ethyl acetate = 5:1) detected the complete reaction of the raw materials. The reaction solution was filtered, concentrated, diluted with ethyl acetate (100 mL), and then washed with saturated sodium chloride (100 mL×3). The organic layer was dried with anhydrous $Na_2SO_4$, filtered, concentrated and purified by flash preparative chromatography (40 g, *V* petroleum ether: *V* ethyl acetate=4:1) to obtain 2.2 g of light yellow oil 3 with a yield of 89%.

$^1H$ NMR (400 MHz, $CDCl_3$) $\delta$ (ppm): 7.38-7.37 (m, 1H, ArH), 7.29-7.28 (m, 1H, ArH), 7.26-7.22 (m, 2H, ArH), 3.80 (t, *J*=4.4 Hz, 2H, $CH_2$), 3.64 (s, 3H, $OCH_3$), 2.67 (t, *J*=4.0 Hz, 2H, $OCH_2$), 1.55 (s, 6H, $(CH_3)_2$).

Step 3 **Methyl 2-(3-(4-hydroxybutyryl)phenyl)-2-methylpropionate (4)**

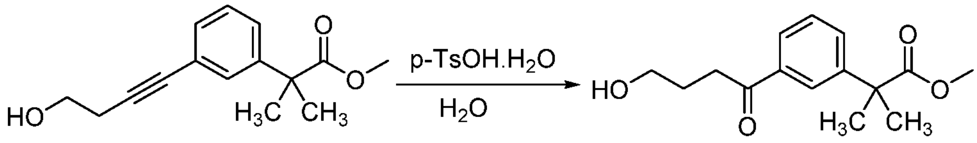


3 4

Add **3** (1.2 g, 4.88 mmol) and p-toluenesulfonic acid monohydrate (190 mg, 1 mmol) into water (20 mL). After the addition was complete, react at 100 °C for 2 h under a nitrogen atmosphere. TLC (*V* petroleum ether : *V* ethyl acetate=2:1) determined that the raw material has reacted completely. The reaction solution was cooled and extracted with ethyl acetate (40 mL×3). The organic phases were combined and washed with saturated sodium chloride (60 mL×3). The organic layer was dried with anhydrous $Na_2SO_4$, filtered, concentrated and purified by flash chromatography (20 g, *V* petroleum ether: *V* ethyl acetate = 2:1) to obtain 1.18 g of pale yellow oil **4** with a yield of 91%.

$^1H$ NMR (400 MHz, $CDCl_3$) $\delta$ (ppm): 7.94 (d, *J*=9.6 Hz, 1H, ArH), 7.80 (dd, $J_1$=5.2 Hz, $J_2$=11.2 Hz, 1H, ArH), 7.52 (d, *J*=6.0 Hz, 1H, ArH), 7.44-7.39 (m, 1H, ArH), 3.72 (t, *J*=4.0 Hz, 2H, $COCH_2$), 3.65 (s, 3H, $OCH_3$), 3.10 (t, *J*=4.8 Hz, 2H, $OCH_2$), 2.03-1.98 (m, 2H, $CH_2$), 1.60 (s, 6H, $(CH_3)_2$).

Step 4 **Methyl 2-(3-(4-bromobutyryl)phenyl)-2-methylpropionate (5)**

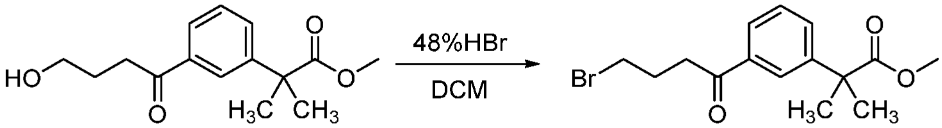


4 5

Dissolve **4** (600 mg, 2.27 mmol) in dichloromethane (10 mL), add 48% hydrogen bromide aqueous solution (1.15 g, 6.81 mmol), react at room temperature for 1 h after the addition, TLC (*V* petroleum ether: *V* acetic acid Ethyl ester=2:1) detected the reaction was complete. The reaction solution was washed with saturated sodium chloride (20 mL×3). The organic layer was dried with anhydrous $Na_2SO_4$, filtered, concentrated and purified by flash preparative chromatography (10 g, *V* petroleum ether: *V* Ethyl acetate=10:1) to obtain 680 mg of colorless transparent oily substance **5** with a yield of 92%.

Step 5 **Methyl 2-(3-(4-(4-(8-chloro-5,6-dihydro-11H-benzo[5,6]cyclohept[1,2-b]pyridine-11-enyl) piperidin-1-yl)butyryl)phenyl)-2-methylpropionate (6)**

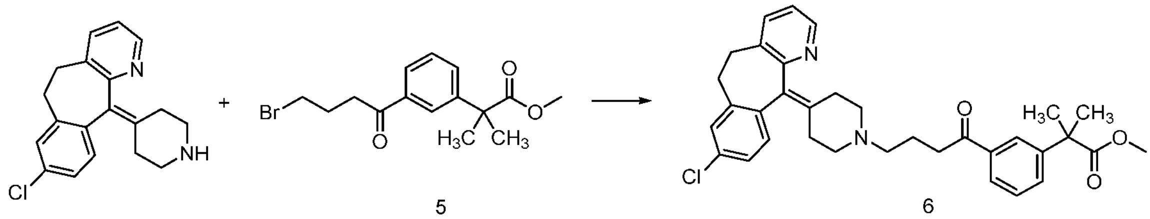

5 6

Dissolve 5 (680 mg, 2.08 mmol) in DMF (10 mL), then add diisopropylethylamine (536 mg, 4.16 mmol) and desloratadine (774 mg, 2.5 mmol) in sequence. After the addition was complete, react at 60 °C for 4 h under nitrogen atmosphere. TLC (*V* petroleum ether: *V* ethyl acetate=10:1) detected that the reaction was complete. The reaction solution was added with ethyl acetate (40 mL), washed with saturated brine (20 mL×3), dried with anhydrous $Na_2SO_4$, filtered, concentrated and purified by flash preparative chromatography (12 g, *V* dichloromethane: *V* methanol = 30: 1) to obtain 820 mg of pale yellow solid **6** with a yield of 71%.

Step 6 **Methyl 2-(3-(4-(4-(8-chloro-5,6-dihydro-11H-benzo[5,6]cyclohept[1,2-b]pyridine-11-enyl) (piperidin-1-yl)-1-hydroxybutyl)phenyl)-2-methylpropionate (7)**

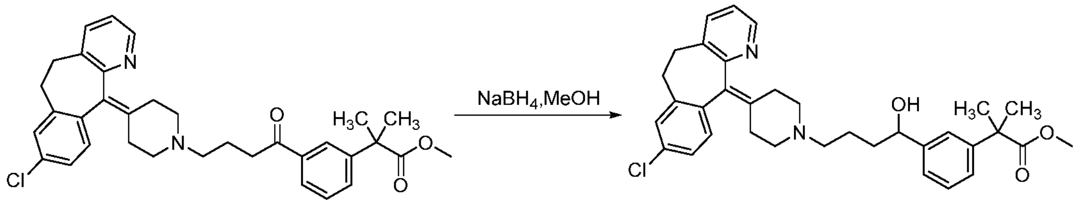


6 7

6 (820 mg, 1.47 mmol) was dissolved in MeOH (10 mL), sodium borohydride (112 mg, 2.94 mmol) was added in batches at 0 °C and the reaction was incubated for 0.5 h after the addition. TLC (*V* dichloromethane: *V* methanol = 10:1) detected that the reaction was complete. The reaction solution was added with water (40 mL), extracted with ethyl acetate (40 mL) and the organic phase was washed with saturated brine (20 mL×3). It was dried with anhydrous $Na_2SO_4$, filtered, concentrated and purified by silica gel column chromatography (*V* dichloromethane: *V* methanol = 10:1) to obtain 802 mg of brown solid 7 with a yield of 97%.

Step 7 **2-(3-(4-(4-(8-chloro-5,6-dihydro-11H-benzo[5,6]cyclohepta[1,2-b]pyridine-11-enyl)piperidine -1-yl)-1-hydroxybutyl)phenyl)-2-methylpropionic acid (Example 30)**

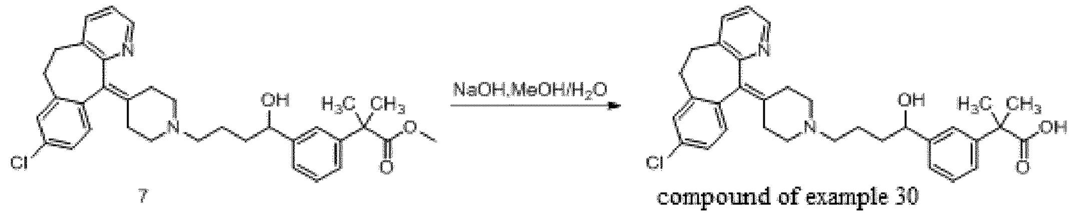


7 compound of example 30

Dissolve 7 (400 mg, 0.72 mmol) in methanol (10 mL), add water (2 mL) and sodium hydroxide (72 mg, 1.79 mmol) and react at 60 °C overnight. TLC (*V* dichloromethane: *V* methanol = 10:1) detected that the reaction was complete. Concentrate to remove methanol, add water (10 mL) and wash with ethyl acetate (10 mL×2). The aqueous phase was adjusted to pH 7 with 2 N hydrochloric acid to precipitate a solid, filter, dissolve in dichloromethane (20 mL), dry with anhydrous sodium sulfate, filter, concentrate and purify by preparation plate (*V* dichloromethane: *V* methanol = 5:1) to obtain 230 mg. Dissolve in 10% NaOH aqueous solution (10 mL) and adjust the pH to 7 with 2 N hydrochloric acid to precipitate a solid. Filter and dry to obtain 220 mg of the compound of Example 30 as a white solid with a yield of 56.0%.

$^1H$ NMR (400MHz, $CD_3OD$) $\delta$ (ppm): 8.31 (d, *J*=3.2 Hz, 1H, ArH), 7.63 (d, *J*=5.2 Hz, 1H, ArH), 7.36 (s, 1H, ArH), 7.28-7.23 (m, 4H, ArH), 7.17-7.15 (m, 2H, ArH), 7.09 (d, *J*=5.2 Hz, 1H, ArH), 4.66-4.64 (m, 1H, $(CH_2)_3CH$), 3.41-3.33 (m, 2H, ArH-$CH_2CH_2$), 3.19-3.15 (m, 2H, ArH-$CH_2CH_2$), 2.89-2.79 (m, 6H, $N(CH_2)_2$), 2.62-2.57 (m, 2H, $N(CH_2)_2$), 2.48-2.46 (m, 1H, $(CH_2)_3CH$), 2.39-2.37 (m, 1H, $(CH_2)_3CH$), 1.79-1.70 (m, 3H, $(CH_2)_3CH$), 1.64-1.59 (m, 1H, $(CH_2)_3CH$), 1.49 (s, 6H, $CH_3CCH_3$).

$^{13}C$ NMR (100 MHz, $CD_3OD$) $\delta$(ppm): 182.10, 156.24, 147.26, 145.87, 144.27, 140.03, 138.55, 136.71, 134.66, 133.74, 133.24, 130.10, 129.09, 127.90, 125.91, 124.42, 123.75, 123.27, 123.09, 73.14, 56.71, 52.86, 52.84, 35.92, 31.14, 30.69, 27.99, 27.84, 26.62, 26.52, 20.94.

**Example 31 2-(3-(3-(4-(8-chloro-5,6-dihydro-11H-benzo[5,6]cyclohepta[1,2-b]pyridine-11-enyl)piperidine -1-yl)-1-hydroxypropyl)phenyl)-2-methylpropionic acid**

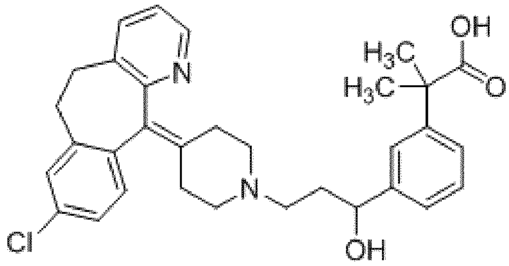

compound of example 31

Refer to the synthesis method of Example 29 and replace 3-butyn-1-ol with propargyl alcohol. 380 mg of the compound of Example 31 was prepared as a white solid with a yield of 44%.

$^1$H NMR (400MHz, $CD_3OD$) $\delta$ (ppm): 8.36 (dd, $J_1$=1.6 Hz, $J_2$=5.2 Hz, 1H, ArH), 7.67 (dd, $J_1$=1.2 Hz, $J_2$=7.6 Hz, 1H, ArH), 7.40 (s, 1H, ArH), 7.32-7.30 (m, 1H, ArH), 7.28-7.23 (m, 3H, ArH), 7.19 (dd, $J_1$=1.6 Hz, $J_2$=8.0 Hz, 2H, ArH), 7.12 (d, $J$=8.4 Hz, 1H, ArH), 4.71-4.74 (m, 1H, $(CH_2)_2CH$), 3.44-3.36 (m, 2H, ArH-$CH_2CH_2$), 3.20-3.13 (m, 2H, ArH-$CH_2CH_2$), 3.00-2.74 (m, 6H, $N(CH_2)_2$), 2.61-2.53 (m, 2H, $N(CH_2)_2$), 2.48-2.35 (m, 2H, $(CH_2)_2CH$), 2.07-1.99 (m, 2H, $(CH_2)_2CH$), 1.51 (s, 6H, $CH_3CCH_3$).

$^{13}$C NMR (100 MHz, $CD_3OD$) $\delta$(ppm): 181.61, 156.28, 147.10, 145.86, 143.88, 140.04, 138.53, 136.75, 134.62, 133.85, 133.23, 130.12, 129.08, 127.99, 125.90, 124.80, 123.32, 123.07, 71.87, 54.41, 53.09, 53.02, 33.51, 31.16, 30.70, 28.18, 28.04, 26.50.

**Example 32**

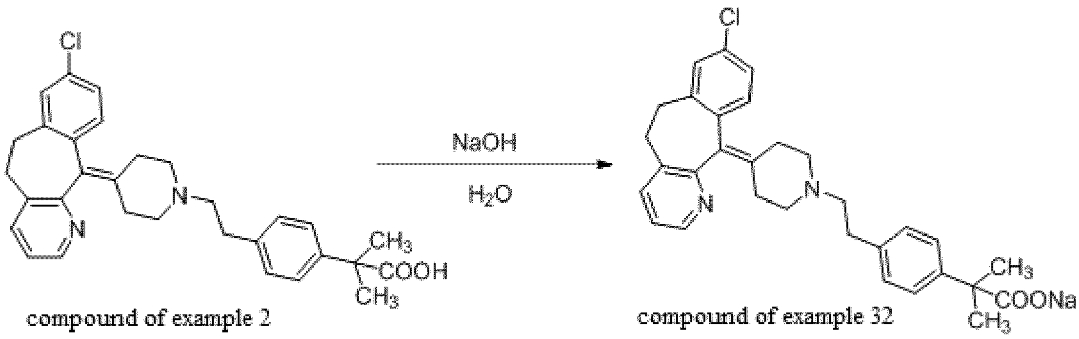


compound of example 2 compound of example 32

The compound of Example 2 (190 mg, 0.36 mmol) and methanol (2 mL) were added to a 25 mL single-necked flask, stirred magnetically and protected by $N_2$. A solution of NaOH (15 mg, 0.37 mmol) in water (0.5 mL) was added to the reaction system. After the addition, the reaction was stirred overnight at room temperature. TLC (DCM:MeOH=10:1) detected that the raw material and the product were in the same position. The reaction solution was concentrated under reduced pressure to obtain 185 mg of crude product. The crude product was slurried with n-heptane (6 mL) for 1 h, filtered with suction, the filter cake was washed with n-heptane (3 mL × 2), and dried (-0.1 MPa, 45 °C, 18 h) to obtain Compound of Example 32 (173 mg, 86.6% yield), as a off-white solid. HPLC: 98.637%.

$^1$H NMR (400 MHz, $CD_3OD$): δ ppm 8.29 (dd, $J_1$=4.8 Hz, $J_2$=1.6 Hz, 1 H), 7.63 (dd, $J_1$=7.6 Hz, $J_2$=1.6 Hz, 1 H), 7.41-7.36(m, 1H), 7.24-7.19 (m, 2 H), 7.16-7.13 (m, 1 H), 7.11-7.04 (m, 4 H), 3.43-3.34 (m, 2 H), 2.89-2.76 (m, 4 H), 2.62-2.58 (t, $J$=7.5 Hz, 2 H), 2.48-2.34 (m, 3H), 2.25-2.15 (m, 3 H), 1.83-1.78 (m, 2 H), 1.44 (s, 6 H).

**Example 33**

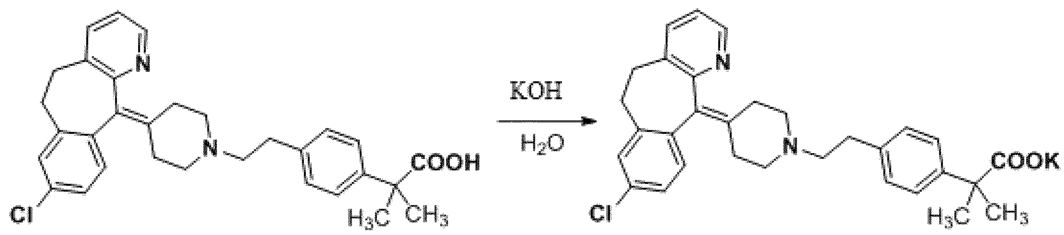


compound of example 2 compound of example 33

Refer to the preparation method of Example 31. NaOH was replaced with KOH to obtain the compound of Example 33 (173 mg, 86.6% yield) as a white solid. HPLC: 98.433%.

$^1$H NMR (400 MHz, $CD_3OD$): δ ppm 8.29 (dd, $J_1$=4.8 Hz, $J_2$=1.6 Hz, 1 H), 7.63 (dd, $J_1$=7.6 Hz, $J_2$=1.6 Hz, 1 H), 7.41-7.36(m, 1H), 7.24-7.19 (m, 2 H), 7.16-7.13 (m, 1 H), 7.11-7.04 (m, 4 H), 3.43-3.34 (m, 2 H), 2.89-2.76 (m, 4 H), 2.62-2.58 (t, $J$=7.5 Hz, 2 H), 2.48-2.34 (m, 3H), 2.25-2.15 (m, 3 H), 1.83-1.78 (m, 2 H), 1.44 (s, 6 H),

**Example 34**

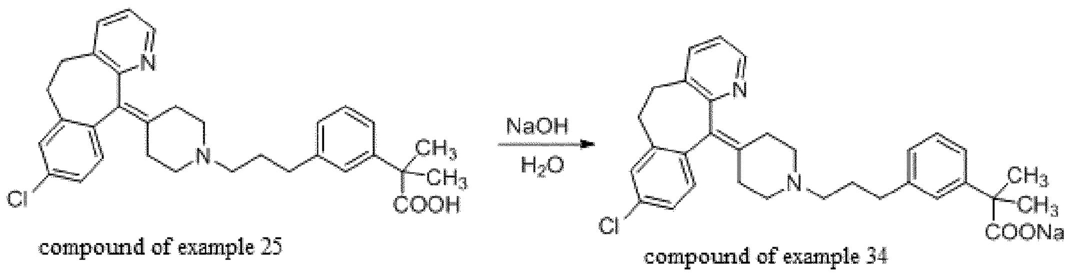


compound of example 25 compound of example 34

With reference to the preparation method of Example 31, the starting material was replaced from Example 2 to Example 25 to obtain the compound of Example 34 (152 mg, 83.3% yield) as an off-white solid. HPLC: 98.433%. $^1$H NMR (400 MHz, $CD_3OD$): δ ppm 8.28-8.33 (m, 1 H), 7.64 (d, $J$=6.80 Hz 1 H), 7.19-7.26 (m, 4 H), 7.08-7.18 (m, 3 H), 6.96 (d, $J$=7.3 Hz, 1 H), 3.34-3.36 (m, 2 H), 2.78-2.91 (m, 4 H), 2.60 (t, $J$=7.55 Hz, 2 H), 2.34-2.52 (m, 5 H), 2.17-2.30 (m, 3 H), 1.84 (t, $J$=7.55 Hz, 2 H), 1.46 (s, 6 H)

With reference to the preparation method of Example 31, the following compounds were prepared.

| Example | Structure formula | Structural confirmation data |
|---|---|---|
| 35 | 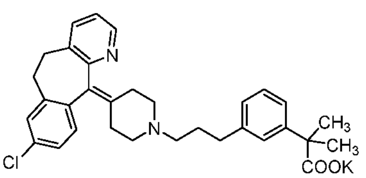 | HRMS(ESI): m/z Calculated 553.2024, found 553.2018 $[M+H]^+$. |
| 36 | 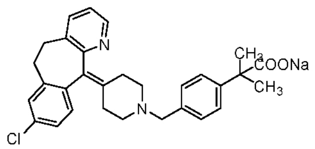 | $^1$H NMR (400 MHz, $CD_3OD$): δ ppm 8.28 (dd, $J_1$=1.2 Hz, $J_2$=5.2 Hz 1 H), 7.63 (dd, $J_1$=1.6 Hz, $J_2$=7.6 Hz 1 H), 7.35-7.37 (m, 2 H), 7.18-7.24 (m, 4 H), 7.14 (dd, $J_1$=2.4 Hz, $J_2$=8.4 Hz 1 H), 7.09 (d, $J$=8.0Hz, 1 H), 3.47 (s, 2 H), 3.35-3.43 (m, 2 H), 3.35-3.42 (m, 2H), 2.71-2.89 (m, 4 H), 2.39-2.46 (m, 2 H), 2.32-2.35 (m, 1 H), 2.12-2.21 (m, 3 H), 1.49(s, 6 H) |

(continued)

| Example | Structure formula | Structural confirmation data |
|---|---|---|
| 37 | 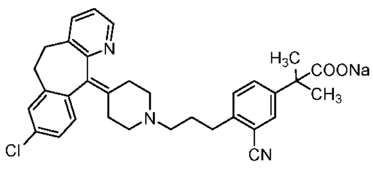 | $^1$H NMR (600 MHz, CD3OD) δ ppm 8.27-8.28 (m, 1 H), 7.64 (d, *J*=2.1 Hz, 1 H), 7.59-7.62 (m, 2 H), 7.29 (d, *J*=8.2 Hz, 1 H), 7.21 (dd, $J_1$=7.6 Hz, $J_2$=4.9 Hz, 1 H), 7.18 (d, *J*=2.2 Hz, 1 H), 7.13 (dd, $J_1$=8.1 Hz, $J_2$=2.2 Hz, 1 H), 7.09 (d, J=8.1 Hz, 1 H), 3.35-3.40 (m, 2 H), 2.74-2.86 (m, 6 H), 2.32-2.45 (m, 5 H), 2.10-2.22 (m, 3 H), 1.79-1.88 (m, 2 H), 1.45 (s, 6 H) |
| 38 | 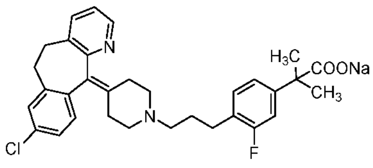 | $^1$H NMR (400 MHz, $CD_3OD$): δ ppm 8.29 (dd, $J_1$=4.8 Hz, $J_2$=1.6 Hz, 1 H), 7.63 (dd, $J_1$=7.6 Hz, $J_2$=1.6 Hz, 1 H), 7.24-7.19 (m, 2 H), 7.16-7.13 (m, 1 H), 7.11-7.04 (m, 4 H), 3.43-3.34 (m, 2 H), 2.89-2.76 (m, 4 H), 2.62-2.58 (t, *J*=7.5 Hz, 2 H), 2.48-2.34 (m, 5 H), 2.25-2.15 (m, 3 H), 1.83-1.78 (m, 2 H), 1.44 (s, 6 H) |
| 39 | 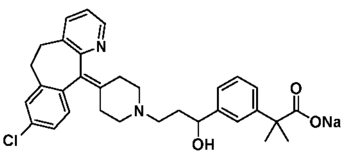 | $^1$H NMR (400MHz, $CD_3OD$): δ ppm 8.31 (d, *J*=4.8 Hz, 1 H), 7.65 (d, *J*=7.6 Hz, 1 H), 7.39 (s, 1 H), 7.31 (d, *J*=7.6 Hz, 1 H), 7.19-7.26 (m, 3 H), 7.10-7.17 (m, 3 H), 4.68-4.71 (m, 1 H), 3.35-3.44 (m, 2 H), 2.77-2.90 (m, 4 H), 2.53-2.58 (m, 1 H), 2.37-2.48 (m, 4 H), 2.12-2.26 (m, 3 H), 1.87-1.97 (m, 2 H), 1.48 (s, 6 H);<br>$^{13}$C NMR (100 MHz, $CD_3OD$): *δ* ppm 185.36, 158.71, 150.02, 147.00, 145.38, 141.14, 139.67, 139.46, 138.58, 135.87, 134.11, 133.72, 131.86, 130.33, 128.85, 127.02, 126.20, 124.89, 124.06, 75.03, 56.64, 55.65, 55.53, 36.37, 32.74, 32.03, 31.69, 31.62, 31.57, 28.63 |
| 40 | 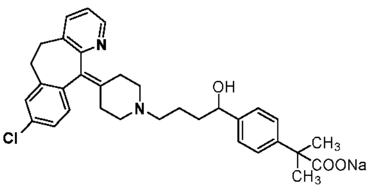 | $^1$H NMR(400 MHz, $CD_3OD$): δ ppm 7.31 (d, *J*=4.8 Hz, 1 H), 7.65 (d, *J*=6.8 Hz, 1 H), 7.30-7.40 (m, 2 H), 7.17-7.27 (m, 4 H), 7.11-7.16 (m, 2 H), 4.58-4.63 (m, 1 H), 3.36-3.46 (m, 2 H), 2.81-2.92 (m, 4 H), 2.23-2.49 (m, 8 H), 1.45-1.80 (m, 4 H), 1.48-1.49 (m, 6 H);<br>$^{13}$C NMR (100 MHz, $CD_3OD$): δ ppm 182.81, 156.11, 147.40, 146.42, 144.60, 143.32, 140.99, 138.72, 137.06, 136.60, 136.07, 133.41, 131.50, 129.38, 127.91, 126.41, 124.50, 124.12, 122.52, 121.67, 72.76, 72.27, 56.83, 52.89, 52.81, 36.04, 30.28, 29.59, 28.75, 28.60, 26.14, 21.56 |

**Example 41**

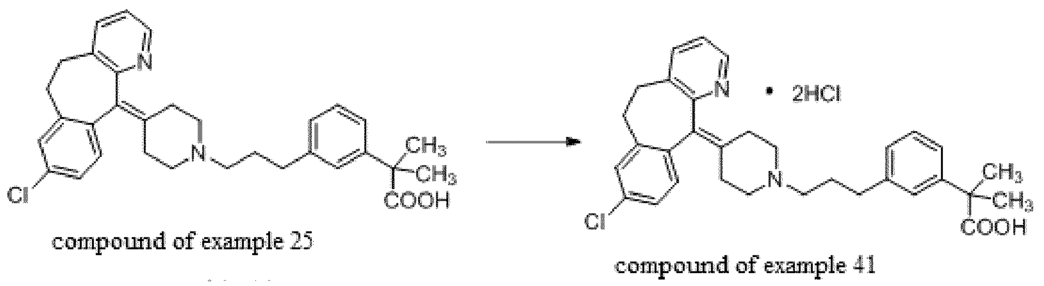

compound of example 25

compound of example 41

Add the compound of Example 25 (500 mg, 0.97 mmol) and dioxane hydrochloride (3 mL, 2.7 M) to a 25 mL single-necked flask, and react at room temperature for 3 hours. The reaction solution was directly concentrated to obtain a residue. DCM (10 mL) was added thereto. The solution was stirred to clear, concentrated and n-heptane (6 mL) was added to make a slurry overnight. After filtration, the solid filter cake was collected and dried under the conditions of -0.1 MPa, 49 °C and 8 hours to obtain the compound of Example 41 (380 mg, 66.46% yield) as an off-white solid.

$^1$H NMR (400 MHz, $D_2O$): $\delta$ ppm 8.46 (d, $J$=5.0 Hz, 1 H), 8.28 (d, $J$=7.9 Hz, 1 H), 7.78 (dd, $J_1$=7.9 Hz,$J_2$=5.8 Hz, 1 H), 7.29-7.39 (m, 2 H), 7.19-7.29 (m, 3 H), 7.11-7.17 (m, 2 H), 3.45-3.66 (m, 3 H), 3.17-3.44 (m, 2 H), 2.91-3.16 (m, 3 H), 2.70-2.89 (m, 4 H), 2.53-2.67 (m, 4 H), 1.98-2.03(m, 2 H), 1.51 (s, 1.3 H), 1.48 (s, 4.7 H).

**Example 42**

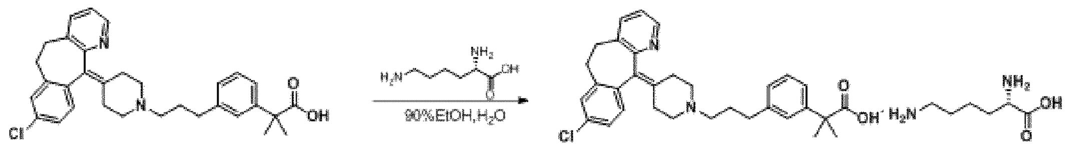


compound of example 25

compound of example 42

Add the compound of Example 25 (153 mg, 0.3 mmol), 90% ethanol (3 mL), water (0.5 mL) and L-lysine (44 mg, 0.3 mmol) to a 25 mL single-necked flask and react at 60°C under a nitrogen atmosphere for 1.5 h. The reaction solution was concentrated to dryness and became viscous oil. Add diethyl ether (20 mL), make a slurry for 1 h and filter. The solid was vacuum dried at room temperature for 2 h to obtain the compound of Example 42 (167 mg, 84% yield) as an off-white solid.

$^1$H NMR (400 MHz, $CD_3OD$): $\delta$ ppm 8.20 (dd, $J_1$=1.6 Hz, $J_2$=5.2 Hz, 1 H), 7.53 (dd, $J_1$=0.8 Hz, $J_2$=7.6 Hz, 1 H), 7.11-7.16 (m, 4 H), 7.00-7.09 (m, 3 H), 6.87 (d, $J$=7.6 Hz, 1 H), 3.36-3.39 (m, 1 H), 3.24-3.34 (m, 2 H), 2.69-2.80 (m, 6 H), 2.49 (t, $J$=7.6 Hz, 2 H), 2.33-2.42 (m, 4 H), 2.13-2.31 (m, 4 H), 1.64-1.79 (m, 4 H), 1.51-1.59 (m, 2 H), 1.37-1.42 (m, 2 H), 1.33 (s, 6 H).

The composition examples of the present invention taking the compound of Example 34 as an example include but are not limited to the excipients and formulation ratios used in the following examples.

**Example 43**

Coated tablets of the compound of Example 34 (1000 tablets)

Tablet core prescription

| Raw material | Amount (g) |
|---|---|
| Compound of example 34 | 10.0 |
| Microcrystalline cellulose | 37.5 |
| Dicalcium phosphate | 12.5 |
| Pregelatinized starch | 43.8 |
| Magnesium stearate | 1.3 |
| 30%EtOH | Appropriate amount |

Coating liquid prescription

| Coating liquid prescription | |
|---|---|
| Raw material | Amount (g) |
| Opadry | 10.0 |
| 80%EtOH | 100mL |

Preparation:
The compound of Example 34 and magnesium stearate were passed through a 120-mesh sieve, and the microcrystalline cellulose, calcium hydrogen phosphate and pregelatinized starch were passed through a 100-mesh sieve. Weigh the compound of Example 34, microcrystalline cellulose, dibasic calcium phosphate and pregelatinized starch in the prescription proportions and mix them evenly through an 80-mesh sieve in equal increments. Make the soft material with

30% ethanol solution as a wetting agent. Granulate with a 20-mesh sieve, dry at 50-60°C for 3 to 4 hours and size with a 20-mesh sieve, add the prescribed amount of magnesium stearate, mix well and compress to tablets.

**Example 44**

Ordinary tablets of the compound of Example 34 (1000 tablets)

| Raw material | Amount (g) |
|---|---|
| Compound of example 34 | 10.0 |
| Microcrystalline cellulose | 37.5 |
| Carboxymethyl starch sodium | 12.5 |
| Lactose | 43.8 |
| Magnesium stearate | 1.3 |
| 30%EtOH | Appropriate amount |

Preparation:
The compound of Example 34 and magnesium stearate were passed through a 120-mesh sieve, and the microcrystalline cellulose, sodium carboxymethyl starch and lactose were passed through an 80-mesh sieve. Weigh the compound of Example 34, microcrystalline cellulose, sodium carboxymethyl starch and lactose in the prescription proportions and mix them evenly through an 80-mesh sieve in equal increments. Prepare the soft materials with 30% ethanol solution as a wetting agent. Granulate with a 30-mesh sieve, dry at 50~60°C for 3-4 hours and size with a 30-mesh sieve, add prescription amount of magnesium stearate, mix well and compress to tablets.

**Example 45**

Capsules of the compound of Example 34 (1000 capsules)

| Raw material | Amount (g) |
|---|---|
| Compound of example 34 | 10.0 |
| Low-substituted hydroxypropyl cellulose | 37.5 |
| Carboxymethyl starch sodium | 12.5 |
| Lactose | 43.8 |
| Magnesium stearate | 1.3 |
| 30%EtOH | Appropriate amount |

Preparation:
The compound of Example 34 and magnesium stearate were passed through a 120-mesh sieve, and the low-substituted hydroxypropyl cellulose, sodium carboxymethyl starch, and lactose were passed through an 80-mesh sieve. Weigh the compound of Example 34, low-substituted hydroxypropyl cellulose, sodium carboxymethyl starch and lactose in the prescription proportions and mix them evenly through an 80-mesh sieve in equal increments. Prepare the soft materials with 30% ethanol solution as a wetting agent. Granulate with a 30-mesh sieve, dry at 50~60°C for 3-4 hours and size with a 30-mesh sieve, add prescription amount of magnesium stearate, mix well and fill the capsule.

**Example 46**

Granules of the compound of Example 34 (1000 packets)

| Raw material | Amount (g) |
|---|---|
| Compound of example 34 | 10.0 |
| Microcrystalline cellulose | 125.0 |
| Sucrose powder | 875.0 |
| Carboxymethyl starch sodium | 150.0 |
| Lactose | 125.0 |

(continued)

| Raw material | Amount (g) |
|---|---|
| Aspartame | 75.0 |
| Orange flavor | 18.8 |
| Sodium dodecyl sulfate | 6.3 |
| 3% povidone in 30% ethanol solution | Appropriate amount |

Preparation:
The compound of Example 34, microcrystalline cellulose, sucrose powder, sodium carboxymethyl starch, lactose and aspartame were passed a 100 mesh sieve, and orange flavor and sodium dodecyl sulfate were passed an 80 mesh sieve. Weigh the compound of Example 34, microcrystalline cellulose, sucrose powder, sodium carboxymethyl starch, lactose and aspartame in the prescription ratio and mix them in equal increments, prepare the soft materials with 3% povidone in 30% ethanol solution as a wetting agent, granulate with a 20-mesh sieve, dry at 50~60°C for 3-4 hours and size with an 18-mesh sieve, add prescription amount of orange flavor and sodium dodecyl sulfate, mix well, fill bags and seal.

**Example 47**

Oral liquid of the compound of Example 34 (1000 bottles

| Raw material | Amount (g) |
|---|---|
| Compound of example 34 | 10.0 |
| Aspartame | 25.0 |
| Orange flavor | 2.5 |
| Sodium citrate | 6.8 |
| Water for Injection | 5000mL |

Preparation:
Dissolve aspartame, orange flavor and sodium citrate in freshly prepared water for injection, filter, add the prescribed amount of the compound of Example 34 at room temperature and dissolve it, filter and fill.

**Example 48**

Syrup of the compound of Example 34

| Raw material | Amount (g) |
|---|---|
| Compound of example 34 | 10.0 |
| Sucrose | 875.0 |
| Aspartame | 25.0 |
| Orange flavor | 2.5 |
| Sodium citrate | 6.8 |
| Water for Injection | 1000mL |

Preparation:
Add sucrose to 900ml of water for injection, heat to boil, dissolve, filter while hot, and cool to room temperature for later use. Dissolve the compound of Example 34, aspartame, orange flavor and sodium citrate in the prescription amount in 60 mL of water for injection, filter and add to the above syrup, add water for injection to 1000 mL, mix well and fill.

**Example 49**

Eye drops of the compound of Example 34

| Raw material | Amount (g) |
|---|---|
| Compound of example 34 | 0.05 |
| Citric acid | 0.2 |
| Borax | Appropriate amount |
| Benzalkonium chloride | 0.005 |
| Sodium chloride | 0.8 |
| Polysorbate 80 | 0.1 |
| Sterile water for injection | 100mL |

Preparation:
Polysorbate 80, citric acid, sodium chloride, the compound of Example 34 and benzalkonium chloride were completely dissolved in 70 ml of sterile water for injection. The pH was adjusted to 7.8 with borax. Make up to 100 mL with sterile water for injection. Filter. The filtrate was sterilized and filled in a container suitable for eye drops.

**Example 50**

Injection of the compound of Example 34 (1000 bottles

| Raw material | Amount (g) |
|---|---|
| Compound of example 34 | 250.0 |
| Citric acid | 3.0 |
| Disodium phosphate | 5.0 |
| Disodium ethylenediaminetetraacetate | 0.01 |
| Sodium chloride | 3.0 |
| Activated carbon | 0.5 |
| Water for injection to | 2000mL |

Preparation:
Dissolve citric acid, disodium hydrogen phosphate and disodium ethylenediaminetetraacetate in 1600 mL of water for injection, add the compound of Example 34 and sodium chloride in the prescribed amount and stir until completely dissolved, adjust the pH to 7.0 -7.8 with 0.1 mol/L dilute hydrochloric acid, add a prescription amount of activated carbon, stir and adsorb, filter to decarburize, add water for injection to 2000mL, then filter with a 0.22μm filter, and fill in a 2mL ampoule. The ampoule is sealed and sterilized at 115°C for 30 minutes.

**Example 51**

Nasal spray of the compound of Example 34

| Raw material | Amount (g) |
|---|---|
| Compound of example 34 | 10 |
| Chitosan | 0.5 |
| Metal ion chelating agent EDTA-2Na | 0.1 |
| Mannitol | 3.2 |
| Chlorhexidine acetate | 0.01 |
| Phosphate buffer | Adjust the pH to 3.8~5.5 |
| Water for injection to | 100mL |

The preparation process was as follows:
60% of the prescription amount of buffer solution was placed in the preparation container. The bioadhesive was added to fully swell until it was completely dissolved. Add the bacteriostatic agent, isotonic regulator and metal ion chelating agent, stir and dissolve. Add the compound of Example 34 and stir for 1 to 2 hours. After the compound was completely

dissolved, the buffer was added to the full amount and the mixture was uniformly mixed. After the pH and content were qualified, the drug solution was filtered through a 0.45μm microporous filter membrane until it was clear. It was then filtered through a 0.22μm microporous filter membrane for sterilization and divided into sterilized nasal spray bottles. Close and seal the cap with a quantitative spray pump. Store it at low temperature.

**Example 52**

Nasal spray of the compound of Example 34

| Raw material | Amount (g) |
|---|---|
| Compound of example 34 | 1.5 |
| Glycerate | 1.5 |
| Vitamin E | 1.0 |
| Mannitol | 1.0 |
| Citric acid | 1.0 |
| Polyoxyethylene 40 hydrogenated castor oil | 3.0 |
| Water for injection to | 10mL |

Preparation:
Mannitol, polyoxyethylene 40 hydrogenated castor oil, vitamin E and the compound of Example 34 were added to the water for injection to 10mL. Heat it slowly at 60°C, add glycerate and disperse at high speed. Adjust the pH to 6.0 with citric acid, filter and place in a container after autoclaving.

**Claims**

1. A tricyclic compound having the structure of the following formula (I), and its pharmaceutically acceptable salt, prodrug, tautomer, stereoisomer, or mixture of stereoisomers,

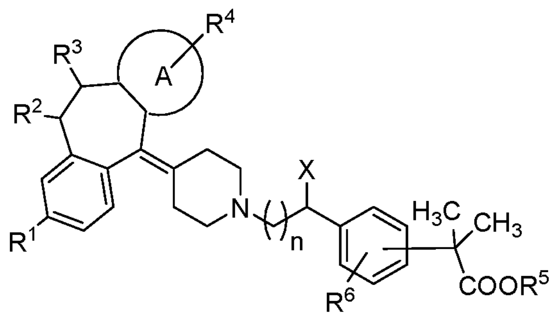

(I)

among formula (I):

X is H or OH;
n is an integer from 0-6;
Ring A is a benzene ring, a pyridine ring or a thiophene ring;
$R^1$, $R^4$ are H or halogen;
$R^2$, $R^3$ are selected from H, hydroxyl or =O;
$R^5$ is H;
$R^6$ is H, any mono- or poly-substituted $C_1$-$C_6$ alkyl, halogen, hydroxy, trifluoromethyl, cyano, oxytrifluoromethyl, methoxy, amino, nitro or carboxy.

2. The compound according to claim 1, **characterized in that** in formula (I):

n is an integer from 0-4;

$R^1$ is H or Cl;
$R^5$ is H;
$R^6$ is H, methyl, F, Cl, Br, hydroxyl, trifluoromethyl, cyano, oxytrifluoromethyl, methoxy, amino, or nitro.

**3.** The compound of claim 1, wherein the structure of formula (I) is:

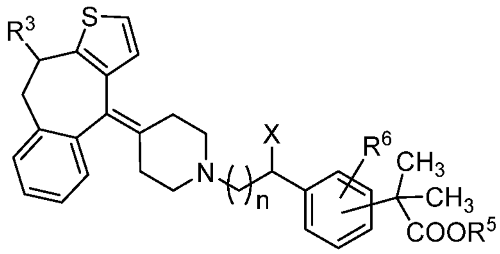

Wherein,

n is an integer of 0-6;
X is H or OH;
$R^3$ is H, hydroxyl or =O;
$R^5$ is H;
$R^6$ is H, any mono- or poly-substituted C1-C6 alkyl, halogen, hydroxy, trifluoromethyl, cyano, oxytrifluoromethyl, methoxy, amino, nitro or carboxy.

**4.** The compound of claim 1, wherein the structure of formula (I) is

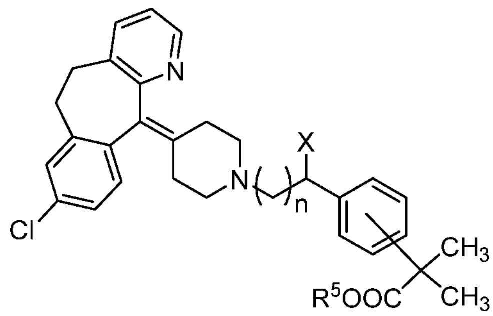

Wherein,

n is an integer of 0-6;
X is H or OH;
$R^5$ is H。

**5.** The compound according to any one of claims 1 to 4, wherein the said pharmaceutically acceptable salt is a salt formed by a compound of formula (I) with an alkali metal, alkaline earth metal, an amino acid or a basic compound containing an amino group, or a salt formed by a compound of formula (I) with an inorganic acid or organic acid, or a complex salt formed by compound of formula (I) with a polybasic acid and an alkali metal or alkaline earth metal; preferably, wherein the said pharmaceutically acceptable salt is as follows: potassium, sodium or ammonium salt of the compound of formula (I); a salt formed by compound of formula (I) and hydrochloric acid, sulfuric acid, phosphoric acid, hydrobromic acid, maleic acid, fumaric acid, citric acid, methanesulfonic acid, p-toluenesulfonic acid, tartaric acid or acetic acid; or a complex salt formed by compound of formula (I) with a polybasic acid and an alkali metal or alkaline earth metal, wherein the said polybasic acid is selected from the group consisting of citric acid, succinic acid, tartaric acid, fumaric acid, maleic acid, oxalic acid, sulfuric acid, phosphoric acid, sulfurous acid and malic acid, and the said alkali metal or alkaline earth metal is selected from sodium, potassium, calcium, magnesium and zinc.

**6.** The compound according to any one of claims 1 to 5, wherein the compound is selected from the group consisting

of compound 1 to compound 42:

| Compound | Structural formula | Compound | Structural formula |
|---|---|---|---|
| 1 | 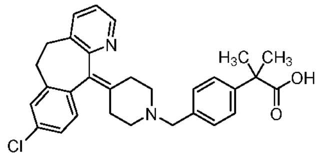 | 22 | 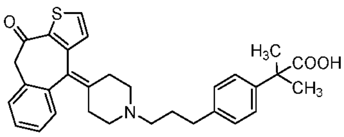 |
| 2 | 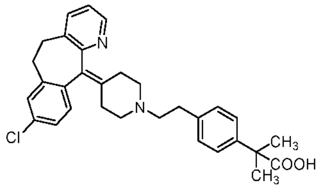 | 23 | 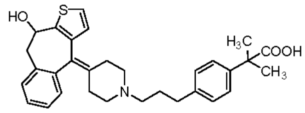 |
| 3 | 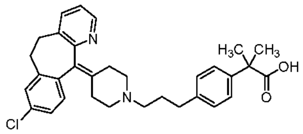 | 24 | 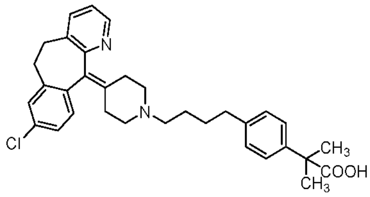 |
| 4 | 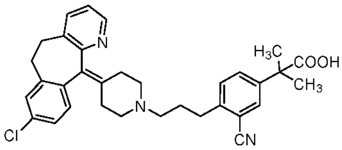 | 25 | 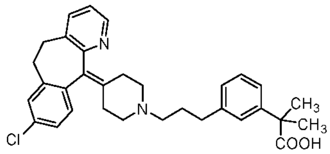 |
| 5 | 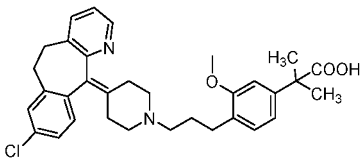 | 26 | 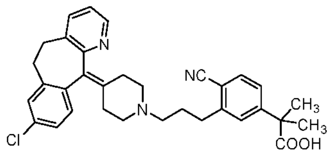 |
| 6 | 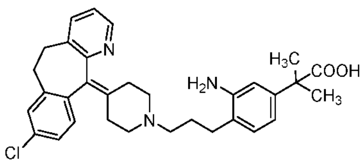 | 27 | 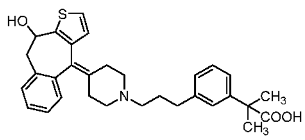 |
| 7 | 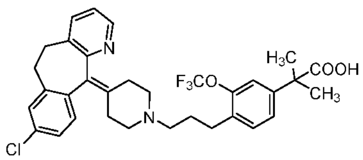 | 28 | 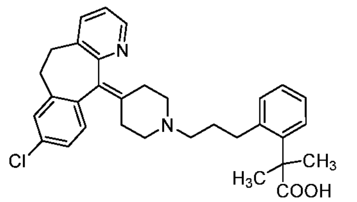 |
| 8 | 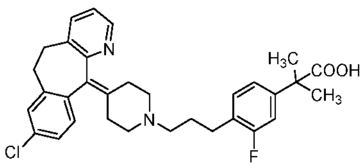 | 29 | 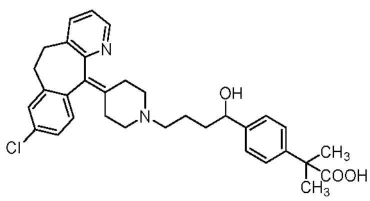 |

(continued)

| Compound | Structural formula | Compound | Structural formula |
|---|---|---|---|
| 9 | 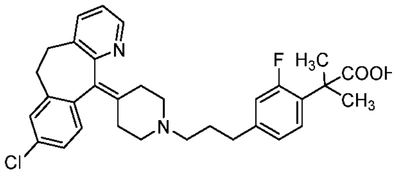 | 30 | 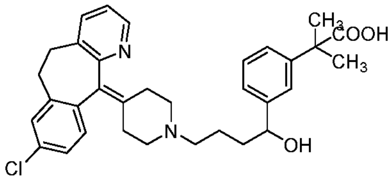 |
| 10 | 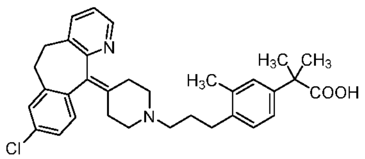 | 31 | 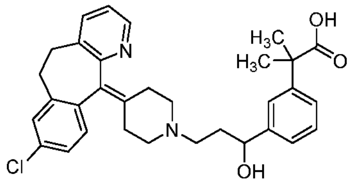 |
| 11 | 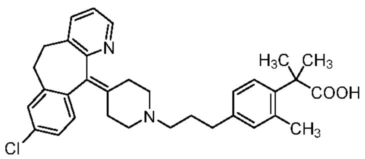 | 32 | 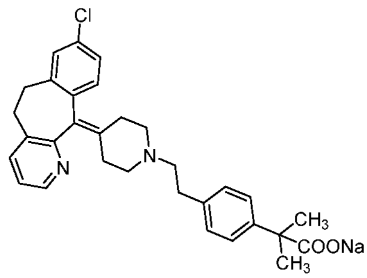 |
| 12 | 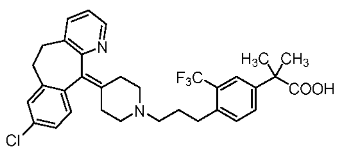 | 33 | 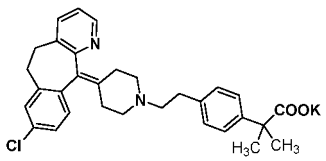 |
| 13 | 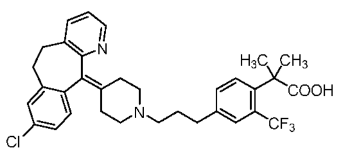 | 34 | 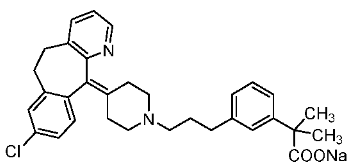 |
| 14 | 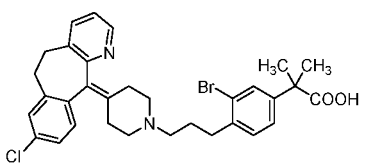 | 35 | 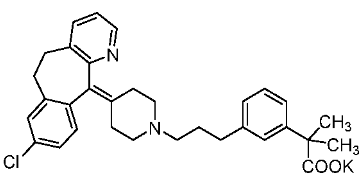 |
| 15 | 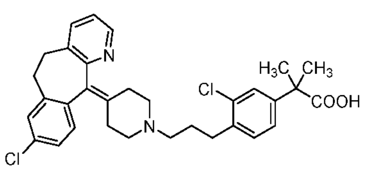 | 36 | 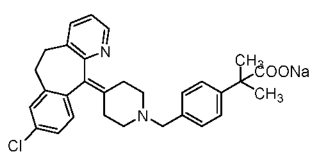 |
| 16 | 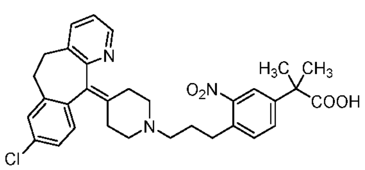 | 37 | 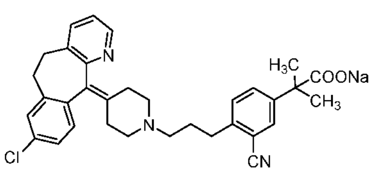 |

(continued)

| Compound | Structural formula | Compound | Structural formula |
|---|---|---|---|
| 17 | 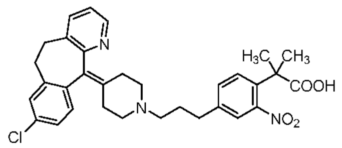 | 38 | 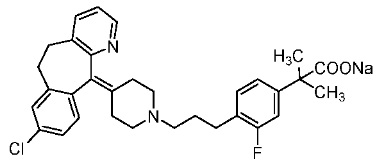 |
| 18 | 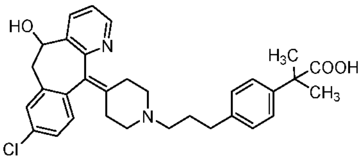 | 39 | 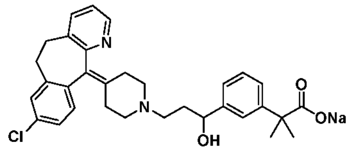 |
| 19 | 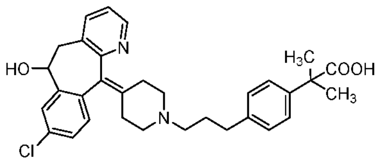 | 40 | 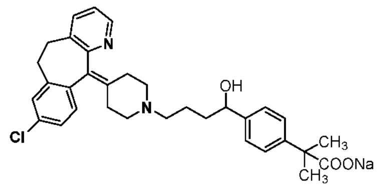 |
| 20 | 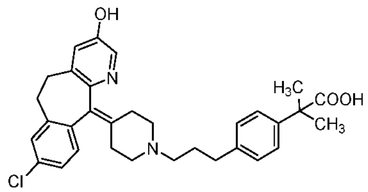 | 41 | 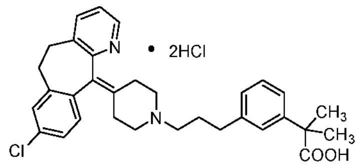 |
| 21 | 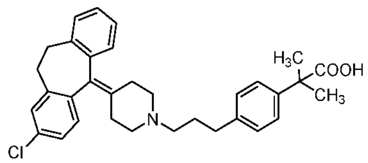 | 42 | 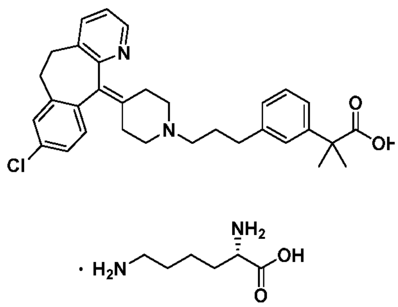 |

**7.** The method for preparing the compound according to any one of claims 1 to 6, comprising the step of generating the compound of formula (I) by reacting the compound of formula (II) as follows:

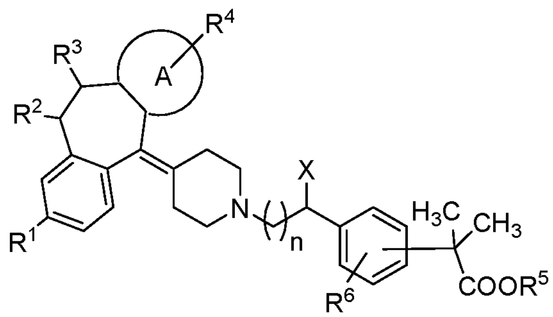

(II)

Wherein, X, n, A, $R^1$, $R^2$, $R^3$, $R^4$ and $R^6$ are as defined in claims 1-6, and $R^5$ is a $C_1$-$C_6$ alkyl group.

**8.** The compound of the following formula (II),

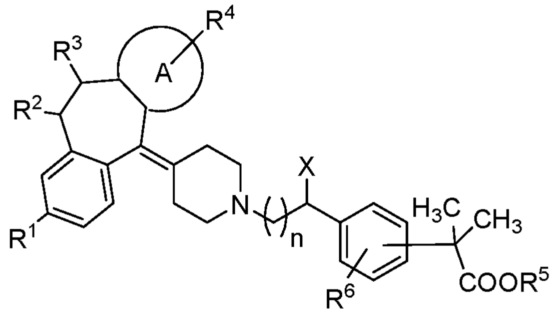

(II)

Wherein, X, n, A, $R^1$, $R^2$, $R^3$, $R^4$ and $R^6$ are as defined in claims 1-6, and $R^5$ is a $C_1$-$C_6$ alkyl group.
Preferably, the said compound of formula (II) is a compound of the following structure:

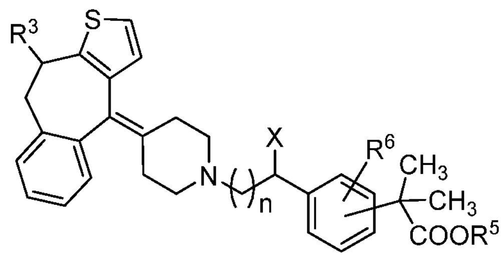

Wherein,

n is an integer of 0-6;
X is H or OH;
$R^3$ is H, hydroxyl or =O;
$R^5$ is a C1 ~ C6 alkyl group;
$R^6$ is H, any mono- or poly-substituted $C_1$-$C_6$ alkyl, halogen, hydroxy, trifluoromethyl, cyano, oxytrifluoromethyl, methoxy, amino, nitro or carboxy.
Or, the said compound of formula (II) is a compound of the following structure:

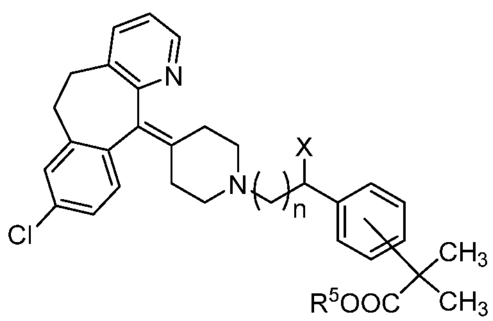

Wherein,

n is an integer of 0-6;
X is H or OH;
$R^5$ is a C1 ~ C6 alkyl group.

**9.** A pharmaceutical composition, which contains the compound described in claims 1-6 and pharmaceutically acceptable excipients; preferably, the dosage form of the pharmaceutical composition is selected from the group consisting of tablets, capsules, granules, oral liquids, oral sprays, suppositories, transdermal preparations, injections, nasal

drops, eye drops and nasal sprays.

**10.** The use of the compound of claims 1-6 in the preparation of antihistamine drugs; preferably, the drug is a drug for preventing or treating allergic diseases; preferably, the allergic diseases are selected from the group consisting of allergic rhinitis, urticaria, chronic urticaria, allergic purpura, asthma, allergic dermatitis, eczema, allergic conjunctivitis, atopic dermatitis, sinusitis and chronic sinusitis.

| INTERNATIONAL SEARCH REPORT | International application No. PCT/CN2021/090073 |
|---|---|

**A. CLASSIFICATION OF SUBJECT MATTER**

C07D 405/04(2006.01)i；C07D 211/14(2006.01)i；C07D 401/06(2006.01)i

According to International Patent Classification (IPC) or to both national classification and IPC

**B. FIELDS SEARCHED**

Minimum documentation searched (classification system followed by classification symbols)

C07D 405/04，C07D 211/14，C07D 401/06，C07D

Documentation searched other than minimum documentation to the extent that such documents are included in the fields searched

Electronic data base consulted during the international search (name of data base and, where practicable, search terms used)

数据库: DWPI, SIPOABS, CNABS, CNKI, REGISTRY, CAPLUS: 氯雷他定, 抗组胺, 非索非那定, 环庚, 哌啶, antihistamine, piperidinyl, cyclohepten, fexofenadine, structural formula search according to structures of formulae I, II

**C. DOCUMENTS CONSIDERED TO BE RELEVANT**

| Category* | Citation of document, with indication, where appropriate, of the relevant passages | Relevant to claim No. |
|---|---|---|
| X | WO 8912443 A1 (FISONS CORP) 28 December 1989 (1989-12-28)<br>see entire document, in particular embodiment 26, claims 1, 5, 7-8 | 1-10 |
| A | WO 9200293 A1 (SCHERING CORP.) 09 January 1992 (1992-01-09)<br>see entire document, in particular abstract, claims 1, 39-43 | 1-10 |
| A | US 2014135361 A1 (LIN TONGJUN et al.) 15 May 2014 (2014-05-15)<br>see entire document, in particular abstract, embodiment 2, claim 1 | 1-10 |
| A | US 5231101 A (SS PHARMACEUTICAL CO) 27 July 1993 (1993-07-27)<br>see entire document, in particular table 6, claim 1 | 1-10 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 July 2021** | **02 August 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/090073**

| Patent document cited in search report | | | Publication date (day/month/year) | Patent family member(s) | | | Publication date (day/month/year) |
|---|---|---|---|---|---|---|---|
| WO | 8912443 | A1 | 28 December 1989 | EP | 0347123 | A2 | 20 December 1989 |
| | | | | JP | H03500053 | A | 10 January 1991 |
| | | | | EP | 0347123 | A3 | 03 July 1991 |
| | | | | PT | 90868 | A | 29 December 1989 |
| | | | | DK | 39490 | A | 11 April 1990 |
| | | | | DK | 39490 | D0 | 15 February 1990 |
| | | | | AU | 3850389 | A | 12 January 1990 |
| WO | 9200293 | A1 | 09 January 1992 | DE | 122008000047 | I2 | 16 June 2011 |
| | | | | PL | 297300 | A1 | 13 July 1992 |
| | | | | IE | 912124 | A1 | 01 January 1992 |
| | | | | NO | 924955 | L | 22 February 1993 |
| | | | | AU | 646878 | B2 | 10 March 1994 |
| | | | | EP | 0535152 | B1 | 09 August 1995 |
| | | | | NO | 924955 | A | 22 February 1993 |
| | | | | NZ | 238629 | A | 27 September 1993 |
| | | | | NO | 924955 | D0 | 21 December 1992 |
| | | | | DE | 69112061 | T2 | 14 December 1995 |
| | | | | DK | 0535152 | T3 | 25 September 1995 |
| | | | | AU | 8225291 | A | 23 January 1992 |
| | | | | JP | H0761998 | B2 | 05 July 1995 |
| | | | | FI | 925820 | A0 | 22 December 1992 |
| | | | | IE | 68935 | B1 | 24 July 1996 |
| | | | | CA | 2085878 | A1 | 23 December 1991 |
| | | | | KR | 960012368 | B1 | 20 September 1996 |
| | | | | US | 5422351 | A | 06 June 1995 |
| | | | | RU | 2086549 | C1 | 10 August 1997 |
| | | | | JP | H05507092 | A | 14 October 1993 |
| | | | | OA | 10032 | A | 14 October 1996 |
| | | | | IL | 98572 | A | 31 July 1995 |
| | | | | NO | 303223 | B1 | 15 June 1998 |
| | | | | ES | 2096657 | T3 | 16 March 1997 |
| | | | | CZ | 374892 | A3 | 13 October 1993 |
| | | | | CZ | 9203748 | A3 | 13 October 1993 |
| | | | | SK | 374892 | A3 | 12 June 2000 |
| | | | | NL | 300378 | I1 | 06 May 2009 |
| | | | | CA | 2085878 | C | 13 June 2000 |
| | | | | ZA | 9104764 | A | 25 March 1992 |
| | | | | EP | 0535152 | A1 | 07 April 1993 |
| | | | | NL | 300378 | I2 | 01 September 2009 |
| | | | | HU | 9204079 | D0 | 29 March 1993 |
| | | | | FI | 925820 | A | 22 December 1992 |
| | | | | HK | 186496 | A | 11 October 1996 |
| | | | | GR | 3017924 | T3 | 31 January 1996 |
| | | | | AT | 126226 | T | 15 August 1995 |
| | | | | SK | 280717 | B6 | 12 June 2000 |
| | | | | HU | 220622 | B1 | 28 March 2002 |
| | | | | CZ | 284610 | B6 | 13 January 1999 |
| | | | | ZA | 914764 | B | 25 March 1992 |
| | | | | IL | 98572 | D0 | 15 July 1992 |
| | | | | PL | 173224 | B1 | 27 February 1998 |

Form PCT/ISA/210 (patent family annex) (January 2015)

**INTERNATIONAL SEARCH REPORT**
**Information on patent family members**

International application No.
**PCT/CN2021/090073**

| Patent document cited in search report | Publication date (day/month/year) | Patent family member(s) | Publication date (day/month/year) |
|---|---|---|---|
| | | DE 69112061 D1 | 14 September 1995 |
| | | HU T63161 A | 28 July 1993 |
| | | DK 535152 T3 | 25 September 1995 |
| US 2014135361 A1 | 15 May 2014 | CN 103619839 A | 05 March 2014 |
| | | CN 103619839 B | 01 June 2016 |
| | | JP 2014526438 A | 06 October 2014 |
| | | JP 5978296 B2 | 24 August 2016 |
| | | EP 2727919 A1 | 07 May 2014 |
| | | US 9296731 B2 | 29 March 2016 |
| | | EP 2727919 A4 | 10 December 2014 |
| | | US 10022362 B2 | 17 July 2018 |
| | | WO 2013000406 A1 | 03 January 2013 |
| | | US 2016166563 A1 | 16 June 2016 |
| | | EP 2727919 B1 | 08 May 2019 |
| US 5231101 A | 27 July 1993 | CA 2059363 A1 | 19 July 1992 |
| | | CA 2059363 C | 22 December 1998 |
| | | JP H0559040 A | 09 March 1993 |
| | | JP H0676403 B2 | 28 September 1994 |
| | | KR 920014799 A | 25 August 1992 |
| | | TW 207538 B | 11 June 1993 |
| | | EP 0495484 A1 | 22 July 1992 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107602534 B
- CN 109096251 A
- WO 9200293 A1


### Non-patent literature cited in the description


- **ZOLALY MA.** Histamine H1 antagonists and clinical characteristics of febrile seizures[J. *Int J Gen Med.,* 2012, vol. 5, 277-281